# EUROPEAN PATENT APPLICATION

(11) **EP 4 520 751 A1**
(43) Date of publication of application: **12.03.2025**
(21) Application number: 23195496.7
(22) Date of filing: 05.09.2023
(51) Int. Cl.: C07D 209/94, C07B 59/00, C07D 235/02, C07D 237/08, C07D 241/36, C07D 257/08, C07D 401/04, C07D 487/04

(54) **METHOD FOR RAPID OXIDATION OF DIHYDROPYRIDAZINES TO PYRIDAZINES**

(71) Applicant: University of Copenhagen, 1165 Copenhagen K (DK); Tetrakit Technologies ApS, 2200 Copenhagen N (DK)
(72) Inventor: HERTH, Matthias Manfred, 21121 Malmö (SE); BATTISTI, Umberto Maria, 2100 Copenhagen (DK); POULIE, Christian Bernard Matthijs, 21837 Bunkeflostrand (SE); SHALGUNOV, Vladimir, 2820 Gentofte (DK)
(74) Representative: Budde Schou A/S

(57) **Abstract**

The present invention relates to a method for providing pyridazines by rapid oxidation of dihydropyridazines. The pyridazines can be used in therapy such as radiotherapy, diagnostics, imaging, and other photochemistry methods. The method applies to dihydropyridazine mixture that arises from the click reaction between a first chemical entity which is either a 1,2,4,5-tetrazine or a strained alkene having inverse electron demand Diels-Alder cycloaddition reactivity and a second chemical entity which is either a 1,2,4,5-tetrazine or a strained alkene having complementary inverse electron demand Diels-Alder cycloaddition reactivity. The ligation between the first chemical entity and the second chemical entity is followed by a rapid acid catalyzed oxidation.

## Description

### FIELD

The present invention relates to a method for providing pyridazines by rapid oxidation of dihydropyridazines. The pyridazines can be used in therapy such as radiotherapy, diagnostics, imaging, and other photochemistry methods.

### BACKGROUND

Labeled targeting vectors based on the tetrazine ligation can be used both for imaging purposes, such as diagnostics and other photochemistry imaging methods and in therapy. Such targeting vectors have for instance been labeled with radiolabels that can be applied in diagnostics and/or in therapy. The specific use depends on the identity of the radiolabeling used because different radionuclides provide for different purposes. The specific use moreover depends on the specific target that the vector is directed at. Several combinations of radiolabels and vectors are applied presently in diagnosis, therapy, theranostic and imaging. Different chemical entities connecting the radiolabeled entity with the target directed entity exists. The present invention is based on the tetrazine ligation wherein a tetrazine (Tz) and a *trans*-cylcooctene (TCO) are ligated, followed by a rapid oxidation of the corresponding click-product, i.e. dihydropyridazines, to the corresponding pyridazines, thereby reducing the complexity of the reaction products, which are bridging the radiolabel and the target directed entity.

The term "tetrazine ligation" refers to a class of click reactions that is fast, simple to use, versatile, chemoselective, and give high product yields. These reactions have found application in a wide variety of research areas, including materials science, polymer chemistry, and pharmaceutical sciences. Radiochemistry is one of the fields that showed the true potential of click chemistries as for example disclosed in Zeng et al, Journal of Nuclear Medicine, 54, 829-832, 2013. Essentially, the selectivity, ease, rapidity, and modularity of click ligations make them nearly ideally suited for the construction of radiotracers, a process that usually involves working with biomolecules in aqueous conditions with fast decaying radioisotopes.

The tetrazine ligation is characterized by the formation of covalent bonds between a 1,2,4,5-tetrazines (Tz) and typically a *trans*-cyclooctene (TCO). The reaction is initiated by an inverse electron-demand Diels-Alder reaction, followed by a retro-Diels-Alder reaction, driven by the expulsion of N₂. The tetrazine ligation is among the fastest known chemical ligations, with second order rate constants up to 10⁶ M⁻¹s⁻¹ in acetonitrile at 25 °C. This stands in contrast to other known click reactions, such as the Staudinger ligation (0.003 M⁻¹s⁻¹) and strain-promoted azide-alkyne cycloaddition (SPAAC) (0.1 M⁻¹s⁻¹). For this reason, and because of its specificity, the Tz ligation is ideally suited for synthon-based labeling. One major drawback of the conventional ligation of a Tz with a TCO is that the ligation takes place without any regio-specificity. Moreover, the conventional ligation of a Tz with a TCO gives rise to several tautomeric forms. This means that conventional ligation of a Tz with a TCO typically results in complex reaction mixtures of at least sixteen unique isomeric products (Scheme 1). Such mixtures of isomeric products cannot be applied directly for pharmaceutical purposes because the particular isomeric form of the compound influences the pharmacokinetics of the therapeutic agent and any potential toxicological effects of the individual isomeric forms cannot be distinguished. While the multiple dihydropyridazines that are obtained from conventional ligation may slowly auto-oxidize to the corresponding pyridazines over the course of several hours to several days, thereby slowly reducing the number of tautomeric forms of the products (WO2012/121746), the number of regio-isomeric and stereo-isomeric products are not reduced by this slow oxidation transformation. Means for speeding up the transformation from dihydropyridines to pyridazines have been described (Karaki Fumika et al, Tetrahedron, 97, 132411, 2021; Keinänen et al., ACS Medicinal Chemistry Letters, 7, 62-66, 2015), however, in none of reported cases was the oxidation completed within two hours, which is required if the pyridazines are to be used as radiopharmaceuticals. WO2017/059397, WO2020/242948, Syvänen et al., ACS Chemical Neuroscience, 11, 4460-4468, 2020, and WO2012/121746 discloses ligations between tetrazines and TCO's, which will inevitably provide several isomeric chemical entities.

Alternatively, pyridazines can be prepared via the ligation of a Tz to a strained cyclic alkyne, however this reaction suffers from slow second order rate constants.

Radiopharmaceuticals are increasingly used in theranostic, especially within oncology, both for diagnostic imaging and for targeted radionuclide therapy. Positron emission tomography (PET) is the gold standard in nuclear imaging with better resolution and quantification than other modalities. 2,200,800 clinical PET scans were performed in 2019 in the US alone. Targeted radionuclide therapy is more effective at treating cancer than many state-of-the-art chemotherapies. It also has the advantage over external beam radiotherapy (e.g. "gamma knife") in that it offers a way to confine the delivered dose to the tumor and its immediate surrounding area, which makes particular sense in the radiotherapy of micrometastatic disease. The combination of both diagnostic imaging and targeted radiotherapy can be used in "theranostics", a concept with powerful application in personalized medicine, with respect to patient selection, dose-finding and therapy response monitoring. A theranostic pair is two radionuclides, which can be substituted with each other, without changing the pharmacokinetics of the radiopharmaceutical, but shifting their application between diagnostic imaging and radionuclide therapy.

The two most widely used Diagnostic Imaging methods are the nuclear based PET and SPECT. Both methods rely on the combination of radionuclides with vectors that specifically target cancer cells. In imaging, such radiolabeled vectors are referred to as "radiotracers". Radiotracers are accumulated in tumor lesions, the location of which can then be visualized by detecting the emitted radiation. PET is strongly favored in oncology, while SPECT is dominant in cardiology and for producing bone scans and certain other specialized organ scans. Globally, the ratio of SPECT cameras to PET cameras used in hospitals is circa 5:1.
Single-photon emission computed tomography (SPECT) is the older of the two methods. SPECT imaging employs radionuclides emitting gamma photons, typically in the 100-200 keV range. A series of 2D projection images of radiotracer distribution in the body are acquired by one of more gamma cameras from multiple angles. These projection images are then assembled to produce a 3D image.
Positron emission tomography (PET) is currently considered the most advanced form of nuclear imaging. The key use of PET in oncology is diagnosis and treatment monitoring, especially of metastatic cancer. Compared to previous modalities, notably SPECT, PET offers improved resolution and sensitivity, and generally higher quality images. These properties are especially relevant in the detection, and subsequent treatment, of very small metastases. New innovations, notably total-body PET dramatically increase sensitivity and detects more metastases and are expected to further strengthen the advantageous position of PET in the modern clinic. PET relies on the use of radionuclides that emit positrons upon their decay. These positrons travel a limited distance, and then undergo annihilation with an electron in the surrounding medium. This produces two annihilation photons, each of 511 keV, which are emitted in opposite directions. These photons can be detected by a PET scanner. The most optimal radionuclide for PET is fluorine-18 (¹⁸F). With a decay half-life of 110 minutes and 97% positrons emitted per decay, ¹⁸F is close to ideal for clinical PET applications. This holds true especially for small molecular and peptide-based radiopharmaceuticals, which represent the vast majority of relevant PET tracers. Of equal importance, ¹⁸F can be practically produced in enormous quantities (>300 doses per production) on standard biomedical cyclotrons, which are readily available throughout most of the world, with more than 200 present in Europe alone. Accordingly, ¹⁸F does not share the concerns for sufficient supply associated with its closest competitor, the generator-produced radiometal gallium-68 (⁶⁸Ga). In addition, the lower positron energy of ¹⁸F provides higher resolution images. Compared to the current diagnostic radionuclide of most widespread use, the SPECT radionuclide technetium-99m, ¹⁸F offers the highest quality images through its status as a PET radionuclide. Accordingly, ¹⁸F is poised as the key diagnostic radionuclide of the future.
Radioactive iodine is widely used in SPECT imaging. Traditionally, iodine-131 (¹³¹I) was used, but nowadays, clinical SPECT scans are typically done with iodine-123 (¹²³I). This is due to the shorter half-life of ¹²³I (t½ = 13.2 hours) which together with its lack of beta minus emission offers a more favorable radiotoxicity profile than ¹³¹I. Its gamma photon energy of 159 keV is excellently suited for clinical SPECT imaging. Due to the intrinsic accumulation of iodine in the thyroid, ¹²³I in its free form is widely used for imaging thyroid disease. As a component of SPECT radiotracers, ¹²³I is for example used in the imaging agents MIBG (oncology) and ioflupane (CNS). ¹²³I forms a theranostic pair with the clinically used beta minus emitting therapeutic radionuclide ¹³¹I and the investigational Auger electron radiotherapeutic ¹²⁵I. Iodine-123 is produced in a cyclotron by proton irradiation of xenon in a capsule and is commercially available.
lodine-124 (¹²⁴I) can be used for PET imaging. It is usually produced in a cyclotron by bombardment of enriched tellurium-124. However, the imaging characteristics of ¹²⁴I are not ideal. It has a complex decay scheme with many high energy γ-emissions. Only 23% of its decay leads to positron emissions.
Astatine-211 (²¹¹At) is primarily a therapeutic nuclide, which emits alpha-particles upon decay. Alpha particles are absorbed in just 100 µm of tissue and cannot be detected by external scanners. However, one of the decay branches of ²¹¹At also generates X-rays in the range of 70-90 keV, which can be imaged with a gamma-camera or a SPECT scanner. Therefore, the distribution of ²¹¹At-labeled radiopharmaceuticals within the patient can be clinically assessed by SPECT imaging.

There are, however, challenges to introduce above mentioned radionuclides into molecules which limits the practical use of radionuclide-based therapy, diagnostic, and imaging.
Most of the PET, SPECT and therapeutic radionuclides mentioned above are radiometals. Conventional method of introducing radiometals into the vector molecules is the use of chelator groups that form coordinate bonds with the radiometal atom. The radiolabeling procedure typically involves mixing the radiolabeling precursor (vector with a chelator group attached) with radiometal ions and heating the mixture to allow the chelation reaction to proceed. Although chelation of radiometals is conceptually simple, it has a number of drawbacks, namely:
- the radiolabeled product often cannot be separated from the unlabeled precursor, because the difference in physico-chemical properties is not significant;
- chelation reaction is sensitive to trace metal impurities in solutions used for the radiolabeling, which makes upscaling problematic;
- heating, which is necessary to overcome the activation barrier of the chelation reaction, may degrade temperature-sensitive vectors.
Compared to its radiometal alternatives ¹⁸F is a halogen and requires covalent bonding to targeting vectors. This stands in the contrast to the chelator-based labelling techniques utilized for radiometals. Covalent bonds are currently typically formed via direct nucleophilic displacement of a leaving group, such as triflate. The conditions for such chemistry are harsh, lengthy and poorly scalable, and therefore incompatible with many vectors, notably the peptide class, which is growing in importance.
Small molecular radiopharmaceuticals containing radioiodine are typically prepared using either electrophilic destannylation or iodine-iodine exchange radiochemistry. The former is a mild, versatile and practical reaction, in which radioactive iodide is oxidized to a positively charged iodine species, which then replaces a leaving group, typically stannyl, in an aromatic substitution reaction. This reaction occurs at room temperature in often quantitative yield. Iodine-iodine isotopic exchange is used when high molar activity is not a concern and when substrates can withstand harsh conditions. The exchange occurs at elevated temperature with acid and copper as catalysts.
Like fluorine and iodine, astatine-211 is a halogen and can be attached to targeting vectors via covalent bonds. Aliphatic astatine-carbon bonds do not provide sufficient in vivo stability, whereby ²¹¹At is typically introduced onto aryl rings, forming astatoaryl moieties. Unlike iodine, astatine cannot be stably coupled to tyrosine residues of proteins: instead of binding to tyrosine, ²¹¹At has been found to form weak bonds with the sulfhydryl groups of cysteine. Therefore, ²¹¹At-labeling requires the synthesis of dedicated precursors exhibiting a suitable leaving group, such as trialkylstannyl, connected to an aryl ring. Standard ²¹¹At-labeling protocols use oxidation agents such as chloramine-T or *N*-chlorosuccinimide. Such agents can potentially degrade the biomolecules used as targeting vectors.

¹⁸F has long established itself as the best-in-class radionuclide for diagnostic PET imaging, while ²¹¹At is the most promising therapeutic radionuclide for alpha-therapy. Iodine radioisotopes ¹²³I, ¹²⁴I,¹²⁵I and ¹³¹I are useful for SPECT imaging, PET imaging, Auger therapy and beta-therapy, respectively. Moreover, all three elements can be introduced into aryl rings forming fluoro/iodo/astatoaryl moieties with maximum structural similarity, which is essential for theranostic pairs. Nevertheless, there are no reports of combined use of ¹⁸F with either ²¹¹At or radioiodine ¹²³I, ¹²⁴I, ¹²⁵I,¹³¹I, for theranostic applications.
One obstacle to the development of ¹⁸F/²¹¹At and ¹⁸F/¹²³I, ¹²⁴I, ¹²⁵I, ¹³¹I, theranostic pairs is the harsh labeling chemistry, as described above, which prevents direct regioselective labeling of biomolecular targeting vectors with these radionuclides. Another obstacle is that not all targeting vectors contain aryl moieties, so these must be introduced as prosthetic groups, also known as synthons.
Due to these challenges, synthon-based methods have been investigated for the preparation of radiopharmaceuticals. These methods involve the direct labeling of a separate intermediate compound ("synthon"), which is subsequently conjugated to the vector under mild conditions. Thus, the vector is not subjected to the harsh conditions of direct radiolabeling, although it does need to be modified by a chemical tag complementary to the radiolabeled synthon. What becomes crucial then, is the chemistry used to conjugate the synthon with the vector. This chemistry must be specific, compatible with pharmaceutically relevant aqueous media and high yielding, to avoid loss of radioactivity and minimize the need for subsequent purification. Critically, it must also be extremely fast, as procedures for preparing radiopharmaceuticals occur in nano- to micromolar concentrations and must proceed to completion or near completion within minutes, due to radionuclide decay. In light of that, click-chemistry has emerged as a strategic approach to radiolabel an array of targeting vectors such as mAbs, nanomedicines, peptides or small molecules.

Targeted radionuclide therapy (TRT) can be based on beta-emitters, Auger electron emitters and alpha-emitters.
Beta-particle emitting radionuclides (such as ⁹⁰Y, ¹⁷⁷Lu, ¹³¹I) decay via the emission of high-energy electrons (beta particles) which travel distances in the tissue of up to about 12 mm. The decay energy is deposited within the largest volume of tissue of the three therapy types mentioned here. Beta-emitters are thus suitable for the treatment of medium-sized tumors, where most of the dose will be absorbed by the cancer cells. For micrometastases or heterogeneous tumors however, even with perfect concentration of the radionuclide in and around the tumor, a large fraction of the irradiation dose is absorbed by surrounding healthy cells. Therefore, beta-emitters are not optimal for the treatment of micrometastases or heterogeneous tumors. This is an important drawback of beta-emitters, because micrometastases are one of the major causes of cancer recurrence and cancer mortality.
Alpha-emitters (such as ²²⁵Ac, ²¹¹At and ²¹²Pb) decay with the emission of alpha particles. Alpha-particles are much heavier than beta-particles, and their tracks are straight and short - on the order of 30-100 µm, in the order of the diameter of a handful of mammalian cells. Thus, all energy from a decay is delivered to just a few neighboring cells. Alpha-radiation possesses greater cytotoxicity, compared to beta-radiation and can be delivered to micro metastases in a highly focused manner.
In a telling example of the advantages of alpha therapy, a patient with metastatic prostate cancer resistant to the flagship beta-therapy agent ¹⁷⁷Lu-PSMA achieved complete remission after three cycles of the alpha-therapy agent ²²⁵Ac-PSMA.
Auger electron radiotherapy (AeRT) employs radionuclides that upon decay by electron capture (EC) or internal conversion (IC) emit a shower of extremely short ranged electrons. With advanced drug delivery technology, these specialized radionuclides can be delivered to the nuclei of cancer cells. Here, the emitted Auger electrons destroy the DNA and kill the cancer cells. Notably, the short range of the Auger electrons ensures that their energy is deposited mainly within the targeted cell, allowing for extremely localized therapy. Both ¹²³I and ¹²⁵I have high Auger electron yields and are suitable for AeRT.
lodine-131 (¹³¹I) is a beta particle emitter that is widely used in clinical radionuclide therapy. It has a decay half-life of 8.0 days and a main emission of beta particles with a maximal energy of 606 keV at 90% abundance. These beta-particles have a maximum range in tissue of about 2 mm, enabling ¹³¹I to treat small to medium sized tumor lesions. It is widely used for thyroid ablation due to its intrinsic accumulation in thyroid tissue. In addition, a therapeutic variant of MIBG is available, radiolabeled with ¹³¹I, and ¹³¹I is used in radioimmunotherapy. It forms theranostic pairs with ¹²³I (SPECT) and ¹²⁴I (PET). Both iodine-123 and iodine-125 have substantial emission of Auger electrons, about 10 and 20 electrons, respectively. This makes them suitable for Auger electron radiotherapy, a currently investigational form of radionuclide therapy.
Astatine-211 is an alpha-emitting radionuclide with a half-life of 7.2 hours. Unlike most other alpha-emitters used for targeted alpha-therapy, ²¹¹At yields one α-particle per decay chain, which offers a number of translational advantages. First, there are limited toxic side-effects from radioactive daughter nuclides, which are released from the targeting vector as a result of the initial decay. Second, radiation dosimetry calculations are simplified. Moreover, due to the relatively short half-life of ²¹¹At (for a therapeutic nuclide), enhanced control of the radiation dose delivered to patients is possible.
Phase I/II Clinical studies with tumor-targeting ²¹¹At-labeled radiopharmaceuticals showed low acute toxicity and absence of radiation side-effect. Preclinical studies showed efficient tumor eradication by ²¹¹At in animal models. Hence, ²¹¹At is a highly promising therapeutic radionuclide.

However, in order for click-chemistry such as the Tz-TCO ligation to furnish end-products that are viable in a regulatory and commercial context, it is desirable to reduce the number of ligation products produced. The presently available methods yields a large number of isomeric products that are impossible to separate, barring it from clinical translation due to toxicity concerns and unpredictable pharmacokinetics. Moreover, a limitation to practical applicability of radiolabeled products is that the radiolabeled compound will have to be administered to a patient within a very limited time period from production for use in imaging, therapy or diagnostics. Therefore, any process that reduces the ligation step would be a great benefit, particularly when radioactive agents are used as labeling agents, diagnostic agents, therapeutic agents, theranostic agents or when used in combination with other agents.
The present invention provides a rapid method for providing labeled pyradizine products wherein the ligation step is less than 180 minutes and wherein a pyradizine in one form or only a few forms are provided. This means that complexity of the inverse-electron demand Diels-Alder reaction is reduced, which ease the clinical translation and production of these compounds. The method advantageously enables radiolabeling, for example with ¹⁸F, ¹²³I, ¹²⁴I, ¹²⁵I or ¹³¹I, and ²¹¹At, of any tracer in unmatched efficiency and practicality.

### SUMMARY

The present invention provides a method for rapid oxidation of dihydropyridazines to pyridazines. The method applies to dihydropyridazine mixture that arises from the click reaction between a first chemical entity which is either a 1,2,4,5-tetrazine or a strained alkene having inverse electron demand Diels-Alder cycloaddition reactivity and a second chemical entity which is either a 1,2,4,5-tetrazine or a strained alkene having complementary inverse electron demand Diels-Alder cycloaddition reactivity. Either the first or the second chemical entity is conjugated to a label and the other is conjugated to a targeting vector. The ligation between the first chemical entity and the second chemical entity is followed by a rapid acid catalyzed oxidation. The advantage of the method is that the oxidation of dihydropyridazines to pyridazines is provided within a minimum period of time and the complexity of the reaction mixture is highly reduced, thereby easing clinical translation and production costs.

The method for providing for rapid oxidation of dihydropyridazines to pyridazines comprises the following steps:
a) labeling a first chemical entity having inverse electron demand Diels-Alder cycloaddition reactivity and being conjugated to a pharmaceutic agent, a diagnostic agent, or a therapeutic agent, with a labeling agent; wherein the first chemical entity is a 1,2,4,5-tetrazine or a dienophile selected from the group consisting of a cyclic unsaturated hydrocarbon with at least one double bond in the *trans*-configuration; an unsaturated heterocycle, with at least one double bond in the *trans*-configuration; and
b) ligating the labeled first chemical entity obtained in step a) with a second chemical entity having complementary inverse electron demand Diels-Alder cycloaddition reactivity and being conjugated to a targeting vector; wherein the second chemical entity is a 1,2,4,5-tetrazine or a dienophile selected from the group consisting of an cyclic unsaturated hydrocarbon, with at least one double bond in the *trans*-configuration; an unsaturated heterocycle, with at least one double bond in the *trans*-configuration; and
c) oxidizing the resulting mixture of dihydropyridazines labeled targeting vector obtained from step b) in a solvent at a temperature ranging from 15 °C to 50 °C for up to 180 minutes, by addition of an acid catalyst, wherein the acid catalyst has a pKa of 5 or below in H₂O at 25 °C, at a concentration of 0.1 M - 5 M to the mixture obtained from step b), and an oxidant selected from quinone-based oxidants, Bobbits salt-based oxidants, peroxide-based oxidants, peroxy acid-based oxidants, UV-light with a minimum UV irradiance of 2 mW/cm².

This labeling agent can be any agent that is useful as a marker, an imaging agent, a therapeutic agent or a theranostic agent and includes radionuclides and fluorescent entities.

The targeting vector can be any suitable vector directed at a specific target and includes antibodies, nanobodies, polymers, nanomedicines, cells, proteins, peptides, and small molecules.

The present invention moreover provides for use of the labeled pyridazines conjugated targeting vectors obtained by the method in theranostic, therapy, radiotherapy, diagnostic and imaging.

### BRIEF DESCRIPTION OF DRAWINGS

Figure 1: Reaction scheme of ligated products tested for oxidation with different chemical oxidants and TFA, and table identifying the specific compounds and the results obtained.
Figure 2: Reaction scheme of ligated products tested for oxidation with UV-light or air as oxidant under different conditions (con. 1 - con. 5) and table identifying the specific compounds and the time (minutes) to obtain an oxidation efficiency of ≥90%.
Figure 3: Reaction scheme of ligated products tested for oxidation with UV-light or air as oxidant under different conditions (con. 6 - con. 11) and table identifying the specific compounds and the time (minutes) to obtain an oxidation efficiency of ≥90%.
Figure 4: Reaction scheme of specific ligated products tested for oxidation with chemical oxidants or UV-light under different conditions (con. 3 - con. 13) and table showing the time (minutes) to obtain an oxidation efficiency of ≥90%.
Figure 5: Reaction scheme of a specific ligated product tested for oxidation with UV-light under different conditions (con. 14 - con. 21) and table showing the time (minutes) to obtain an oxidation efficiency of ≥90%.
Figure 6: Reaction scheme of ligated products tested for oxidation with UV-light under different conditions (con. 21 - con. 22) and table identifying the specific compounds and the time (minutes) to obtain an oxidation efficiency of ≥90%.
Figure 7: Reaction scheme of ligated products tested for oxidation under different conditions (con. 6 - con. 11) and table identifying the specific compounds and the time (minutes) to obtain an oxidation efficiency of ≥90%.
Figure 8: Diagram showing the time (minutes) to obtain an oxidation efficiency of ≥90% for the F-labeled click-reaction shown above the diagram under various conditions.
Figure 9: Diagram showing the time (minutes) to obtain an oxidation efficiency of ≥90% for the I-labeled click-reaction shown above the diagram under various conditions.
Figure 10: Reaction scheme of ligated products tested for oxidation under different conditions (con. 1 - con. 12) and table identifying the specific compounds and the time (minutes) to obtain an oxidation efficiency of ≥90%.
Figure 11: Reaction scheme of ligated products tested for oxidation under different conditions (con. 1 - con. 4) and table identifying the specific compounds and the time (minutes) to obtain an oxidation efficiency of ≥90%.

### DEFINITIONS

Container refers to the entity wherein a chemical reaction, such as an oxidation step, takes place. The container is in the present context laboratory glassware such as vials. The vial can be made of borosilicate, quartz or other suitable material.

Labeling agent refers herein to an imaging agent that has been attached to a chemical entity having inverse electron demand Diels-Alder cycloaddition reactivity and being conjugated to a pharmaceutic agent, a diagnostic agent, or a therapeutic agent. The pharmaceutic agent, diagnostic agent, or therapeutic agent that the chemical entity is conjugated to is in some embodiments identical to the labeling agent. This may for instance be the case when the labeling agent is an agent that can be applied both as a label and as a therapeutic or diagnostic agent, such as the diagnostic imaging agent ¹⁸F. Labeling agents include radionuclides. However, since labeling of a diene or dienophile with a radionuclide will normally not provide 100% labeling efficiency with the radionuclide, some of the products labeled will inevitably be labeled with a stable isotope of the corresponding radionuclide element.

Ligating refers in the present context to a click reaction between the a first chemical entity having inverse electron demand Diels-Alder cycloaddition reactivity with a second chemical entity having complementary inverse electron demand Diels-Alder cycloaddition reactivity.

A "first chemical entity" and a "second chemical entity" refers to a pair of compounds having complementary inverse electron demand Diels-Alder cycloaddition reactivity. This pair is a 1,2,4,5-tetrazine and a dienophile being either a cyclic unsaturated hydrocarbon with at least one double bond in the *trans*-configuration or an unsaturated heterocycle, with at least one double bond in the *trans*-configuration. Either the 1,2,4,5-tetrazine or the dienophile is labeled with a labeling agent and conjugated to a pharmaceutic agent, a diagnostic agent or a therapeutic agent, whereas the other is conjugated to a targeting vector. Accordingly, in some embodiments, the "first chemical entity" is a 1,2,4,5-tetrazine and the "second chemical entity" is a dienophile being either a cyclic unsaturated hydrocarbon with at least one double bond in the *trans*-configuration or an unsaturated heterocycle, with at least one double bond in the *trans*-configuration. In other embodiments, the "first chemical entity" is a dienophile being either a cyclic unsaturated hydrocarbon with at least one double bond in the *trans*-configuration or an unsaturated heterocycle, with at least one double bond in the *trans*-configuration and the "second chemical entity" is a 1,2,4,5-tetrazine.

Solvent is in the present context a mixture of water and water miscible substances.

### DETAILED DESCRIPTION OF THE INVENTION

The present invention provides in a first aspect a method for the rapid oxidation of dihydropyridazines to pyridazines. The method applies to the oxidation of the various dihydropyridazines, that arise from the ligation of 1,2,4,5-tetrazines with a cyclic unsaturated hydrocarbon, with at least one double bond in the *trans-*configuration or an unsaturated heterocycle, with at least one double bond in the *trans*-configuration. The rapid oxidation is achieved by applying the combination of an acid with various oxidants, including chemical oxidants, as well as UV-light catalyzed oxidations.

Either the tetrazine or the strained alkene is conjugated to an agent of interest such as a pharmaceutic agent, a diagnostic agent, an imaging agent, or a therapeutic agent and labeled with a labeling agent. The compatible diene or dienophile, respectively, is conjugated to a targeting vector of interest.

The ligation between the tetrazine and the strained alkene is based in inverse electron demand Diels-Alder cycloaddition reactivity, and accordingly, the tertrazine and the strained alkene must have complementary inverse electron demand Diels-Alder cycloaddition reactivity. The ligation between the tetrazine and strained alkene as specified will have reaction kinetics with a minimum second order rate constant of 250 M⁻¹ s⁻¹ in PBS at 25 °C as determined by stopped-flow spectrophotometry. Ligating the tetrazines and strained alkenes specified here ensures that ligations with second order rate constants below 250 M⁻¹ s⁻¹ in phosphate-buffered saline (PBS) at 25 °C are avoided. Ligations with second order rate constants below 250 M⁻¹ s⁻¹ in phosphate-buffered saline (PBS) at 25 °C will take too long time to provide the labeled targeting vectors because the radioactive labeling agent often have a limited period of time for use on imaging and/or therapy after the ligation step.

Second order rate constant can be measured by different means, but is typically measured by stopped flow spectrophotometry as for example described in (Chance, Rev. Sci. Instrum. 1951, 22, 619- 627). Herein, the method described in Battisti et al. J. Med. Chem. 2021, 64, 20, 15297-15312 was applied.

In order to rapidly provide a labeled chemical entity or targeting vector, with improved homogeneity, that will bear a pyridazine, derived from the ligation between a tetrazine and a strained alkene, the resulting complex mixture of various dihydropyridazines will have to be oxidized.
It is shown herein that under specific conditions, the oxidation, either via chemical or photochemical means is rapidly accelerated with the addition of an acid. It is shown herein that not all oxidants are suitable for this method either because the oxidation is not efficient enough when working within the required time frame available or give side products or because the oxidation impacts on the structure of the targeting vector, thereby potentially preventing the labeled targeting vector from binding to its target. The oxidation efficiency of the present method oxidation step c) is at least 90% i.e., at least 90% of the labeled and clicked targeting vector will be in the pyridazine form after the oxidation step. Oxidation conditions providing less than 90% of the product would not be of sufficient purity for use in therapy/imaging/diagnosis and it would require additional toxicological studies and/or purification procedures.

It is shown herein that a suitable oxidation is performed in a solvent at a temperature ranging from 15 °C to 50 °C for up to 180 minutes by addition of an acid catalyst, wherein the acid catalyst has a pKa of 5 or below in H₂O at 25 °C, at a concentration of 0.1 M - 5M in combination with an oxidant when the oxidant is a quinone-based oxidant, Bobbits salts based oxidant, peroxide-based oxidant, peroxy acid-based oxidant or UV-light with a minimum UV irradiance of 2 mW/cm². When UV-light is used as oxidant a photosensitizer can optionally be included in the oxidation step for further accelerating the reaction. Photosensitizers are dependent on the wavelength of the UV light applied and accordingly, the photosentiziser should be selected in accordance with the wavelength of the UV applied in the oxidation step. It is commonly known which photosentizisers that are suitable at a given wavelength and it is expected that the oxidation step c) in the present method can be performed at UV-light wavelengths from 200 - 400 nm and the photosentiziser can be selected accordingly. In a preferred embodiment, the oxidation in step c) is performed at 200 - 300 nm, such as 200 - 350 nm, 250 - 350 nm, 250- 300 nm or such as at 254 nm. Preferred photosensitizers when UV-light at a wavelength of 200 - 350 nm such as at 254 nm is applied are Fluoroscein-based or Porphyrin-based photosensitizers such as fluorescein or substituted 4,4',4"-(20-phenylporphyrin-5,10,15-triyl)tribenzenesulfonic acids or substituted 4,4',4"-(20-phenylporphyrin-5,10,15-triyl)tris(1-methylpyridin-1-ium)s.

The term acid applied in relation to catalyze the oxidation in step c), refers to any molecule that has a proton with a pKa of at least 5 or lower, as measured in H₂O at 25 °C. Suitable acids have a pKa of -10 to 5, such as -8 to 3, -8 to 1, -6 to 1, -4 to 1, -2 to 1 or acids that have a pKa such as -10 to 2, -9 to 1, -8 to 0, -10 to -2, -10 to -4, -10 to -6 or such as acids that have a pKa of -8 to -10. The lower the pKa, the better in terms of reaction rate. Thus, in a preferred embodiment, the acids have a pKa of - 10 to 0 such as -10 to -8, -10 to -6, -10 to -4, -10 to -2 and -8 to 0.
Additionally, the concentration of acid in the solvent has to be 0.1 M - 5 M, such as 0.5-5 M, 1 - 5 M, 1.5-5 M, 2-5 M, 2.5-5 M, 3-5 M, 3.5-5 M, 4-5 M or 4.5-5 M. In a preferred embodiment, the concentration of acid in the solvent is 0.5 - 3 M, such as 0.5 - 2 M, such as 1 M.

Accordingly, the method for rapid oxidation of dihydropyridazines to pyridazines: comprises:
a) labeling a first chemical entity having inverse electron demand Diels-Alder cycloaddition reactivity and being conjugated to a pharmaceutic agent, a diagnostic agent or a therapeutic agent, with a labeling agent; wherein the first chemical entity is a 1,2,4,5-tetrazine or a dienophile selected from the group consisting of a cyclic unsaturated hydrocarbon with at least one double bond in the *trans*-configuration; an unsaturated heterocycle, with at least one double bond in the *trans*-configuration; and
b) ligating the labeled first chemical entity obtained in step a) with a second chemical entity having complementary inverse electron demand Diels-Alder cycloaddition reactivity and being conjugated to a targeting vector; wherein the second chemical entity is a 1,2,4,5-tetrazine or a dienophile selected from the group consisting of an cyclic unsaturated hydrocarbon, with at least one double bond in the *trans*-configuration; an unsaturated heterocycle, with at least one double bond in the *trans*-configuration; and
c) oxidizing the resulting mixture of dihydropyridazines labeled targeting vector obtained from step b) in a solvent at a temperature ranging from 15 °C to 50 °C for up to 180 minutes, by addition of an acid catalyst, wherein the acid catalyst has a pKa of 5 or below in H₂O at 25 °C, at a concentration of 0.1 M - 5 M to the mixture obtained from step b), and an oxidant selected from quinone-based oxidants, Bobbits salt-based oxidants, peroxide-based oxidants, peroxy acid-based oxidants,UV-light with a minimum UV irradiance of 2 mW/cm².

The pharmaceutic agent, diagnostic agent, or therapeutic agent that the first chemical entity is conjugated to is in some embodiments identical with the labeling agent. This may for instance be the case when the labeling agent is an agent that can be applied both as a label and as a therapeutic or diagnostic agent.

In some embodiments, the labeling agent is a radionuclide. Some radionuclides can be applied both in imaging, in diagnostics and/or in therapy and in the present examples, the same radionuclide have been applied as labeling agent as well as imaging or therapeutic agent. Labeling of a diene or dienophile with a radionuclide will normally not provide 100% labeling efficiency with the radionuclide, some of the products labeled will inevitably be labeled with a stable isotope of the corresponding radionuclide element.

The method enables labeling such as radiolabeling, for example with ¹⁸F, radioiodine (¹²³I, ¹²⁴I, ¹²⁵I or ¹³¹I) and ²¹¹At, of any targeting vector in unmatched efficiency and practicality. The ground-breaking nature of the method is the possibility of forming an end-product with improved homogeneity, within 180 minutes, often within 60 minutes, such as within 1-20 minutes, and thereby easing clinical translation. In contrast, conventional tetrazine ligations result in multiple products and as a result need massive and unmanageable toxicological packages or tedious and time-consuming separation.

The pyridazine-containing targeting vectors obtainable by the method according to the present invention, can be applied for various purposes depending on the characteristics of the agent applied as a label. Labeling agents that are suitable for the method includes radiolabels and fluorescent labels.

In a preferred embodiment, the labeling agent applied in step a) in the method for providing a labeled pyridazine-containing targeting vector is a radionuclide or a stable isotope of a corresponding element. The characteristics and accordingly the use of the different radionuclides normally applied are well known in the art.

Radionuclide labeling agents and stable isotopes of a corresponding element that are suitable for use as a labeling agent in step a) in the method for providing a labeled pyridazine-containing targeting vector includes: ¹H, ²H, ³H, ¹¹C, ¹²C, ¹³C, ¹⁴C ¹³N, ¹⁴N, ¹⁵N ¹⁸F, ¹⁹F, ¹²³I, ¹²⁴I, ¹²⁵I, ¹²⁷I, ¹³¹I, ¹⁵O, ¹⁶O, ¹⁷O, ¹⁸O, ⁴³Sc, ⁴⁴Sc, ⁴⁵Sc, ⁴⁵Ti, ⁴⁶Ti, ⁴⁷Ti, ⁴⁸Ti, ⁴⁹Ti, ⁵⁰Ti, ⁵⁵Co, ⁵⁸mCo, ⁵⁹Co, ⁶⁰Cu, ⁶¹Cu, ⁶³Cu, ⁶⁴Cu, ⁶⁵Cu, ⁶⁷Cu, ⁶⁷Ga, ⁶⁸Ga, ⁶⁹Ga, ⁷¹Ga, ⁷⁶Br, ⁷⁷Br, ⁷⁹Br, ⁸⁰mBr, ⁸¹Br, ⁷²As, ⁷⁵As, ⁸⁶Y, ⁸⁹Y, ⁹⁰Y, ⁸⁹Zr, ⁹⁰Zr, ⁹¹Zr, ⁹²Zr, ⁹⁴Zr, ¹⁴⁹Tb, ¹⁵²Tb, ¹⁵⁹Tb, ¹⁶¹Tb, ¹¹¹In, ¹¹³In, ¹¹⁴mIn, ¹¹⁵mIn, ¹⁷⁵Lu, ¹⁷⁷Lu, ¹⁸⁵Re, ¹⁸⁶Re, ¹⁸⁸Re, ²⁰¹Tl, ²⁰³Tl, ²⁰⁵Tl, ²⁰⁶Pb, ²⁰⁷Pb, ²⁰⁸Pb, ²¹²Pb, ²⁰⁹Bi, ²¹²Bi, ²¹³Bi, ³¹P, ³²P, ³³P, ³²S, ³⁵S ⁴⁵Sc, ⁴⁷Sc, ⁸⁴Sr, ⁸⁶Sr, ⁸⁷Sr, ⁸⁸Sr, ⁸⁹Sr, ¹⁶⁵Ho, ¹⁶⁶Ho, ¹⁵⁶Dy, ¹⁵⁸Dy, ¹⁶⁰Dy, ¹⁶¹Dy, ¹⁶²Dy, ¹⁶³Dy, ¹⁶⁴Dy, ¹⁶⁵Dy , ²²⁷Th, ²³²Th, ⁵¹Cr, ⁵²Cr, ⁵³Cr, ⁵⁴Cr, ⁷³Se, ⁷⁴Se, ⁷⁵Se, ⁷⁶Se, ⁷⁷Se, ⁷⁸Se, ⁸⁰Se, ⁸²Se, ⁹⁴Tc, ^{99m}Tc , ¹⁰³Rh,¹⁰³mRh, ¹¹⁹Sb, ¹²¹Sb, ¹²³Sb, ¹³⁵La, ¹³⁸La, ¹³⁹La, ¹⁶²Er, ¹⁶⁴Er, ¹⁶⁵Er, ¹⁶⁶Er, ¹⁶⁷Er, ¹⁶⁸Er, ¹⁷⁰Er, ¹⁹³mPt, ¹⁹⁵mPt, ¹⁹²Pt, ¹⁹⁴Pt, ¹⁹⁵Pt, ¹⁹⁶Pt, ¹⁹⁸Pt, ²¹¹At, ²²³Ra, ²²⁵Ac.
In a preferred embodiment, the radionuclide labeling agents is selected from the group consisting of: ¹¹C, ¹³N, ¹⁵O, ¹⁸F, ⁴³Sc, ⁴⁴Sc, ⁴⁵Ti, ⁵⁵Co, ⁶⁰Cu, ⁶¹Cu, ⁶⁴Cu, ⁶⁸Ga, ⁷⁶Br, ⁷²As, ⁸⁶Y, ⁸⁹Zr, ⁹⁰Y, ¹⁴⁹Tb, ¹⁵²Tb; and the stable isotopes of the corresponding element is selected from the group consisting of: ¹²C, ¹³C,¹⁴N, ¹⁵N, ¹⁶O, ¹⁷O, ¹⁸O, ¹⁹F, ⁴⁵Sc, ⁴⁶Ti, ⁴⁷Ti, ⁴⁸Ti, ⁴⁹Ti, ⁵⁰Ti, ⁵⁹Co, ⁶³Cu, ⁶⁵Cu, ⁶⁹Ga, ⁷¹Ga, ⁷⁵As, ⁷⁹Br, ⁸¹Br, ⁸⁹Y, ⁹⁰Zr, ⁹¹Zr, ⁹²Zr, ⁹⁴Zr, ¹⁵⁹Tb. These radionuclides and their stable isotopes of the corresponding elements are particularly useful in Positron Emission Tomography (PET).

In another preferred embodiment, the radionuclide labeling agents is selected from the group consisting of: ⁶⁴Cu, ⁶⁷Cu, ⁶⁷Ga, ¹¹¹In, ¹³¹I, ¹⁷⁷Lu, ¹⁸⁶Re, ²⁰¹Tl, ²¹²Pb, ²¹³Bi; and the stable isotope of the corresponding element is selected from the group consisting of: ⁶³Cu, ⁶⁵Cu, ⁶⁹Ga, ⁷¹Ga, ¹¹³In, ¹²⁷I, ¹⁷⁵Lu, ¹⁸⁵Re, ²⁰³Tl, ²⁰⁵Tl, ²⁰⁶Pb, ²⁰⁷Pb, ²⁰⁸Pb, ²⁰⁹Bi. These radionuclides and their stable isotopes of the corresponding elements are particularly useful in Single Photon Emission Computed Tomography (SPECT).

In another preferred embodiment, the radionuclide labeling agents is selected from the group consisting of: ³²P, ³³P, ⁴⁷Sc, ⁶⁴Cu, ⁶⁷Cu, ⁸⁹Sr, ⁹⁰Y, ¹⁶⁶Ho, ¹⁶¹Tb, ¹⁶⁵Dy, ¹⁷⁷Lu, ¹⁸⁶Re, ¹⁸⁸Re; and the stable isotope of the corresponding element is selected from the group consisting of: ³¹P, ⁴⁵Sc, ⁶³Cu, ⁶⁵Cu, ⁸⁴Sr, ⁸⁶Sr, ⁸⁷Sr, ⁸⁸Sr, ⁸⁹Y, ¹⁶⁵Ho, ¹⁵⁹Tb, ¹⁵⁶Dy, ¹⁵⁸Dy, ¹⁶⁰Dy, ¹⁶¹Dy, ¹⁶²Dy, ¹⁶³Dy, ¹⁶⁴Dy, ¹⁷⁵Lu, ¹⁸⁵Re. These radionuclides are beta-particle emitters and these radionuclides along with their stable isotopes of the corresponding element are applied in therapy for instance in relation to the treatment of various tumorous diseases.

In another preferred embodiment, the radionuclide labeling agents is selected from the group consisting of: ¹⁴⁹Tb, ²¹²Pb, ²¹²Bi, ²¹³Bi, ²²⁷Th; and the stable isotope of the corresponding element is selected from the group consisting of: ¹⁵⁹Tb, ²⁰⁶Pb, ²⁰⁷Pb, ²⁰⁸Pb, ²⁰⁹Bi, ²³²Th. These radionuclides are alpha-particle emitters and these radionuclides along with their stable isotopes of the corresponding element are applied in therapy for instance in relation to the treatment of various tumorous diseases.

In another preferred embodiment, the radionuclide labeling agents is selected from the group consisting of: ⁵¹Cr, ⁵⁸mCo, ⁶⁴Cu, ⁶⁷Ga, ⁷³Se, ⁷⁵Se, ⁷⁷Br, ⁸⁰mBr, ⁹⁴Tc, ^{99m}Tc, ¹⁰³mRh, ¹¹¹In, ¹¹⁴mIn, ¹¹⁵mIn, ¹¹⁹Sb, ¹²³I, ¹²⁴I, ¹²⁵I, ¹³⁵La, ¹⁶⁵Er, ¹⁹³mPt, ¹⁹⁵mPt; and the stable isotope of the corresponding element is selected from the group consisting of: ⁵²Cr, ⁵³Cr, ⁵⁴Cr, ⁵⁹Co, ⁶³Cu, ⁶⁵Cu, ⁶⁹Ga, ⁷¹Ga, ⁷⁴Se, ⁷⁶Se, ⁷⁷Se, ⁷⁸Se, ⁸⁰Se, ⁸²Se, ⁷⁹Br, ⁸¹Br, ¹⁰³Rh, ¹¹³In, ¹²¹Sb, ¹²³Sb, ¹²⁷I, ¹³⁸La, ¹³⁹La, ¹⁶²Er, ¹⁶⁴Er, ¹⁶⁶Er, ¹⁶⁷Er, ¹⁶⁸Er, ¹⁷⁰Er, ¹⁹²Pt, ¹⁹⁴Pt, ¹⁹⁵Pt, ¹⁹⁶Pt, ¹⁹⁸Pt. These radionuclides emit electrons via the Auger effect with low kinetic energy. These radionuclides along with their stable isotopes of the corresponding element are applied in Auger therapy for instance in relation to highly targeted treatment of various tumorous diseases.

In another preferred embodiment, the radionuclide labeling agents is selected from the group consisting of: ³H, ¹⁴C and ³⁵S and the stable isotope of the corresponding element is selected from the group consisting of: ¹H, ²H, ¹²C, ¹³C, ³²S. These radionuclides are applied to in vitro studies such as displacement and tritiation assays.

In another preferred embodiment, the radionuclide labeling agents is selected from the group consisting of: ¹¹C, ¹³N, ¹⁸F, ¹²³I, ¹²⁵I, ¹³¹I, or ²¹¹At; and the stable isotope of the corresponding element (when such a stable isotope of the element is obtainable) is selected from the group consisting of: ¹²C, ¹⁴N, ¹⁹F, ¹²⁷I. These radionuclides along with their stable isotopes of the corresponding element if available, are among the most frequently used radionuclides in therapy and imaging presently.

The targeting vector that is conjugated to either the tetrazine or to the cyclic unsaturated hydrocarbon, with at least one double bond in the *trans*-configuration or an unsaturated heterocycle, with at least one double bond in the *trans*-configuration mentioned in step b) in the for providing a labeled pyridazine-containing targeting vector can be any kind of targeting vector that is suitable for use in therapy, imaging, or diagnostics. Such commonly used targeting vectors that are suitable in the present method include antibodies, nanobodies, polymers, nanomedicines, cells, proteins, peptides, and small molecules.

Commonly applied targeting vectors, that are suitable in the present method includes: peptides such as Octreotide, Octreotate, AE105; small molecules such as FAPI derivatives and PSMA derivatives.

In a preferred embodiment, the targeting vector applied in the method for providing a labeled pyridazine-containing targeting vector is selected from the group comprising: Octreotide derivatives, Octreotate derivatives, AE105 derivatives, FAPI derivatives and PSMA derivatives.

Ligation of the compounds subject to step a) and step b) in the method will, due to their structure, occur at a minimum second order rate constant of 250 M⁻¹ s⁻¹ in phosphate-buffered saline (PBS) at 25 °C, determined by stopped-flow spectrophotometry.

The oxidizing step c) in the method for providing a pyridazine-containing targeting vectors is carried out at a certain temperature and time, by adding a specific oxidant to the mixture of ligated compounds obtained in step b). These conditions ensure that the efficiency of the oxidation step is ≥90%, thereby meeting the speed required for therapeutic, diagnostic or imaging use of the labeled pyridazine-containing targeting vector.

The time required to obtain an oxidation efficiency of >_90% depends on the specific compound being oxidized, temperature, oxidant equivalents and on the oxidation agent. With the conditions applied in the present method, the >_90% oxidation efficiency will be obtained within 180 minutes, such as from 0 - 160 minutes, from 0 - 120, from 0 - 90 minutes, from 0 - 60 minutes, from 0 - 50 minutes, from 0 - 40 minutes, from 0 - 30 minutes, from 0 - 20 minutes, from 0-10 minutes, or from 0 - 5 minutes. In a preferred embodiment, the oxidation efficiency obtained is >_90% in 0 - 20 minutes.

The temperature for the oxidation step is 15 °C - 50 °C, such as 15 °C - 45 °C, 15 °C - 40°C, 15 °C - 35 °C, 20 °C - 30 °C, or at approximately 20 °C - 25 °C. The preferred temperature is room temperature such as between 20 °C - 25 °C.

All oxidations should be catalyst by addition of an acid. In one embodiment, the oxidant should be a chemical oxidant should be a quinone-based oxidant, selected from quinone-based oxidants, Bobbits salt-based oxidants, peroxide-based oxidants, peroxy acid-based oxidants. It has surprisingly been found herein, that using other types of oxidants will not provide the desired pyridazine-containing targeting vector or will negatively impact the structure of the targeting vector. The acid and the chemical oxidant are added to the complex mixture of the various dihydropyridazines, resulting from the ligation of a tetrazine and a strained alkene obtained from step b) in the method for providing a labeled pyridazine-containing targeting vector from 1 to 100 equivalents of the product obtained in step b), such as from 10 to 90, 20 to 80, 30 to 70, 40 to 60 or 50 equivalents of the product obtained in step b). Preferably 1 to 10, such as 1 equivalent oxidant is added to the labeled compound obtained from step b).

In another embodiment, the oxidation should be a photochemically catalyzed by UV-light (with a minimum UV irradiance of 2 mW/cm²), in the presence of an acid. The acid is added to the complex mixture of the various dihydropyridazines, resulting from the ligation of a tetrazine and a strained alkene obtained from step b) and is exposed to UV-light, such as from 200 to 400 nm, 200 to 280 nm (UVC), 280 to 315 nm (UVB), 315 to 400 (UVA). Preferably 254 nm.

In another embodiment, the oxidation should be a photochemically catalyzed by UV-light (with a minimum UV irradiance of 2 mW/cm²) or by UV-light (with a minimum UV irradiance of 2 mW/cm²) with the addition of a photosensitizer, such as fluoroscein-based or porphyrin-based photosensitizers, in the presence of an acid. The acid is added to the complex mixture of the various dihydropyridazines, resulting from the ligation of a tetrazine and a strained alkene obtained from step b) and is exposed to UV-light, such as from 200 to 400 nm, 200 to 280 nm (UVC), 280 to 315 nm (UVB), 315 to 400 (UVA). Preferably at 254 nm.

In another preferred embodiment, the oxidation in step c) is photochemically catalyzed by UVC-light with UV irradiance such as 2 to 50 mW/cm², 2 to 40 mW/cm², 2 to 20 mW/cm², 2 to 15 mW/cm², 5 to 15 mW/cm², 10 to 15 mW/cm², Preferably 12 mW/cm².

In a preferred embodiment, the method for oxidation of dihydropyridazines to pyridazines comprises:
a) labeling a first chemical entity having inverse electron demand Diels-Alder cycloaddition reactivity and being conjugated to a pharmaceutic agent, a diagnostic agent or a therapeutic agent, with a labeling agent; wherein the first chemical entity is a 1,2,4,5-tetrazine or a dienophile selected from the group consisting of a cyclic unsaturated hydrocarbon with at least one double bond in the *trans*-configuration; an unsaturated heterocycle, with at least one double bond in the *trans*-configuration; and
b) ligating the labeled first chemical entity obtained in step a) with a second chemical entity having complementary inverse electron demand Diels-Alder cycloaddition reactivity and being conjugated to a targeting vector; wherein the second chemical entity is a 1,2,4,5-tetrazine or a dienophile selected from the group consisting of an cyclic unsaturated hydrocarbon, with at least one double bond in the *trans*-configuration; an unsaturated heterocycle, with at least one double bond in the *trans*-configuration, and
c) oxidizing the resulting mixture of dihydropyridazines labeled targeting vector obtained from step b) in a solvent at a temperature ranging from 15 °C to 50 °C for up to 180 minutes, by addition of an acid catalyst, wherein the acid catalyst has a pKa of 5 or below in H₂O at 25 °C, at a concentration of 0.1 M - 5M to the mixture obtained from step b), and an oxidant selected from quinone-based oxidants, Bobbits salt-based oxidants, peroxide-based oxidants, peroxy acid-based oxidants, UV-light with a minimum UV irradiance of 2 mW/cm², with the proviso that when the oxidant is UV-light, and the tetrazine in step a) or b) is a bispyridyl tetrazine, then the oxidation must be made in a quartz container.

When the oxidation in step c) is photochemically catalyzed by UV-light with a minimum UV irradiance of 2 mW/cm² or by UV-light with a minimum UV irradiance of 2 mW/cm² with the addition of a photosensitizer, it was found that the solvent may influence on the oxidation rate and thus on the time required for completing the oxidation by up to at least 90%. The more water that is present in the solvent, the faster the oxidation rate. Accordingly, in a preferred embodiment, the solvent in step c) comprises 5 - 95% water, such as 20 - 95%, 30 - 95%, 40 - 95%, 50 - 95%, 60 - 95%, 70 - 95%, 80 - 95%, or 90 - 95% water when the oxidant in step c) is UV-light with a minimum UV irradiance of 2 mW/cm², optionally with the addition of a photosentiziser.

It was also found that when the oxidation in step c) is photochemically catalyzed by UV-light with a minimum UV irradiance of 2 mW/cm² or by UV-light with a minimum UV irradiance of 2 mW/cm² with the addition of a photosensitizer, the material of the container, such as a vial, wherein the oxidation is performed may influence the reaction rate of the oxidation step and thus the time required for completing the oxidation by up to at least 90%. As shown in the Examples, the reaction time for the UV-light catalysed oxidations decreases remarkedly when the oxidation step is performed in a quartz vial compared to a standard borosilicate glassware vial. Thus, in a preferred embodiment, step c) is performed in a quartz container when the oxidant in step c) is UV-light with a minimum UV irradiance of 2 mW/cm², optionally with the addition of a photosentiziser.

It was moreover found that when the oxidation in step c) is photochemically catalyzed by UV-light with a minimum UV irradiance of 2 mW/cm², or by UV-light with a minimum UV irradiance of 2 mW/cm² with the addition of a photosensitizer, using an open container, such as vial without a lid, for the oxidation step may influence the reaction rate of the oxidation step and thus the time required for completing the oxidation by up to at least 90%. As shown in the Examples, the reaction time for the UV-light catalysed oxidations decreases when the oxidation step is performed in an open vial as compared to a closed vial. Thus, in a preferred embodiment, step c) is performed in an open container when the oxidant in step c) is UV-light optionally with the addition of a photosentiziser.

It is found herein that that when the oxidation in step c) is photochemically catalyzed by UV-light or by UV-light with the addition of a photosensitizer the intensity of the UV-light source may influence the reaction rate of the oxidation step. Thus, the oxidation should be performed with UV-light with a minimum UV irradiance of 2 mW/cm² for the UV-light to oxidize the reaction rapidly, i.e. within 180 minutes.

In one embodiment, the oxidant is solid phase supported. Any commonly available solid support would be applicable, for instance oxidants supported by alumina, silica gel, polymer, montmorillonite, zeolite or a nanomaterial. The advantages of using a solid supported oxidants in general include easy removal from reactions by filtration, excess reagents can be used to drive reactions to completion without introducing difficulties in purification, easy to handle, recycling of recovered reagents is economical, and efficient.

The conventional ligation between a tetrazine and a TCO will result in a complex mixture of different tautomers, regioisomers and enantiomers as schematically shown in Scheme 1.

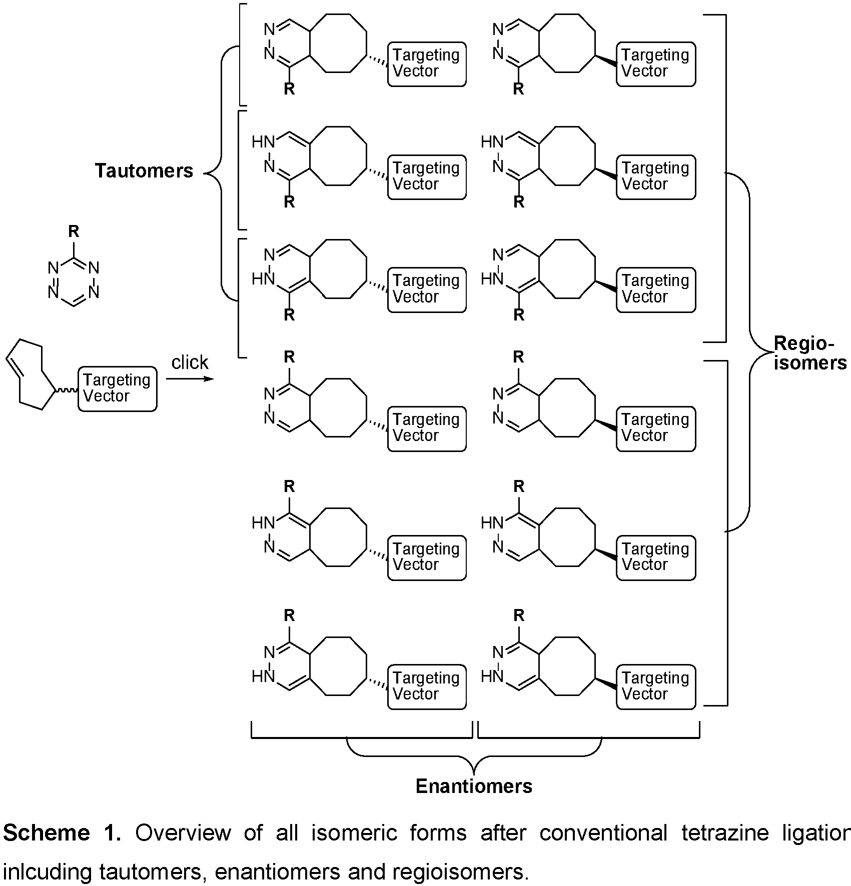

The below scheme 2 is an example of a current state-of-the-art tetrazine ligation. A reaction which gives too many isomeric products:

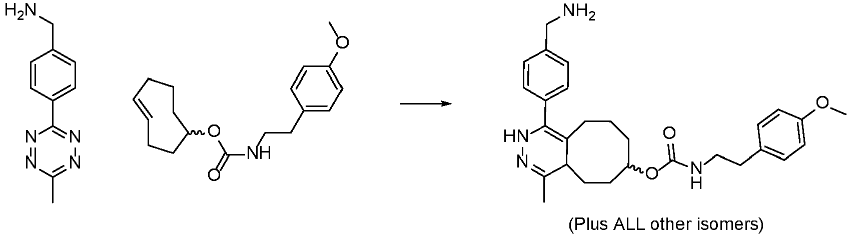

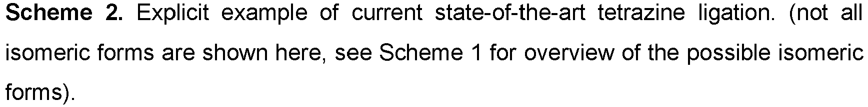

Multiple isomers (at least 12 isomers) can be formed in this reaction, which all have very similar polarities and are accordingly very difficult to separate for instance by HPLC.
Herein, we describe a method, which allows for the reduction of complexity, i.e. increased homogeneity for the use of a labeled first chemical entity such as radiolabelled tetrazine-based synthons in the radiolabelling of cyclic unsaturated hydrocarbon, with at least one double bond in the *trans*-configuration; an unsaturated heterocycle, with at least one double bond in the *trans*-configuration functionalized vectors and vice versa, which upon subsequent oxidation yields pyridazine-containing targeting vectors within short time, such as within 0 - 180 minutes. The method comprises three steps: a labeling step, a ligation step, andan oxidation step.

The starting entities to be ligated is a cyclic unsaturated hydrocarbon, with at least one double bond in the *trans*-configuration or an unsaturated heterocycle, with at least one double bond in the *trans*-configuration and 1,2,4,5-tetrazine.

The below scheme 2 and scheme 3 are illustrations of examples of ligations in accordance with the method of the invention here exemplified in using an unsymmetrical tetrazine and a TCO conjugated to a targeting vector:

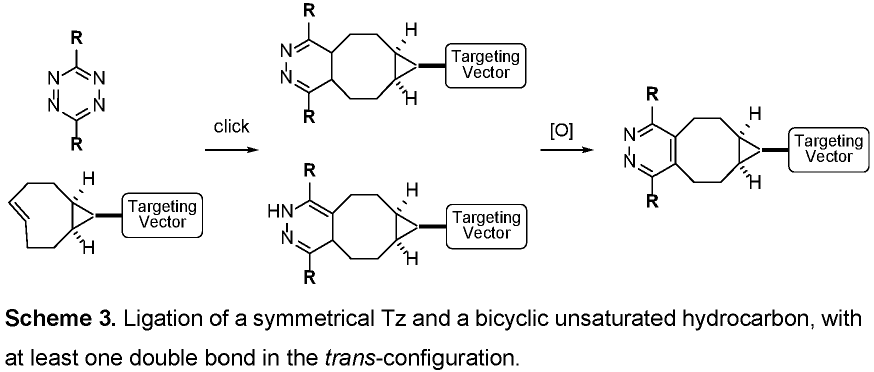

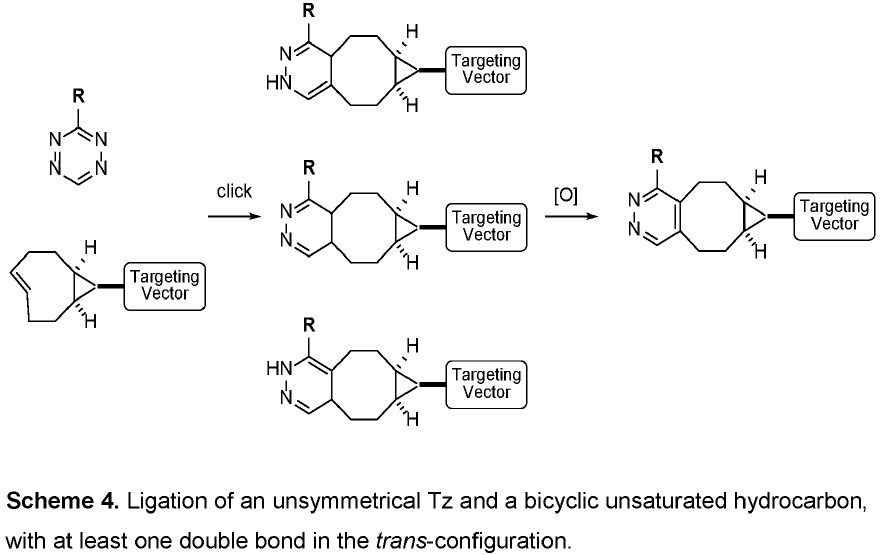

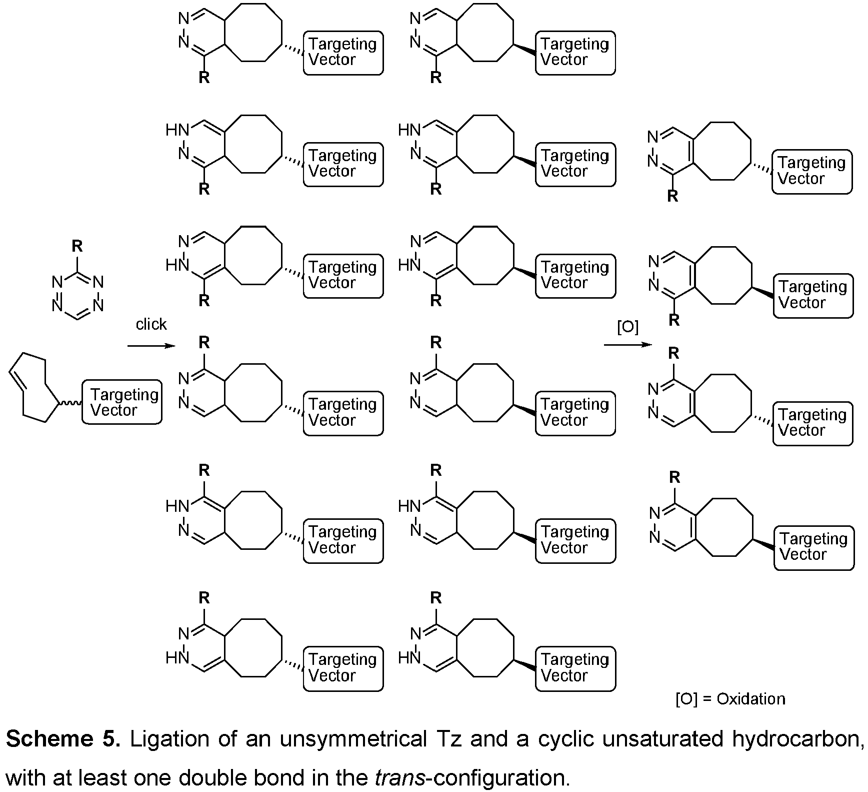

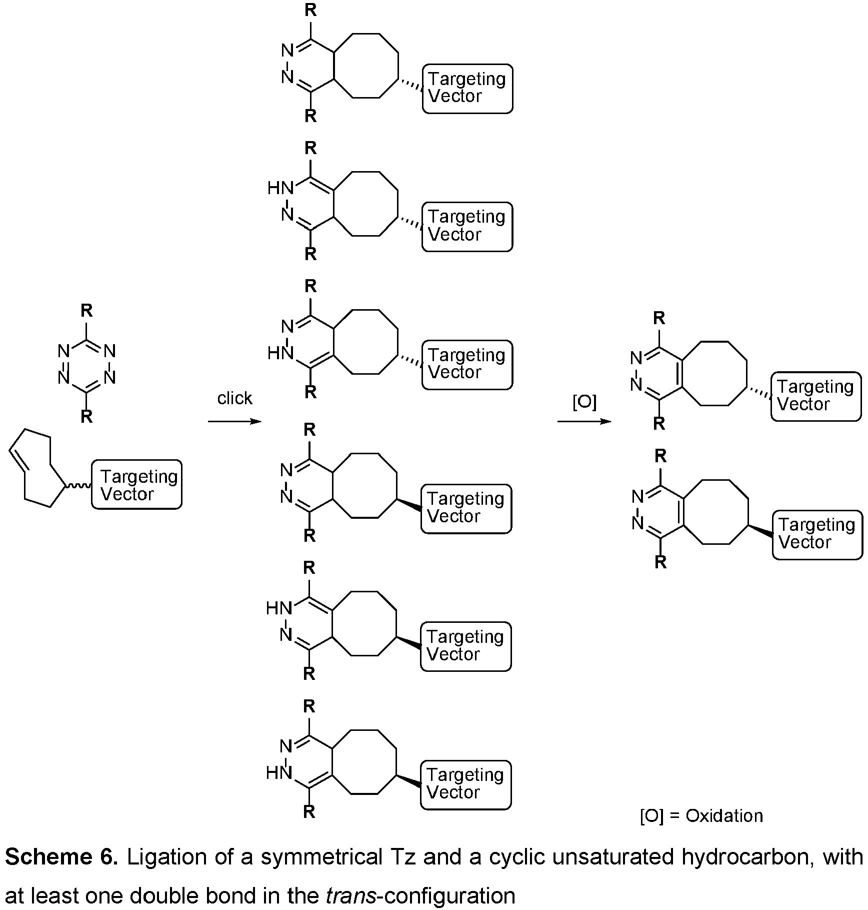

The targeting vector is an antibody, a nanobody, a polymer, a nanomedicine, a cell, a protein, a peptide, or a small molecule.

The following tetrazines of formula Tz1 are suitable for ligation in step b) of the method for providing a labeled pyridazine-containing targeting vector: 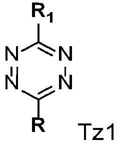
wherein R and R₁ are independently selected from -H, -Me or 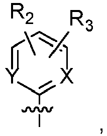 wherein the curly sign indicates the link to the tetrazine; and where R₂ is -H or (i) an isotope labeling agent directly connected to the aromatic ring; or (ii) an isotope labeling agent connected to the aromatic ring via a linker, said linker being selected from the group consisting of -(CH₂)ₙ, LO(CH₂)ₙ, -LNH(CH₂)ₙ, -LCONH(CH₂)ₙ, - LNHCO(CH₂)ₙ, where L is -(CH₂)ₘ or -(CH₂CH₂O)ₘ , where n and m are independently selected from 1-25; or (iii) an isotope labeling agent that is chelated through a chelator selected from: 1,4,7,10-tetraazacyclododecane-*N,N',N',N"-*tetraacetic acid (DOTA), *N,N'*-bis(2-hydroxy-5-(carboxyethyl)benzyl)ethylenediamine *N,N'*-diacetic acid (HBED-CC), 14,7-triazacyclononane-1,4,7-triacetic acid (NOTA), 2-(4.7-bis(carboxymethyl)-1,4,7-triazonan-1-yl)pentanedioic acid (NODAGA), 2-(4,7,10-tris(carboxymethyl)-1,4,7,10-tetraazacyclododecan-1- yl)pentanedioic acid (DOTAGA), 14,7-triazacyclononane phosphinic acid (TRAP), 14,7-triazacyclononane-1-methyl(2-carboxyethyl)phosphinic acid-4,7-bis(methyl(2-hydroxymethyl)phosphinic acid (NOPO), 3,6,9,15-tetraazabicyclo9.3.1.pentadeca-1 (15), 11,13-triene-3,6,9-triacetic acid (PCTA), *N*'-(5-acetyl (hydroxy)aminopentyl-*N*-(5-(4-(5-aminopentyl)(hydroxy)amino-4-oxobutanoyl)amino)pentyl-*N*-hydroxysuccinamide (DFO), diethylenetriaminepentaacetic acid (DTPA), *trans*-cyclohexyl-diethylenetriaminepentaacetic acid (CHX-DTPA), 1-oxa-4,7,10-triazacyclododecane-4,7,10-triacetic acid (OXO-Do3A), *p*-isothiocyanatobenzyl-DTPA (SCN-BZ-DTPA), 1-(*p*-isothiocyanatobenzyl)-3-methyl-DTPA (1B3M), 2-(p-isothiocyanatobenzyl)-4-methyl-DTPA (1M3B), and 1-(2)-methyl-4-isocyanatobenzyl-DTPA (MX-DTPA) connected to the aromatic ring through a linker, said linker being selected from the group consisting of -(CH₂)ₙ, -LO(CH₂)ₙ, - LNH(CH₂)ₙ, -LCONH(CH₂)ₙ, -LNHCO(CH₂)ₙ, where L is -(CH₂)ₘ or --(CH₂CH₂O)ₘ, and n and m are independently selected from 1-25;
wherein, when R₂ is either (i) or (ii) the isotope labeling agent is selected from the group consisting of:
   ¹H, ²H, ³H, ¹¹C, ¹²C, ¹³C ¹⁴C, ¹³N, ¹⁴N, ¹⁵N ¹⁸F, ¹⁹F ¹²³I, ¹²⁴I,¹²⁵I, ¹²⁷I, ¹³¹I, ²¹¹At, ¹⁵O, ¹⁶O, ¹⁷O, ¹⁸O, ⁴³Sc, ⁴⁴Sc, ⁴⁵Sc, ⁴⁵Ti, ⁴⁶Ti, ⁴⁷Ti, ⁴⁸Ti, ⁴⁹Ti, ⁵⁰Ti, ⁵⁵Co, ⁵⁸mCo, ⁵⁹Co, ⁶⁰Cu, ⁶¹Cu, ⁶³Cu, ⁶⁴Cu, ⁶⁵Cu, ⁶⁷Cu, ⁶⁷Ga, ⁶⁸Ga, ⁶⁹Ga, ⁷¹Ga, ⁷⁶Br, ⁷⁷Br, ⁷⁹Br, ⁸⁰mBr, ⁸¹Br, ⁷²As, ⁷⁵As, ⁸⁶Y, ⁸⁹Y, ⁹⁰Y, ⁸⁹Zr, ⁹⁰Zr, ⁹¹Zr, ⁹²Zr, ⁹⁴Zr, ¹⁴⁹Tb, ¹⁵²Tb, ¹⁵⁹Tb, ¹⁶¹Tb, ¹¹¹In, ¹¹³In, ¹¹⁴mIn, ¹¹⁵mIn, ¹⁷⁵Lu, ¹⁷⁷Lu, ¹⁸⁵Re, ¹⁸⁶Re, ¹⁸⁸Re, ²⁰¹Tl, ²⁰³Tl, ²⁰⁵Tl, ²⁰⁶Pb, ²⁰⁷Pb,²⁰⁸Pb,²¹²Pb, ²⁰⁹Bi, ²¹²Bi, ²¹³Bi, ³¹P, ³²P, ³³P, ³²S, ³⁵S, ⁴⁵Sc, ⁴⁷Sc, ⁸⁴Sr, ⁸⁶Sr, ⁸⁷Sr, ⁸⁸Sr, ⁸⁹Sr, ¹⁶⁵Ho, ¹⁶⁶Ho, ¹⁵⁶Dy, ¹⁵⁸Dy, ¹⁶⁰Dy, ¹⁶¹Dy, ¹⁶²Dy, ¹⁶³Dy, ¹⁶⁴Dy, ¹⁶⁵Dy , ²²⁷Th, ²³²Th, ⁵¹Cr, ⁵²Cr, ⁵³Cr, ⁵⁴Cr, ⁷³Se, ⁷⁴Se, ⁷⁵Se, ⁷⁶Se, ⁷⁷Se, ⁷⁸Se, ⁸⁰Se, ⁸²Se, ⁹⁴Tc, ^{99m}Tc, ¹⁰³Rh,¹⁰³mRh, ¹¹⁹Sb, ¹²¹Sb, ¹²³Sb, ¹³⁵La, ¹³⁸La, ¹³⁹La, ¹⁶²Er, ¹⁶⁴Er, ¹⁶⁵Er, ¹⁶⁶Er, ¹⁶⁷Er, ¹⁶⁸Er, ¹⁷⁰Er, ¹⁹³mPt, ¹⁹⁵mPt, ¹⁹²Pt, ¹⁹⁴Pt, ¹⁹⁵Pt, ¹⁹⁶Pt, ¹⁹⁸Pt ²¹¹At ²²³Ra, ²²⁵Ac,
   and wherein X and Y are independently selected from: -CH and -N- ;
   and wherein R₃ is independently selected from H or a moiety selected from the group consisting of a hydroxy group, a sulfonamide, a guanidyl group, a carboxyl group, a sulfonyl group, amine, a substituted amine with 1-25 polyethylene glycol unit(s), a -(O-CH₂-CH₂)ₙ₋-OCH₂-COOH, and n is selected from 1-25; or Methyl, Ethyl, Propyl, optionally substituted heteroaryl, and optionally substituted arylalkyl; wherein optionally substituted in relation to said substituted amine means one or more substituents selected from, a halogen, a hydroxy group, a sulfonamide, a carboxyl group, a sulfonyl group, amine, (C1-C10) alkyl, (C2-C10)alkenyl, (C2-C10)alkynyl, (C1-C10)alkylene, (C1-C10)alkoxy, (C2-C10)dialkylamino, (C1-C10)alkylthio, (C2-C10)heteroalkyl, (C2-C10)heteroalkylene, (C3-C10)cycloalkyl, (C3-C10)heterocycloalkyl, (C3-10)cycloalkylene, (C3-C10)heterocycloalkylene, (C1-C10)haloalkyl, (C1-C10)perhaloalkyl, (C2-C10)-alkenyloxy, (C3-C10)-alkynyloxy, aryloxy, arylalkyloxy, heteroaryloxy, heteroarylalkyloxy, (C1-C6)alkyloxy-(C1-C4)alkyl, optionally substituted aryl, optionally substituted heteroaryl, and optionally substituted arylalkyl; wherein optionally substituted means one or more substituents selected from a halogen, a hydroxy group, a sulfonamide, a carboxyl group, a sulfonyl group, amine, a substituted amine with 1-25 polyethylene glycol unit(s), a -(O-CH₂-CH₂)ₙ-OCH₂-COOH, and n is selected from 1-25; or H, Methyl, Ethyl, Propyl, optionally substituted heteroaryl, and optionally substituted arylalkyl; wherein optionally substituted in relation to said substituted amine means one or more substituents selected from a halogen, a hydroxy group, a sulfonamide, a carboxyl group, a sulfonyl group, and amine;
   and wherein R and R₁ are identical or differs only in the isotope number of the labelling agent.

In preferred embodiments, the 1,2,4,5-tetrazine is a H-tetrazine, a methyl-Tz, a bisphenyl or a bispyridyl. In a preferred embodiment, the 1,2,4,5-tetrazine is a H-tetrazine.

The below tetrazines are preferred tetrazines of Formula Tz1 for use in the ligation in step b) of the method for providing labeled pyridazine-containing targeting vector:

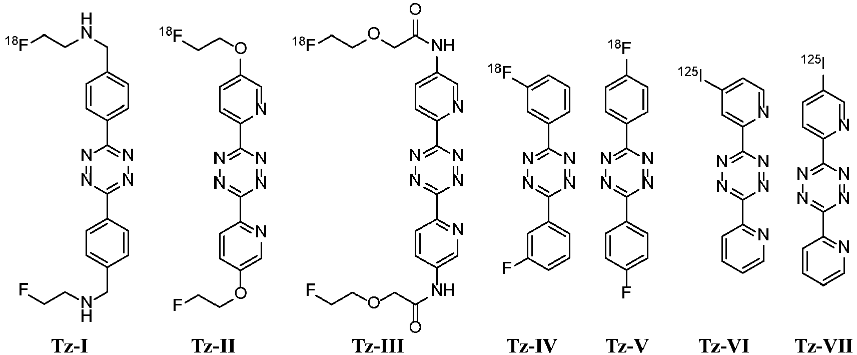

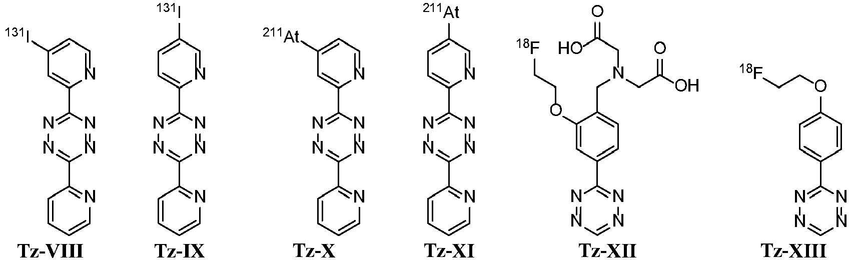

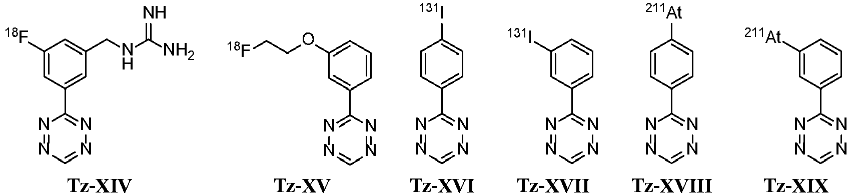

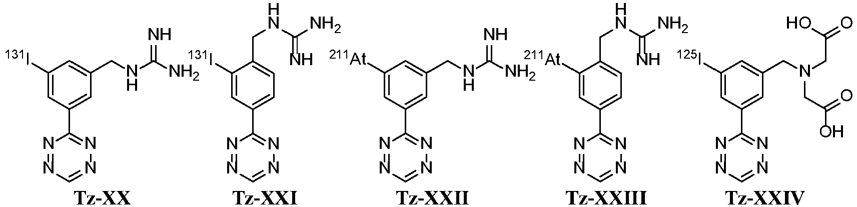

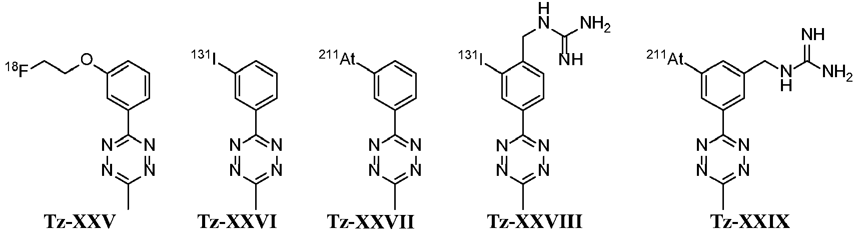

The below structures are preferred cyclic unsaturated hydrocarbon, with at least one double bond in the trans-configuration or an unsaturated heterocycle, with at least one double bond in the trans-configuration for use in ligating in step b) of the method for providing a labeled pyridazine-containing targeting vector: 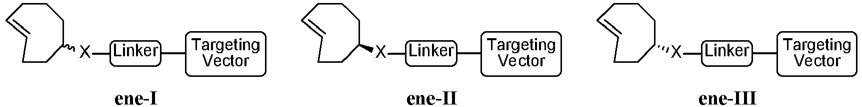 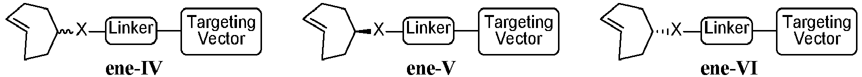 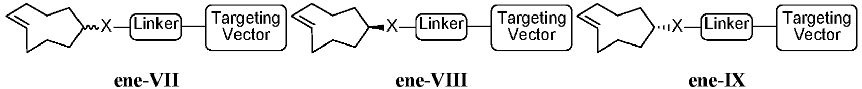 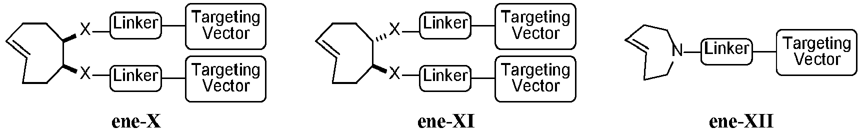 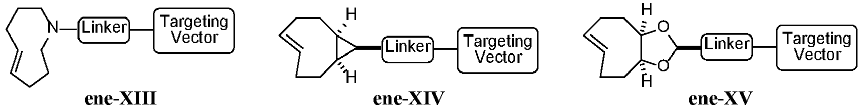 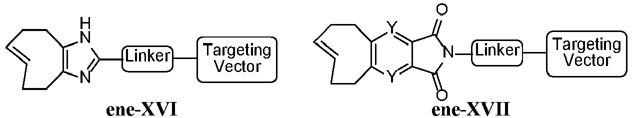 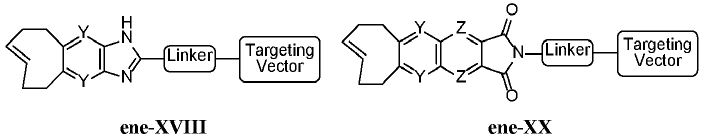 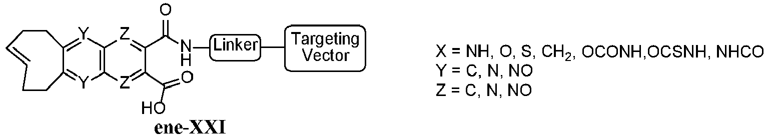
wherein X is NH, O, S, CH₂, OCONH, OCSNH, NHCO; Y is N, NO or CR₈; Z is N, NO or CR₈; R₈ is selected from the group consisting of: -H, -F, -OH, -NH₂, -COOH, - COOCH₃, CF₃, -Cl, -CONH₂, CONHCH₃, -CON(CH₃)₂, , -CH₂OH, -CH₂NH₂,-CH₂CH₂OH, -CH₂CH₂NH₂, -CHCH₂N(CH₃)₂ and
wherein the linker is selected from the group comprising: -(CH₂)ₙ- (CH₂)ₙNH, (CH₂)ₙCO, (CH₂)ₙO, (CH₂CH₂O)ₙ, (CH₂CH₂O)ₙCH₂CH₂NH, (CH₂CH₂O)ₙCH₂CH₂CO, - CO(CH)₂- CO(CH₂)ₙNH, CO(CH₂)ₙCO, CO(CH₂)ₙO, CO(CH₂CH₂O)ₙ CO(CH₂CH₂O)ₙCH₂CH₂NH, CO(CH₂CH₂O)ₙCH₂CH₂CO, COO(CH)₂- COO(CH₂)ₙNH, COO(CH₂)ₙCO, COO(CH₂)ₙO, COO(CH₂CH2O)ₙ COO(CH₂CH₂O)ₙCH₂CH₂NH, COO(CH₂CH₂O)ₙCH₂CH₂CO, CONH(CH)₂-CONH(CH₂)ₙNH, CONH(CH₂)ₙCO, CONH(CH₂)ₙO, CONH(CH₂CH₂O)ₙ, CONH(CH₂CH₂O)ₙCH₂CH₂NH, CONH(CH₂CH₂O)ₙCH₂CH₂CO, -CONHPhCO, -COOPhCO, -COPhCO, CONHCHMCO, (CH₂)ₙNHCHMCO, (CH₂)ₙOCONHCHMCO, (CH₂)ₙNHCHMCO, (CH₂)ₙNHCOCHMNH, (CH₂)OCOCHMNH, (CH₂CH₂O)ₙCH₂CH₂NHCHMCO, (CH₂CH₂O)ₙCH₂CH₂CONHCHMCO, (CH₂CH₂O)ₙCH₂CH₂NHCHMCO, (CH₂CH₂O)ₙCH₂CH₂NHCOCHMNH, (CH₂CH₂O)ₙCOCHMNH, where n is 0-25 and where M is a side chain selected from the group consisting of side chains of the natural amino acids: H, CH₃, CH₂SH, CH₂COOH, CH₂CH₂COOH, CH₂C₆H₅, CH₂C₃H₃N₂, CH(CH₃)CH₂CH₃, (CH₂)₄NH₂, CH₂CH(CH₃)₂, CH₂CH₂SCH₃, CH₂CONH₂, (CH₂)₄NHCOC₄H₅NCH₃, CH₂CH₂CH₂, CH₂CH₂CONH₂, (CH₂)₃NH-C(NH)NH₂, CH₂OH, CH(OH)CH₃, CH₂SeH, CH(CH₃)₂, CH₂C₈H₆N, CH₂C₆H₄OH;
and where the targeting vector is an antibody, a nanobody, a polymer, a nanomedicine, a cell, a protein, a peptide, or a small molecule.

Examples of monocyclic unsaturated hydrocarbon, with at least one double bond in the trans-configuration or monocyclic unsaturated heterocycle, with at least one double bond in the *trans*-configuration that are suitable for ligation in step b) of the method for providing a labeled pyridazine-containing targeting vector: 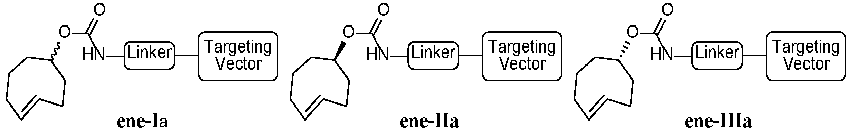 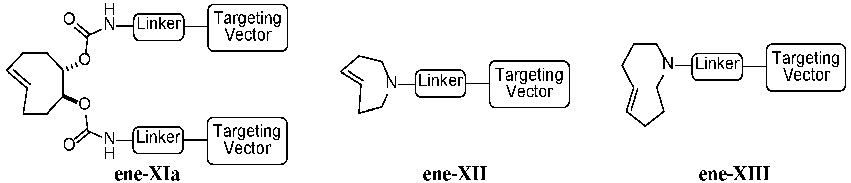 Wherein the linker is selected from the group comprising: : -(CH₂)ₙ- (CH₂)ₙNH, (CH₂)ₙCO, (CH₂)ₙO, (CH₂CH₂O)ₙ, (CH₂CH₂O)ₙCH₂CH₂NH, (CH₂CH₂O)ₙCH₂CH₂CO, - CO(CH)₂- CO(CH₂)ₙNH, CO(CH₂)ₙCO, CO(CH₂)ₙO, CO(CH₂CH₂O)ₙ CO(CH₂CH₂O)ₙCH₂CH₂NH, CO(CH₂CH₂O)ₙCH₂CH₂CO, COO(CH)₂- COO(CH₂)ₙNH, COO(CH₂)ₙCO, COO(CH₂)ₙO, COO(CH₂CH2O)ₙ COO(CH₂CH₂O)ₙCH₂CH₂NH, COO(CH₂CH₂O)ₙCH₂CH₂CO, CONH(CH)₂-CONH(CH₂)ₙNH, CONH(CH₂)ₙCO, CONH(CH₂)ₙO, CONH(CH₂CH₂O)ₙ, CONH(CH₂CH₂O)ₙCH₂CH₂NH, CONH(CH₂CH₂O)ₙCH₂CH₂CO, -CONHPhCO, -COOPhCO, -COPhCO, CONHCHMCO, (CH₂)ₙNHCHMCO, (CH₂)ₙOCONHCHMCO, (CH₂)ₙNHCHMCO, (CH₂)ₙNHCOCHMNH, (CH₂)OCOCHMNH, (CH₂CH₂O)ₙCH₂CH₂NHCHMCO, (CH₂CH₂O)ₙCH₂CH₂CONHCHMCO, (CH₂CH₂O)ₙCH₂CH₂NHCHMCO, (CH₂CH₂O)ₙCH₂CH₂NHCOCHMNH, (CH₂CH₂O)ₙCOCHMNH, where n is 0-25 and where M is a side chain selected from the group consisting of side chains of the natural amino acids: H, CH₃, CH₂SH, CH₂COOH, CH₂CH₂COOH, CH₂C₆H₅, CH₂C₃H₃N₂, CH(CH₃)CH₂CH₃, (CH₂)₄NH₂, CH₂CH(CH₃)₂, CH₂CH₂SCH₃, CH₂CONH₂, (CH₂)₄NHCOC₄H₅NCH₃, CH₂CH₂CH₂, CH₂CH₂CONH₂, (CH₂)₃NH-C(NH)NH₂, CH₂OH, CH(OH)CH₃, CH₂SeH, CH(CH₃)₂, CH₂C₈H₆N, CH₂C₆H₄OH;
and where the targeting vector is an antibody, a nanobody, a polymer, a nanomedicine, a cell, a protein, a peptide, or a small molecule.

Examples of bicyclic unsaturated hydrocarbon, with at least one double bond in the trans-configuration or bicyclic unsaturated heterocycle, with at least one double bond in the trans-configuration that are suitable for ligation in step b) of the method for providing a labeled pyridazine-containing targeting vector: 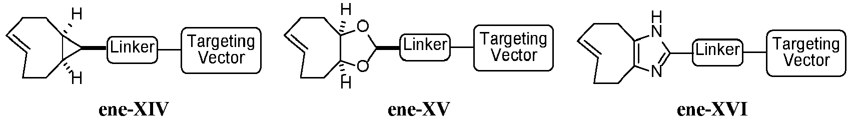 Wherein the linker is selected from the group comprising: : -(CH₂)ₙ- (CH₂)ₙNH, (CH₂)ₙCO, (CH₂)ₙO, (CH₂CH₂O)ₙ, (CH₂CH₂O)ₙCH₂CH₂NH, (CH₂CH₂O)ₙCH₂CH₂CO, - CO(CH)₂- CO(CH₂)ₙNH, CO(CH₂)ₙCO, CO(CH₂)ₙO, CO(CH₂CH₂O)ₙ CO(CH₂CH₂O)ₙCH₂CH₂NH, CO(CH₂CH₂O)ₙCH₂CH₂CO, COO(CH)₂- COO(CH₂)ₙNH, COO(CH₂)ₙCO, COO(CH₂)ₙO, COO(CH₂CH2O)ₙ COO(CH₂CH₂O)ₙCH₂CH₂NH, COO(CH₂CH₂O)ₙCH₂CH₂CO, CONH(CH)₂-CONH(CH₂)ₙNH, CONH(CH₂)ₙCO, CONH(CH₂)ₙO, CONH(CH₂CH₂O)ₙ, CONH(CH₂CH₂O)ₙCH₂CH₂NH, CONH(CH₂CH₂O)ₙCH₂CH₂CO, -CONHPhCO, -COOPhCO, -COPhCO, CONHCHMCO, (CH₂)ₙNHCHMCO, (CH₂)ₙOCONHCHMCO, (CH₂)ₙNHCHMCO, (CH₂)ₙNHCOCHMNH, (CH₂)OCOCHMNH, (CH₂CH₂O)ₙCH₂CH₂NHCHMCO, (CH₂CH₂O)ₙCH₂CH₂CONHCHMCO, (CH₂CH₂O)ₙCH₂CH₂NHCHMCO, (CH₂CH₂O)ₙCH₂CH₂NHCOCHMNH, (CH₂CH₂O)ₙCOCHMNH, where n is 0-25 and where M is a side chain selected from the group consisting of side chains of the natural amino acids: H, CH₃, CH₂SH, CH₂COOH, CH₂CH₂COOH, CH₂C₆H₅, CH₂C₃H₃N₂, CH(CH₃)CH₂CH₃, (CH₂)₄NH₂, CH₂CH(CH₃)₂, CH₂CH₂SCH₃, CH₂CONH₂, (CH₂)₄NHCOC₄H₅NCH₃, CH₂CH₂CH₂, CH₂CH₂CONH₂, (CH₂)₃NH-C(NH)NH₂, CH₂OH, CH(OH)CH₃, CH₂SeH, CH(CH₃)₂, CH₂C₈H₆N, CH₂C₆H₄OH;
and where the targeting vector is an antibody, a nanobody, a polymer, a nanomedicine, a cell, a protein, a peptide, or a small molecule.

Examples of tricyclic unsaturated hydrocarbon, with at least one double bond in the *trans*-configuration or tricyclic unsaturated heterocycle, with at least one double bond in the *trans*-configuration that are suitable for ligation in step b) of the method for providing a labeled pyridazine-containing targeting vector: 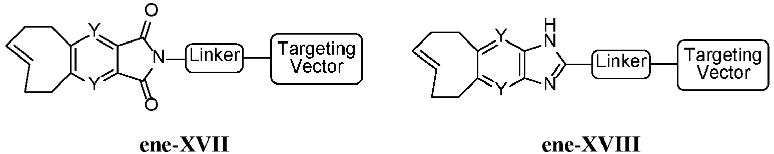
Wherein X is N, NO or CR₈ ; Y is N, NO or CR₈; R₈ is selected from the group consisting of: -H, -F, -OH, -NH₂, -COOH, -COOCH₃, CF₃, -Cl, -CONH₂, CONHCH₃, - CON(CH₃)₂, -CH₂CH₂OH, -CH₂CH₂NH₂, -CHCH₂N(CH₃)₂ and
wherein the linker is selected from the group comprising: : -(CH₂)ₙ- (CH₂)ₙNH, (CH₂)ₙCO, (CH₂)ₙO, (CH₂CH₂O)ₙ, (CH₂CH₂O)ₙCH₂CH₂NH, (CH₂CH₂O)ₙCH₂CH₂CO, - CO(CH)₂- CO(CH₂)ₙNH, CO(CH₂)ₙCO, CO(CH₂)ₙO, CO(CH₂CH₂O)ₙ CO(CH₂CH₂O)ₙCH₂CH₂NH, CO(CH₂CH₂O)ₙCH₂CH₂CO, COO(CH)₂- COO(CH₂)ₙNH, COO(CH₂)ₙCO, COO(CH₂)ₙO, COO(CH₂CH2O)ₙ COO(CH₂CH₂O)ₙCH₂CH₂NH, COO(CH₂CH₂O)ₙCH₂CH₂CO, CONH(CH)₂-CONH(CH₂)ₙNH, CONH(CH₂)ₙCO, CONH(CH₂)ₙO, CONH(CH₂CH₂O)ₙ, CONH(CH₂CH₂O)ₙCH₂CH₂NH, CONH(CH₂CH₂O)ₙCH₂CH₂CO, -CONHPhCO, -COOPhCO, -COPhCO, CONHCHMCO, (CH₂)ₙNHCHMCO, (CH₂)ₙOCONHCHMCO, (CH₂)ₙNHCHMCO, (CH₂)ₙNHCOCHMNH, (CH₂)OCOCHMNH, (CH₂CH₂O)ₙCH₂CH₂NHCHMCO, (CH₂CH₂O)ₙCH₂CH₂CONHCHMCO, (CH₂CH₂O)ₙCH₂CH₂NHCHMCO, (CH₂CH₂O)ₙCH₂CH₂NHCOCHMNH, (CH₂CH₂O)ₙCOCHMNH, where n is 0-25 and where M is a side chain selected from the group consisting of side chains of the natural amino acids: H, CH₃, CH₂SH, CH₂COOH, CH₂CH₂COOH, CH₂C₆H₅, CH₂C₃H₃N₂, CH(CH₃)CH₂CH₃, (CH₂)₄NH₂, CH₂CH(CH₃)₂, CH₂CH₂SCH₃, CH₂CONH₂, (CH₂)₄NHCOC₄H₅NCH₃, CH₂CH₂CH₂, CH₂CH₂CONH₂, (CH₂)₃NH-C(NH)NH₂, CH₂OH, CH(OH)CH₃, CH₂SeH, CH(CH₃)₂, CH₂C₈H₆N, CH₂C₆H₄OH;
and where the targeting vector is an antibody, a nanobody, a polymer, a nanomedicine, a cell, a protein, a peptide, or a small molecule.

Examples of tetracyclic unsaturated hydrocarbon, with at least one double bond in the *trans*-configuration or tetracyclic unsaturated heterocycle, with at least one double bond in the *trans*-configuration that are suitable for ligation in step b) of the method for providing a labeled pyridazine-containing targeting vector: 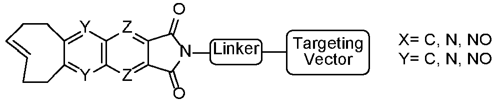 wherein X is N, NO or CR₈; Y is N, NO or CR₈; R₈ is selected from the group consisting of: -H, -F, -OH, -NH₂, -COOH, -COOCH₃, CF₃, -Cl, -CONH₂, CONHCH₃, -CON(CH₃)₂, -CH₂CH₂OH, -CH₂CH₂NH₂, -CHCH₂N(CH₃)₂ and the linker is selected from the group comprising: : -(CH₂)ₙ- (CH₂)ₙNH, (CH₂)ₙCO, (CH₂)ₙO, (CH₂CH₂O)ₙ, (CH₂CH₂O)ₙCH₂CH₂NH, (CH₂CH₂O)ₙCH₂CH₂CO, -CO(CH)₂- CO(CH₂)ₙNH, CO(CH₂)ₙCO, CO(CH₂)ₙO, CO(CH₂CH₂O)ₙ CO(CH₂CH₂O)ₙCH₂CH₂NH, CO(CH₂CH₂O)ₙCH₂CH₂CO, COO(CH)₂- COO(CH₂)ₙNH, COO(CH₂)ₙCO, COO(CH₂)ₙO, COO(CH₂CH2O)ₙ COO(CH₂CH₂O)ₙCH₂CH₂NH, COO(CH₂CH₂O)ₙCH₂CH₂CO, CONH(CH)₂-CONH(CH₂)ₙNH, CONH(CH₂)ₙCO, CONH(CH₂)ₙO, CONH(CH₂CH₂O)ₙ, CONH(CH₂CH₂O)ₙCH₂CH₂NH, CONH(CH₂CH₂O)ₙCH₂CH₂CO, -CONHPhCO, -COOPhCO, -COPhCO, CONHCHMCO, (CH₂)ₙNHCHMCO, (CH₂)ₙOCONHCHMCO, (CH₂)ₙNHCHMCO, (CH₂)ₙNHCOCHMNH, (CH₂)OCOCHMNH, (CH₂CH₂O)ₙCH₂CH₂NHCHMCO, (CH₂CH₂O)ₙCH₂CH₂CONHCHMCO, (CH₂CH₂O)ₙCH₂CH₂NHCHMCO, (CH₂CH₂O)ₙCH₂CH₂NHCOCHMNH, (CH₂CH₂O)ₙCOCHMNH, where n is 0-25 and where M is a side chain selected from the group consisting of side chains of the natural amino acids: H, CH₃, CH₂SH, CH₂COOH, CH₂CH₂COOH, CH₂C₆H₅, CH₂C₃H₃N₂, CH(CH₃)CH₂CH₃, (CH₂)₄NH₂, CH₂CH(CH₃)₂, CH₂CH₂SCH₃, CH₂CONH₂, (CH₂)₄NHCOC₄H₅NCH₃, CH₂CH₂CH₂, CH₂CH₂CONH₂, (CH₂)₃NH-C(NH)NH₂, CH₂OH, CH(OH)CH₃, CH₂SeH, CH(CH₃)₂, CH₂C₈H₆N, CH₂C₆H₄OH;
and where the targeting vector is an antibody, a nanobody, a polymer, a nanomedicine, a cell, a protein, a peptide, or a small molecule.

Step c) in the method for providing a pyridazine-containing targeting vector is an oxidation step. Even though auto-oxidation of the ligated entity targeting vector, such as a pyridazine, obtained in step b) of the method occurs spontaneously, this process is extremely slow and can last from several hours up to several days. Step c) in the method provides a fast way for oxidizing the dihydropyridazines mixture, in order to obtain the pyridazine compound, catalyst by an acid, wherein the pyridazine-containing targeting vector is obtained at least within 180 minutes, such as within 1-20 minutes. These conditions ensure that the efficiency of the oxidation step is ≥90%, thereby meeting the speed required for therapeutic, diagnostic or imaging use of the labeled pyridazine-containing targeting vector. In order to facilitate this process, the dihydropyridazines are oxidized by either a standard, or solid-supported oxidant, preferably solid-supported. The oxidizing step can be performed at a temperature ranging from 15 to 50 °C, such as at 20-30 °C, preferably at room temperature, for approximately 10 to 180 minutes, preferably for less than 20 minutes. To facilitate the oxidation, a chemical oxidant, 1 to 100 equivalents, preferably 1, is added, together with an acid to the ligated compound obtained from the ligation step. The oxidant needs to be selective for the oxidation of the dihydropyrazine to pyridazine. The targeting vector must not be chemically modified by the oxidant. The oxidant is a quinone-based oxidant, Bobbits salt-based oxidant, peroxide-based oxidant or an peroxy acid-based oxidant or UV-light with a minimum UV irradiance of 2 mW/cm², optionally with the addition of a photosensitizer.

The acid is added to the complex mixture of the various dihydropyridazines, resulting from the ligation of a tetrazine and a strained alkene obtained from step b) and is exposed to UV-light, such as from 200 to 400 nm, 200 to 280 nm (UVC), 280 to 315 nm (UVB), 315 to 400 (UVA). Preferably 254 nm.

In a preferred embodiment, the oxidation is photochemically catalyzed by UV-light with a minimum UV irradiance of 2 mW/cm², or by UV-light with a minimum UV irradiance of 2 mW/cm² with the addition of a photosensitizer, such as fluoroscein-based or porphyrin-based photosensitizers, in the presence of an acid. The acid is added to the complex mixture of the various dihydropyridazines, resulting from the ligation of a tetrazine and a strained alkene obtained from step b) and is exposed to UV-light with a minimum UV irradiance of 2 mW/cm², such as UV-light with a wavelength from 200 to 400 nm, 200 to 280 nm (UVC), 280 to 315 nm (UVB), or 315 to 400 (UVA). Preferably at UVC wavelength, such as at a wavelength of 254 nm.

The labeled pyridazine-containing targeting vector provided by the method can be used in therapy, radiotherapy, theranostics, diagnostics, or imaging, depending on the labeling agent, or the pharmaceutical agent, or imaging agent or therapeutic agent and on the targeting vector.

Preferably, the targeting vector is coupled to the linker via a nitrogen on the targeting vector. Alternatively, the targeting vector is preferable coupled to the linker via a carbonyl on the targeting vector.

In a preferred embodiment, the labeled pyridazine-containing targeting vector is used in therapy.

In another preferred embodiment, the labeled pyridazine-containing targeting vector is used in radiotherapy.

In another preferred embodiment, the labeled pyridazine-containing targeting vector is used in theranostics.

In another preferred embodiment, the labeled pyridazine-containing targeting vector is used in diagnostics.

In another preferred embodiment, the labeled pyridazine-containing targeting vector is used in imaging.

The following Examples describes (1) the synthesis of tetrazines and TCOs representative for use in step a) and b) of the present method for providing a labeled pyridazine-containing targeting vector and (2) click reactions and oxidations between such compounds, yielding a pyridazine.

### EXAMPLES

### General

All reagents and solvents were dried prior to use according to standard methods. Commercial reagents were used without further purification. Analytical TLC was performed using silica gel 60 F254 (Merck) with detection by UV absorption and/or by charring following immersion in a 7% ethanolic solution of sulfuric acid or KMnO₄-solution (1.5 g of KMnO₄, 10 g K₂CO₃, and 1.25 mL 10% NaOH in 200 mL water). Purification of compounds was carried out by column chromatography on silica gel (40-60 µm, 60 Å) or employing a CombiFlash NextGen 300+ (Teledyne ISCO). ¹H and ¹³C NMR spectra were recorded on Brucker (400 and 600 MHz instruments), using Chloroform-d, Methanol-*d*₄ or DMSO-*d*₆ as deuterated solvent and with the residual solvent as the internal reference. For all NMR experiences the deuterated solvent signal was used as the internal lock. Chemical shifts are reported in δ parts per million (ppm). Coupling constants (*J* values) are given in Hertz (Hz). Multiplicities of ¹H NMR signals are reported as follows: s, singlet; d, doublet; dd, doublet of doublets; ddd, doublet of doublets of doublets; dt, doublet of triplets; t, triplet; q, quartet; m, multiplet; br, broad signal. NMR spectra of all compounds are reprocessed in MestReNova software (version 12.0.22023) from original FID's files. Mass spectra analysis was performed using MS-Acquity-A: Waters Acquity UPLC with QDa-detector. Purification by preparative HPLC was performed on Agilent 1260 infinity system, column SymmetryPrep-C18, 17 mL/min H₂O-MeCN gradient 50-100% 15 min with 0.1% trifluoroacetic acid. All final compounds were >95% pure as determined by analytical HPLC. Analytical HPLC method: (Thermo Fisher^{®} UltiMate 3000) with a C-18 column (Luna^{®} 5u C18(2) 100Å, 150 × 4.6 mm), eluents: A: H2O with 0.1% TFA, B: MeCN with 0.1% TFA. Gradient from 100% A -> 100% B over 15minutes, back to 100% A over 4 minutes, flow rate 1.5 mL/min. Detection by UV-absorption at λ = 254 nm on a UVD 170U detector.

### Example 1

### Synthesis of tetrazines and their precursors

### Example 1.1

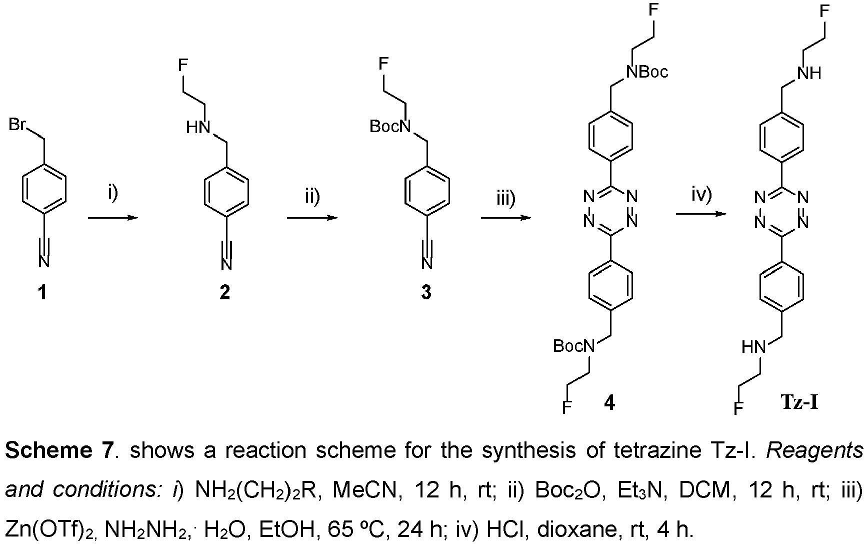

### Synthesis of 4-(((2-fluoroethyl)amino)methyl)benzonitrile (2)

To a solution of 4-(bromomethyl)benzonitrile (0.78 g, 4.00 mmol) in CH₃CN (40 mL) was added K₂CO₃ (0.33 g, 24.0 mmol) and 2-fluoroethylamine hydrochloride (0.16 g, 16.0 mmol). The mixture was stirred at room temperature overnight. The solvent was removed under reduced pressure, and the residue was diluted with water (20 mL), extracted with EtOAc. The combined organic layer was washed with brine, dried over MgSO₄, filtered and concentrated under reduced pressure. The crude product was purified by flash column chromatography using EtOAc (Heptane/EtOAc 50/50) in heptane to afford 0.54 g (76%) of the desired product as a colorless oil. R*_{f}* = 0.24 (Heptane/EtOAc 40/60). ¹H NMR (400 MHz, CDCl₃) δ 7.55 (d, *J* = 8.2 Hz, 2H), 7.40 (d, *J* = 8.0 Hz, 2H), 4.63 - 4.48 (m, 1H), 4.47 - 4.37 (m, 1H), 3.84 (s, 2H), 2.93 - 2.84 (m, 1H), 2.84 - 2.72 (m, 1H), 1.65 (s, 1H). ¹³C NMR (101 MHz, CDCl₃) *δ* 145.6, 132.3, 128.6, 118.9, 110.9, 83.5 (d, *J* = 165.5 Hz), 53.1, 49.1 (d, *J* = 19.7 Hz).

### Synthesis of tert-butyl 4-cyanobenzyl(2-fluoroethyl)carbamate (3)

To a solution of 4-(((2-fluoroethyl)amino)methyl)benzonitrile (540 mg, 3.03 mmol) and Et₃N (1.27 mL, 9.09 mmol) in CH₂Cl₂ (40 mL) was added Boc₂O (790 mg, 3.63 mmol) and the mixture was stirred at room temperature for 12 h. The solution was washed with water and saturated K₂CO₃ solution, dried over Na₂SO_{4,} filtered and concentrated under reduced pressure. The crude product was purified by flash column chromatography using (Heptane/EtOAc 70/30) to afford 0.710 g (84%) of the desired product as a colorless oil (mixture of rotamers). R*_{f}* = 0.42 (Heptane/EtOAc 80/20). ¹H NMR (400 MHz, CDCl₃) *δ* 7.55 (d, *J* = 7.8 Hz, 2H), 7.27 (d, *J* = 7.8 Hz, 2H), 4.79 - 4.10 (m, 4H), 3.62 - 3.28 (m, 2H), 1.96 - 1.05 (m, 9H). ¹³C NMR (101 MHz, CDCl₃) δ 155.4, 144.2, 143.8, 132.4, 128.1, 127.5, 118.7, 111.1, 83.2 (d, *J* = 168.2 Hz), 82.7 (d, *J* = 170.5 Hz), 52.1, 51.2, 47.7, 28.3.

### Synthesis of di-tert-butyl (((1,2,4,5-tetrazine-3,6-diyl)bis(4,1-phenylene))bis(methylene))bis((2-fluoroethyl)carbamate) (4)

To a suspension of *tert*-butyl 4-cyanobenzyl(2-fluoroethyl)carbamate (1.1 gr, 3.95 mmol) and Zn(OTf)₂ (0.72 gr, 1.98 mmol) in EtOH (30 mL) was added hydrazine monohydrate (3.83 mL, 79 mmol). The mixture was allowed to stir at 70 °C for 22 hours, and when the reaction is completed, is cooled at room temperature. The volatiles were removed under reduced pressure and the residue solubilized in EtOH (40 mL). A solution of NaNO₂ (5.52 g, 80.00 mmol ) in water (20 mL) was added to the crude reaction followed by dropwise addition of HCl (2M) until gas evolution ceased and a pH of 2-3 was achieved producing a red mixture. The crude reaction was extracted with DCM (3 × 40 mL) and washed with brine (3 × 20 mL). The organic phase was collected, dried over MgSO₄, filtered and concentrated under reduced pressure. Purification by flash chromatography (DCM/MeOH 98/2) afforded 0.300 g (26%) of a red solid. R*_{f}* = 0.45 (DCM/MeOH 98/2); ¹H NMR (600 MHz, CDCl₃) δ 8.63 (d, *J* = 8.0 Hz, 4H), 7.50 (d, *J* = 7.0 Hz, 4H), 5.11 - 4.38 (m, 8H), 3.97 - 3.26 (m, 4H), 1.95 - 0.57 (m, 18H); ¹³C NMR (151 MHz, CDCl₃) δ 163.77, 155.61, 155.57, 143.70, 130.81, 128.51, 128.21, 127.87, 83.18 (d, *J* = 167.9 Hz), 82.61 (d, *J* = 169.3 Hz), 72.44, 65.78, 52.09, 51.09, 47.67 (d, *J* = 19.9 Hz), 47.19 (d, *J* = 21.0 Hz), 28.38.

### Synthesis of N,N'-(((1,2,4,5-tetrazine-3,6-diyl)bis(4,1-phenylene))bis(methylene))bis(2-fluoroethan-1-amine) (Tz-I)

Di-*tert*-butyl (((1,2,4,5-tetrazine-3,6-diyl)bis(4,1-phenylene))bis(methylene))bis((2-fluoroethyl) carbamate) (0.15 gr, 0.25 mmol) was treated with a solution of HCl (4 M) in dioxane (1 mL). A precipitate was formed. Filtration afforded 0.1 gr (83%) of the desired product as hydrochloride salt. ¹H NMR (600 MHz, DMSO) δ 9.70 (s, 4H), 8.61 (d, *J* = 8.0 Hz, 4H), 7.89 (d, *J* = 8.0 Hz, 4H), 4.86 (t, *J* = 4.6 Hz, 2H), 4.78 (t, *J* = 4.6 Hz, 2H), 4.37 (s, 4H), 3.40 - 3.25 (m, 4H); ¹³C NMR (151 MHz, DMSO) δ 163.63, 136.83, 132.81, 131.66, 128.28, 80.09 (d, *J* = 165.1 Hz), 50.24, 47.21 (d, *J* = 19.8 Hz).

### Example 1.2

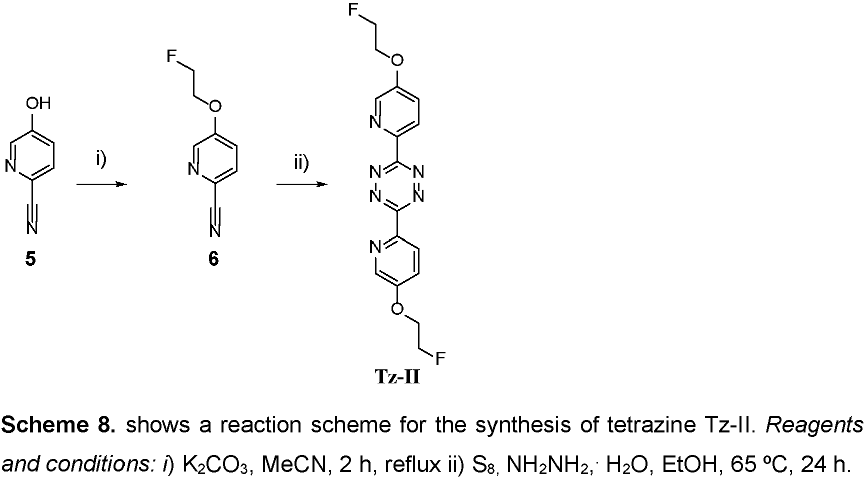

### Synthesis of 5-(2-fluoroethoxy)picolinonitrile (6)

To a solution of 5-hydroxypicolinonitrile (1 g, 8.35 mmol) and CH₃CN (30 mL) was added K₂CO₃ (2.30 g, 16.65 mmol) and 1-fluoroiodoethane (813 µL, 10.00 mmol). The reaction was refluxed until all the starting material was consumed disappeared. Water was added and the solution was ecxtracted with EtOAc. The solvent was removed under reduced pressure to give 1.32 g of a dark solid (95%). ¹H NMR (400 MHz, CDCl₃) δ 8.42 (d, *J* = 2.9 Hz, 1H), 7.66 (d, *J* = 8.6 Hz, 1H), 7.29 (dd, *J* = 8.6, 2.9 Hz, 2H), 4.90 - 4.83 (m, 1H), 4.77 - 4.69 (m, 1H), 4.40 - 4.35 (m, 1H), 4.32 - 4.25 (m, 1H).

### Synthesis of 3,6-bis(5-(2-fluoroethoxy)pyridin-2-yl)-1,2,4,5-tetrazine (Tz-II)

To a suspension of 5-(2-fluoroethoxy)picolinonitrile (810 mg, 4.87 mmol) and S₈ (0.72 gr, 1.98 mmol) in EtOH (1.8 mL) was added hydrazine monohydrate (0.89 mL, 18.27 mmol). The mixture was allowed to stir at 90 °C for 4 hours, and when the reaction is completed, is cooled at room temperature. The volatiles were removed under reduced pressure and the residue solubilized in EtOH (40 mL). A solution of NaNO₂ (5.52 g, 80.00 mmol ) in water (20 mL) was added to the crude reaction followed by dropwise addition of HCl (2M) until gas evolution ceased and a pH of 2-3 was achieved producing a red mixture. The crude reaction was extracted with DCM (3 × 40 mL) and washed with brine (3 × 20 mL). The organic phase was collected, dried over MgSO₄, filtered and concentrated under reduced pressure. Purification by flash chromatography (DCM/MeOH 98/2) afforded 125 mg (28 %) of a red solid. R*_{f}* = 0.45 (DCM/MeOH 98/2).

### Synthesis of 3-(5-iodopyridin-2-yl)-6-(pyridin-2-yl)-1,2,4,5-tetrazine (Tz-VII)

The compound was obtained following the procedure reported in Albu et al. 125I-Tetrazines and Inverse-Electron-Demand Diels-Alder Chemistry: A Convenient Radioiodination Strategy for Biomolecule Labeling, Screening, and Biodistribution Studies. Bioconjug Chem. 2016, ;27, 207-216.

### Synthesis of 2,2'-((2-(2-fluoroethoxy)-4-(1,2,4,5-tetrazin-3-yl)benzyl)azanediyl)diacetic acid (Tz-XII)

The compound was obtained following the procedure reported in Battisti et al. Development of the First Aliphatic 18F-Labeled Tetrazine Suitable for Pretargeted PET Imaging-Expanding the Bioorthogonal Tool Box. J. Med. Chem. 2021, 64, 20, 15297-15312.

### Example 1.3

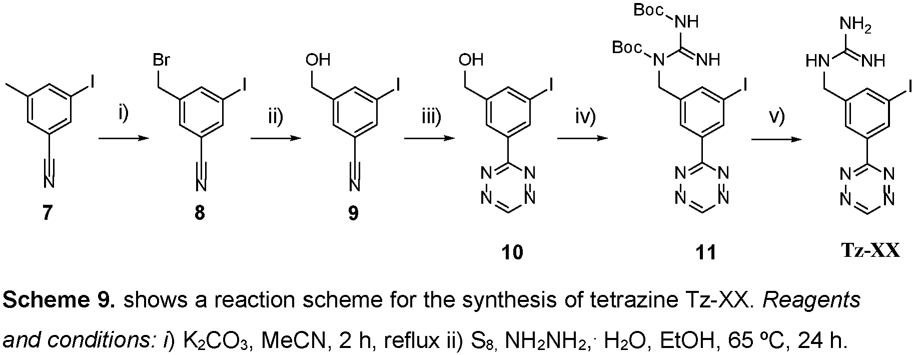

### Synthesis of 3-(bromomethyl)-5-iodobenzonitrile (8)

In a 100mL round-bottomed flask fitted with a stir bar, 3-iodo-5-methylbenzonitrile (2 g, 8.23 mmol), N-bromosuccinmide (2.20 g, 12.34 mmol) and AIBN (540 mg, 3.29 mmol) were dissolved in MecN (25 mL). The reaction was refluxed at 80-85°C for 6h.

Water and DCM were added. The organic layer was washed twice with water, and each water phase was extracted twice with DCM. The combined organic phase was washed in brine, dried over Mg₂SO₄, filtered and concentrated under vacuum. The residue was dissolved in DCM and absorbed unto Celite. Next, the crude product was purified by flash chromatography (heptane/DCM 85:15 -> 70:30) to give 2g (75.6%) of NB35 as a white solid. R*_{f}* = 0.11(heptane:DCM 8:2). ¹H-NMR (CDCl₃, 600MHz): 4.38 (s, 2H), 7.65 (t, 1H, *J* = 1.6), 7.91 (t, 1H, *J* = 1.55), 7.97 (t, 1H, *J* = 1.65). ¹³C-NMR (CDCl₃, 600 MHz): 30.0, 94.2, 114.7, 116.7, 131.8, 140.29, 141.1, 142.4.

### Synthesis of 3-(hydroxymethyl)-5-iodobenzonitrile (9)

3-(Bromomethyl)-5-iodobenzonitrile (1 g, 3.11 mmol), was refluxed (75-80 °C) in 1:1 MeCN/deionized water (24 mL) with barium carbonate (1.23 g, 6.21 mmol). The reaction was stirred overnight. LCMS showed reaction progression, more MeCN. After 28h the reaction was still slowly progressing, more MeCN, water and barium carbonate (1 eq) were added. The reaction was left stirring with reflux overnight. After 45h, complete reaction was observed by LCMS. The mixture was cooled to room temperature. The solids were removed by filtration. The filtrate was extracted three times with DCM. The combined organic phases were dried for Mg₂SO₄, filtered and concentrated. The residue was re-dissolved in DCM and absorbed unto Celite. The compound was purified by flash chromatography (5-20% EtOAc in heptane) to afford a white solid. Yield: 582 mg (69%). R*_{f}* = 0.36 (7:3 heptane:EtOAc). ¹H-NMR (CDCl₃, 600MHz): 1.88 (t, 1H, *J* = 6.76), 4.72 (d, 2H, *J* = 5.7), 7.62 - 7.64 (m, 1H), 7.88 - 7.90 (m, 1H), 7-95-7.97 (m, 1H). ¹³C-NMR (CDCl₃, 600 MHz): 63.3, 94.1, 114.4, 117.2, 129.4, 139.47, 140.1, 144.2.

### Synthesis of (3-iodo-5-(1,2,4,5-tetrazin-3-yl)phenyl)methanol (10)

3-(hydroxymethyl)-5-iodobenzonitrile (548 mg, 2.12 mmol), DCM (135 uL, 2.12 mmol), S₈ (136 mg, 0.53 mmol) and ethanol (3 mL) were mixed together in a 20 mL microwave reaction tube. Hydrazine monohydrate (825 uL, 16.9 mmol) was added slowly with stirring afterwards. The vessel was sealed and the reaction mixture was heated to 50 °C for 22 hours. The reaction turned dark with yellow precipitate. Afterwards 5 mL of DCM and sodium nitrite (1.46 g, 21.15 mmol) in 20 mL of H₂O were added to the mixture. Excess acetic acid (6 mL) was then added slowly during which the solution turned bright red in color. The reaction mixture was left stirring for about 10 min. Then, the water phase was extracted 4 times with DCM. The organic phase was dried over anhydrous magnesium sulfate, filtered, concentrated under reduced pressure. The resulting residue (769 mg) was purified using flash chromatography (heptane/EtOAc) to yield 262mg (39%) of product as a red/pink solid. R*_{f}* = 0.24 (Heptane/EtOAc 70/30). ¹H-NMR(CDCl₃, 600MHz): 1.88 (t, 1H, *J* = 5.9), 4.82 (d, 2H, *J* = 4.8Hz), 8.03 - 8.05 (m, 1H), 8.59 - 8.60 (m, 1H), 8.90 - 8.93 (m, 1H), 10.26 (s, 1H). ¹³C-NMR (CDCl3, 600 MHz): 64.1, 95.2, 125.8, 133.7,136.3, 140.3, 144.4, 158.2, 165.4.

### Synthesis of di-tert-butyl carbamimidoyl(3-iodo-5-(1,2,4,5-tetrazin-3-yl)benzyl)carbamate (11)

(3-iodo-5-(1,2,4,5-tetrazin-3-yl)phenyl)methanol (184mg, 0.59mmol) in 8 mL anhydrous THF was stirred under argon with 1,3-bis(*tert*-butoxycarboxyl)guanidine (167 mg, 0.64 mmol) and triphenylphosphine (310 mg, 1.17 mmol). DIAD (230 uL, 1.17 mmol) was added dropwise. First, the mixture turned dark, but after 15min, it was red/pink from the Tz. After 3h, LCMS showed complete conversion. The solvent was evaporated, and the resulting crude was reconstituted in EtOAc, washed with 2x water and 2x brine, dried over Mg₂SO₄, filtered and concentrated. The compound was dissolved in EtOAc and absorbed unto Celite. The compound was purified by flash chromatography (95:5 Heptane:EtOAc). Yield 186mg(45%). RF= 0.36 (8:2 Heptane:EtOAc). ¹H-NMR (CDCl₃, 400MHz): 1.45 (s, 9H), 1.52 (s, 9H), 5.23 (s, 2H), 8.05 - 8.08 (m, 1H), 8.60 - 8.63 (m, 1H), 8.89 (s, 1H), 9.25 (s, 1H), 9.43 (s, 1H), 10.24 (s, 1H). ¹³C-NMR (CDCl₃, 400 MHz): 28.1, 28.5, 46.9, 79.3, 85.0, 94.8, 127.2, 133.4, 135.9, 142.0, 142.2, 154.7, 158.2, 160.5, 163.7, 165.4.

### Synthesis of 1-(3-iodo-5-(1,2,4,5-tetrazin-3-yl)benzyl)guanidine (Tz-XX)

di-*tert*-butyl carbamimidoyl(3-iodo-5-(1,2,4,5-tetrazin-3-yl)benzyl)carbamate (30 mg, 0.054 mmol) was deprotected using 4 M HCl in dioxane (4 mL) for 41h, after which the residue was concentrated under vacuum. The crude residue was dissolved in 5 mL H₂O containing 0.1% TFA and was submitted to prep-HPLC. Fractions containing the pure product were combined and freeze-dried. Yield 17 mg (1xTFA: 69%, 2xTFA: 56%). ¹H-NMR(MeOD, 600MHz): 4.55 (s, 2H), 8.02 - 8.03 (m, 1H), 8.56 - 8.57 (m, 1H), 8.88 - 8.90 (m, 1H), 10.40 (s, 1H). ¹³C-NMR (MeOD, 600 MHz): 44.9, 95.8, 126.8, 136.0, 137.3, 141.3, 141.5, 158.9, 159.7, 166.4.

### Example 2

### Synthesis of cyclic unsaturated hydrocarbon with at least one double bond in the trans-configuration and their precursors

### Example 2.1

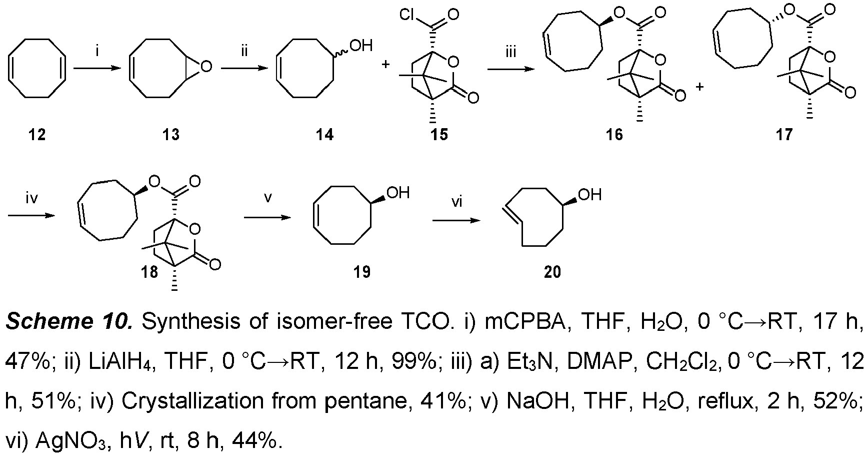

### (Z)-9-Oxabicyclo[6.1.0]non-4-ene (13)

*Cis,cis*-1,5-cyclooctadiene (22.0 g, 203.36 mmol, 1.00 equiv.) and dry CH₂Cl₂ (300 mL) were added to a 500 mL round-bottom flask. The mixture was cooled to 0 °C with an ice bath and mCPBA (45.57 g, 203.36 mmol, 1.00 equiv.) was added portion wise to give a white suspension. The mixture was allowed to reach room temperature and left stirring overnight. The mixture was filtered and washed with NaHCOs saturated solution (3 × 100 mL) and NaCl saturated solution (1 × 100 mL). The organic layer was collected, dried with MgSO₄, filtered and concentrated under reduced pressure. Purification by flash chromatography (n-heptane/EtOAc, 90:10) yielded (Z)-9-oxabicyclo[6.1.0]non-4-ene (11.82 g, 95.16 mmol, 47%) as a colorless oil. ¹H NMR (600 MHz, CDCl₃) δ 5.69 - 5.48 (m, 2H), 3.15 - 2.91 (m, 2H), 2.55 - 2.35 (m, 2H), 2.21 -2.08 (m, 2H), 2.08-1.86 (m, 4H); ¹³C NMR (151 MHz, CDCl₃) δ 129.00, 56.87, 28.25, 23.82.

### (Z)-Cyclooct-4-enol (14)

Lithium aluminum hydride tablets (3.26 g, 85.93 mmol, 3.00 equiv.) were added to an oven-dried 500 mL three-necked round-bottom flask. The flask was sealed and flushed with argon. The flask was cooled to 0 °C using an ice-bath and dry THF (120 mL) was added slowly while vigorously stirring to give a grey suspension. 1,2-Epoxy-5-cyclooctene (3.56 g, 28.64 mmol, 1.00 equiv.) in dry THF (10 mL) was added dropwise and the mixture was allowed to reach room temperature and stirred overnight. The mixture was cooled to 0 °C in an ice bath and quenched with EtOAc (120 mL). A saturated solution of Rochelle salt (100 mL) was added, and the mixture was stirred vigorously for 10 minutes. The mixture was transferred to a separatory funnel and the organic layer was collected. The aqueous layer was extracted with DCM (3 × 150 mL). The combined organic layers were washed with H₂O (200 mL), dried over MgSO₄, filtered and concentrated under reduced pressure to give (Z)-Cyclooct-4-enol (3.49 g, 28.45 mmol, 99%). ¹H NMR (600 MHz, CDCl₃) δ 5.75 - 5.63 (m, 1H), 5.61 - 5.52 (m, 1H), 3.86 - 3.75 (m, 1H), 2.36 - 2.24 (m, 1H), 2.20 - 2.04 (m, 3H), 1.97 (s, 1H), 1.93 - 1.88 (m, 1H), 1.86 - 1.81 (m, 1H), 1.75 - 1.68 (m, 1H), 1.67 - 1.59 (m, 1H), 1.56 - 1.46 (m, 2H); ¹³C NMR (151 MHz, CDCl₃) δ 130.23, 129.63, 72.85, 37.75, 36.36, 25.75, 24.97, 22.88.

### (Z)-cyclooct-4-en-1-yl (1R,4S)-4,7,7-trimethyl-3-oxo-2-oxabicyclo[2.2.1]heptane-1-carboxylate (Epimers mixture) (16)

(±)-(*Z*)-Cyclooct-4-enol (4.5 g, 35.65 mmol) was dissolved in dry CH₂Cl₂ (100 mL), to which was added DMAP (0.87 g, 7.13 mmol) and Et₃N (14.9 mL, 106.97 mmol). The solution was cooled to 0°C and (1*S*)-(-)-camphanic chloride (8.5 g, 38.22 mmol) was added portion wise to the mixture. The resulting solution was allowed to stir at room temperature for 17 hours. The mixture was washed with NaHCO₃ saturated solution (3 × 100 mL) and NaCl saturated solution (1 × 100 mL). The organic layer was collected, dried with MgSO₄, filtered and concentrated under reduced pressure to give 5.63 g (51%) of a mixture of the desired epimers. Recrystallization from pentane afforded 2.31 g (41%) of (*Z*)-cyclooct-4-en-1-yl (1*R*,4*S*)-4,7,7-trimethyl-3-oxo-2-oxabicyclo[2.2.1]heptane-1-carboxylate as crystals (needles). ¹H NMR (400 MHz, CDCl₃) δ 5.75 - 5.58 (m, 2H), 5.05 - 4.94 (m, 1H), 2.46 - 2.29 (m, 2H), 2.28 - 2.08 (m, 3H), 2.06 - 1.84 (m, 4H), 1.84 - 1.74 (m, 1H), 1.74 - 1.33 (m, 4H), 1.10 (s, 3H), 1.04 (s, 3H,). 0.95 (s, 3H); ¹³C NMR (101 MHz, CDCl₃) δ 178.46, 166.97, 130.05, 129.99, 129.59, 129.56, 91.23, 54.96, 54.21, 33.87, 33.85, 33.81, 30.69, 29.12, 29.10, 25.67, 25.65, 24.92, 24.88, 22.37, 22.35, 17.01, 16.95, 16.88, 9.84.

### (S,Z)-cyclooct-4-en-1-ol (19)

(*S,Z*)-cyclooct-4-en-1-yl (1*R*,4*S*)-4,7,7-trimethyl-3-oxo-2-oxabicyclo[2.2.1]heptane-1-carboxylate (0.26 g, 0.85 mmol) was dissolved in THF (10 mL), to which was added a solution of NaOH (0.2 g, 4.24 mmol) in H₂O (1 mL). The reaction was vigorously stirred at reflux for 2 hours. The mixture was quenched with H₂O (10 mL) and the aqueous layer was extracted with DCM (3 × 30 mL). The combined organic layers were washed with H₂O (20 mL), dried over MgSO₄, filtered and concentrated under reduced pressure to give (S,Z)-Cyclooct4enol (0.056 g, 52%) as a colorless oil. ¹H NMR (600 MHz, CDCl₃) δ 5.75 - 5.63 (m, 1H), 5.61 - 5.52 (m, 1H), 3.86 - 3.75 (m, 1H), 2.36 - 2.24 (m, 1H), 2.20 - 2.04 (m, 3H), 1.97 (s, 1H), 1.93 - 1.88 (m, 1H), 1.86 - 1.81 (m, 1H), 1.75 - 1.68 (m, 1H), 1.67 - 1.59 (m, 1H), 1.56 - 1.46 (m, 2H); ¹³C NMR (151 MHz, CDCl₃) δ 130.23, 129.63, 72.85, 37.75, 36.36, 25.75, 24.97, 22.88.

### (S,E)-cyclooct-4-en-1-ol (20)

A flash cartridge (220g, screw top, luer lock end fittings, Cat# FCSTLL-220-6) was packed with 8 cm silica (15-40µm) on the bottom and silver nitrate impregnated silica until the top. The column was flushed with 9:1 diethyl ether/n-heptane (500 mL) and the column was protected from light with aluminium foil. The cooling fence and UV lamps were turned on and after 10 minutes no detection of silver leakage was observed. Methyl benzoate (1 mL), (*S,Z*)-cyclooct-4-en-1-ol (1 g) and an additional 50 mL 9:1 diethyl ether/n-heptane solution were added to a round-bottom flask. The mixture was then added to the quartz flask. The pump was turned on (flowrate = 100 mL/min) and the photoreactor was turned on and photoisomerization was conducted for 8 hours. After 8 hours, the photoreactor was turned off and the column was dried by a stream of air. The silica was removed from the column and washed with 400 mL ammonia and 400 mL DCM. The mixture was stirred for 30 minutes, filtered and the organic layer was collected. The organic layer was washed with brine, dried with MgSO4, filtered and concentrated to give 0.44 g (44%) of the product as a yellowish oil (mixture of axial and equatorial isomers). Major equatorial ¹H NMR (400 MHz, CDCl₃) δ 5.57 (ddd, *J* = 15.3, 10.6, 4.1 Hz, 1H), 5.38 (ddd, *J* = 15.7, 10.9, 3.6 Hz, 1H), 3.50 - 3.40 (m, 1H), 2.32 (tq, *J* = 17.3, 5.1 Hz, 3H), 2.01 - 1.87 (m, 4H), 1.74 - 1.50 (m, 3H).Minor axial ¹H NMR (400 MHz, CDCl₃) δ 6.02 - 5.19 (m, 2H), 4.48 - 3.85 (m, 1H), 2.37 (dddd, *J* = 15.2, 10.5, 7.0, 4.8 Hz, 1H), 2.30 - 1.96 (m, 4H), 2.00 - 1.75 (m, 3H), 1.65 (dddd, *J* = 14.4, 12.8, 5.0, 1.7 Hz, 1H), 1.26 (dddd, *J* = 13.6, 10.9, 3.5, 0.9 Hz, 1H).

### Example 2.2

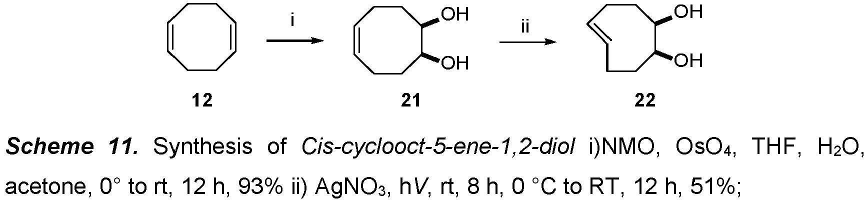

### Cis-Z-cyclooct-5-ene-1,2-diol (21)

To a stirred mixture of 1,5-cyclooctadiene (1 1.22 mL, 12.9 mmol), 4-methylmorpholine N-oxide (1.87 g, 13.75 mmol) and THF: H2 O : acetone (1:1:1) (90 mL) at 0 °C was added osmium tetroxide (12.7 mg, 0.05 mmol). After 12 h at 25 °C the reaction mixture was poured into an aqueous saturated solution of NaHSOs (60 mL), extracted with EtOAc (3 × 150 mL), washed with water (2 × 50 mL) and brine (50 mL). Drying (MgSO₄) and concentration followed by flash chromatography (silica, 30% EtOAc in heptane) afforded the desired compound 1.70 g (93%).¹H NMR (400 MHz, CDCl₃) δ 5.72 - 5.61 (m, 2H), 4.03 - 3.96 (m, 2H), 2.56 - 2.44 (m, 2H), 2.10 - 1.96 (m, 4H), 1.86-1.74 (m, 2H); ¹³C NMR (101 MHz, CDCl₃) δ 130.09, 75.18, 32.08, 23.11.

### Cis-E-cyclooct-5-ene-1,2-diol (22)

A flash cartridge (220g, screw top, luer lock end fittings, Cat# FCSTLL-220-6) was packed with 8 cm silica (15-40µm) on the bottom and silver nitrate impregnated silica until the top. The column was flushed with 9:1 diethyl ether/n-heptane (500 mL) and the column was protected from light with aluminium foil. The cooling fence and UV lamps were turned on and after 10 minutes no detection of silver leakage was observed. Methyl benzoate (1 mL), *cis*-Z-cyclooct-5-ene-1,2-diol (1 g) and an additional 50 mL 9:1 diethyl ether/n-heptane solution were added to a round-bottom flask. The mixture was then added to the quartz flask. The pump was turned on (flowrate = 100 mL/min) and the photoreactor was turned on and photoisomerization was conducted for 8 hours. After 8 hours, the photoreactor was turned off and the column was dried by a stream of air. The silica was removed from the column and washed with 400 mL ammonia and 400 mL DCM. The mixture was stirred for 30 minutes, filtered and the organic layer was collected. The organic layer was washed with brine, dried with MgSO4, filtered and concentrated to give 0.51 g (51%) of the product as a colorless oil.

### Example 3

### Synthesis of unsaturated heterocycle, with at least one double bond in the trans-configuration and their precursors

### Example 3.1

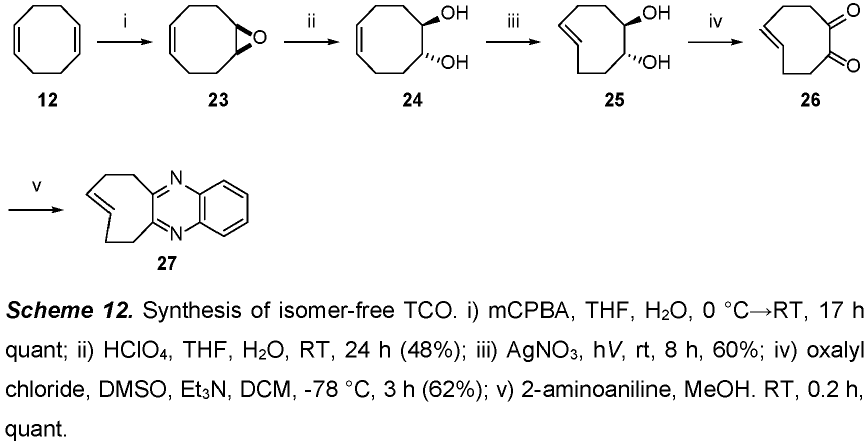

**(Z)-9-Oxabicyclo[6.1.0]non-4-ene (23).** Cylcooctadiene (1.00 g, 9.24 mmol) was dissolved in dry DCM (20 mL) and cooled to 0°C, to which was added mCBPA (2.07 g, 9.24 mmol) in a portion wise manner. After addition, the reaction was stirred for 30 min at 0°C and afterwards the reaction was allowed to warm to room temperature. The reaction was stirred for 17 h. The reaction was quenched with 2M K₂CO₃ (20 mL) and the crude was extracted with 3 × DCM (15 mL). The combined organic phases was washed with K₂CO₃ (25 mL), and brine (25 mL). The solvent was evaporated, and the crude was without purification used in the next reaction.

**(*Z*)-Cyclooct-5-ene-1,2-diol (24).** 25 (1.15 g, 9.24 mmol) was dissolved in THF/water (3:2 v/v, 50 mL) mixture containing HClO₄ (250 µL, 2.90 mmol). The reaction was stirred at room temperature for 24 h. Water (10 mL) was added to the reaction mixture, and the product was extracted with 3 × Et₂O (20 mL). The combined ether fractions were washed with 2 × 2M NaHCOs (25 mL). The crude was purified by CombiFlash, which yielded a clear viscous oil. (624 mg, 4.40 mmol, 48%). ¹H NMR (600 MHz, Chloroform-d) δ 5.64 - 5.56 (m, 2H), 3.72 - 3.65 (m, 2H), 2.41 - 2.33 (m, 2H), 2.17 - 2.09 (m, 4H), 1.63 - 1.55 (m, 2H); ¹³C NMR (151 MHz, Chloroform-d) δ 129.3, 74.1, 33.6, 22.9.

### (E)-Cyclooct-5-ene-1,2-diol (25)

The compound was obtained as described in Royzen, M.; Yap, G. P. A.; Fox, J. M. A Photochemical Synthesis of Functionalized trans-Cyclooctenes Driven by Metal Complexation. Journal of the American Chemical Society 2008, 130, 3760-3761. A flash cartridge (220g, screw top, luer lock end fittings, Cat# FCSTLL-220-6) was packed with 8 cm silica (15-40µm) on the bottom and silver nitrate impregnated silica until the top. The column was flushed with 9:1 diethyl ether/n-heptane (500 mL) and the column was protected from light with aluminium foil. The cooling fence and UV lamps were turned on and after 10 minutes no detection of silver leakage was observed. Methyl benzoate (1 mL), **27** (1 g) and an additional 50 mL 9:1 diethyl ether/n-heptane solution were added to a round-bottom flask. The mixture was then added to the quartz flask. The pump was turned on (flowrate = 100 mL/min) and the photoreactor was turned on and photoisomerization was conducted for 8 hours. After 8 hours, the photoreactor was turned off and the column was dried by a stream of air. The silica was removed from the column and washed with 400 mL ammonia and 400 mL DCM. The mixture was stirred for 30 minutes, filtered and the organic layer was collected. The organic layer was washed with brine, dried with MgSO4, filtered and concentrated to give 0.60 g (60%) of the product as a yellowish oil (mixture of axial and equatorial isomers); ¹H NMR (CDCl₃, 400 MHz) δ 5.45-5.36 (m, 2H), 3.58 (bs, 2H), 3.47 (m, 2H), 2.35-2.21 (m, 4H), 2.02-1.98 (m, 2H), 1.76-1.65 (m, 2H). ¹³C NMR (CDCl3, 100 MHz) δ 132.6, 76.0, 40.7, 32.6.

### (E)-Cyclooct-5-ene-1,2-dione (26)

Under Argon atmosphere, at -78 °C, a solution of DMSO (1.05 mL, 14.77 mmol) in dry DCM (30 mL) was added drop wise to a solution of (COCl)₂ (736 µL, 8.44 mmol) in dry DCM (15 mL). After addition, the reaction mixture is stirred for 30 min at -78 °C. A solution of **28** (500 mg, 3.52 mmol) in dry DCM (10 mL) is added drop wise and the reaction mixture is stirred an additional 30 minutes at -78°C. Et₃N (4.90 mL, 35.16 mmol) is added dropwise and the reaction mixture was kept at -78 °C for an additional 60 minutes. The reaction mixture was allowed to warm to room temperature and was stirred an additional hour. The reaction mixture is washed with water (50 mL), 2 × 0.5 M HCl_{(aq)} (50 mL) and brine (30 mL). The organic phase is dried over MgSO₄ and evaporated, which yielded the desired compound as a clear liquid. (302 mg, 2.19 mmol, 62%).

### (E)-6,7,10,11-Tetrahydrocycloocta[b]quinoxaline (27)

The compound was obtained following the procedure reported in Delpivo, C.; Micheletti, G.; Boga, C. A Green Synthesis of Quinoxalines and 2,3-Dihydropyrazines. Synthesis 2013, 45, 1546-1552. A 50 mL round-bottomed flask was charged with a solution of the appropriate **29** (138 mg, 1 mmol) in MeOH (3 mL). To this solution was added the respective 2-aminoaniline (110 mg, 1 mmol) and the mixture was stirred at r.t. After 30 min, the crude mixture was extracted with CH₂Cl₂ (3 × 10 mL). The combined organic layers were dried (MgSO4) and after filtration, the solvent was removed to give 0.2 g (97%) the desired product. ¹H NMR (400 MHz, CDCl₃) δ 8.23 - 7.93 (m, 2H), 7.77 - 7.55 (m, 2H), 6.19 - 5.93 (m, 1H), 5.24 - 5.09 (m, 1H), 3.40 - 3.23 (m, 2H), 3.22 - 3.12 (m, 1H), 3.02 (td, *J* = 12.8, 2.8 Hz, 1H), 2.80 - 2.69 (m, 1H), 2.69 - 2.54 (m, 1H), 2.50 - 2.38 (m, 1H), 2.36 - 2.25 (m, 1H).

### Example 3.2

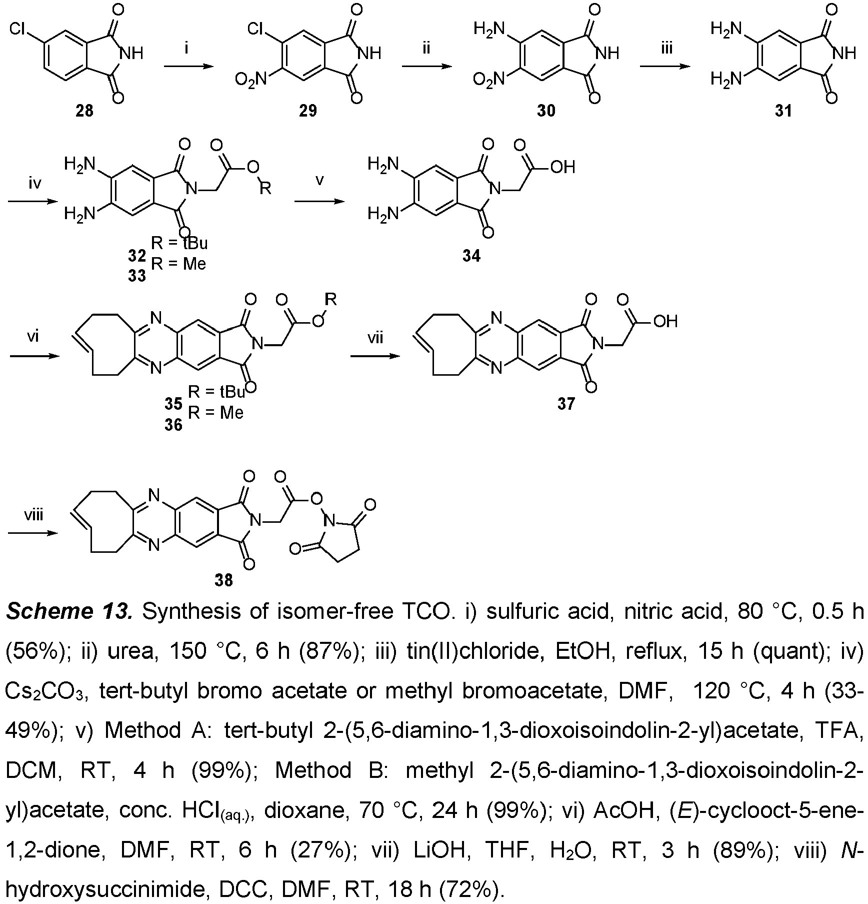

### 5-Chloro-6-nitroisoindoline-1,3-dione (29)

The compound was synthesized according to the literature.1 A mixture of concentrated sulfuric acid (33.1 mL) and fuming nitric acid (2.26 mL) was added dropwise to 4-chloro-phthalimide (5.00 g, 27.53 mmol). The reaction mixture was heated at 80 °C for 0.5 h. After cooling to r.t. the dark red solution was poured into ice. The resulted yellow precipitate was collected by filtration, washed with water, dried in vacuum affording a pale-yellow solid that was recrystallized from EtOH to give 3.5 g (56% yield) of the desired product. Rf = 0.45 (n-Heptane/EtOAc 60/40); ¹H NMR (400 MHz, DMSO) δ 11.87 (s, 1H), 8.51 (s, 1H), 8.27 (s, 1H); ¹³C NMR (101 MHz, DMSO) δ 167.21, 167.17, 151.86, 136.54, 133.00, 130.89, 126.60, 120.20.

### 5-Amino-6-nitroisoindoline-1,3-dione (30)

The compound was synthesized according to the literature.1 A mixture of 5-chloro-6-nitroisoindoline-1,3-dione (2.4 g, 10.59) and urea (6.36 g, 105.92 mmol) was stirred and heated to 150°C. under argon for 6 h. After cooled to rt, the solid was suspended in hot water (80°C.), filtered, washed with hot water (3x50 mL). The solid was recrystallized from EtOH to give 1.92 g (87%) of a yellow solid. Rf = 0.28 (n-Heptane/EtOAc 60/40); ¹H NMR (600 MHz, DMSO) δ 11.43 (s, 1H), 8.56 - 8.05 (m, 3H), 7.40 (s, 1H); ¹³C NMR (151 MHz, DMSO) δ 168.01, 150.64, 138.24, 132.50, 122.38, 117.74, 114.65.

### 5,6-Diaminoisoindoline-1,3-dione (31)

The compound was synthesized according to the literature.1 To a suspension of 4-amino-5-nitro-phthalimide (1.0 g, 4.27 mmol) and Tin(II) chloride (4.57 g, 24.14 mmol) in ethanol (30 mL) was refluxed under argon for 15 h. The resulted bright red suspension was cooled to r.t., the red solid was collected by filtration, washed with ethanol and dried in vacuum to give a red orange solid. Rf = 0.15 (95/5 DCM/MeOH); ¹H NMR (400 MHz, DMSO) δ 10.25 (s, 1H), 6.81 (s, 2H), 5.50 (s, 4H); ¹³C NMR (101 MHz, DMSO) δ 170.72, 140.30, 122.83, 107.03.

### tert-Butyl 2-(5,6-diamino-1,3-dioxoisoindolin-2-yl)acetate (32)

To a suspension of Cs₂CO₃ (1.93 g, 5.92 mmol) and 5,6-diaminoisoindoline-1,3-dione (1.0 g, 5.64 mmol) in anhydrous DMF (5 mL) was added *tert*-butyl bromoacetate (0.87 mL, 5.92 mmol). The reaction was heated at 120 °C for 4 h under argon. The mixture was cooled to r.t. and water (20 mL) was added. The reaction mixture was extracted with DCM (3 × 50 mL). The organic phase was dried over anhydrous MgSO4, filtered and concentrated under reduced pressure. The resulting residue was purified using flash chromatography (98/2 DCM/MeOH) to yield 0.8 g (49%) of the desired compound as a yellow solid. Rf= 0.42 (DCM/MeOH 93/7); ¹H NMR (600 MHz, DMSO) δ 6.89 (s, 2H), 5.61 (s, 4H), 4.11 (s, 2H), 1.40 (s, 9H); ¹³C NMR (151 MHz, DMSO) δ 168.63, 167.62, 140.46, 121.55, 107.34, 82.08, 40.55, 28.08.

### Methyl 2-(5,6-diamino-1,3-dioxoisoindolin-2-yl)acetate (33)

To a suspension of Cs₂CO₃ (1.93 g, 5.92 mmol) and 5,6-diaminoisoindoline-1,3-dione (1.0 g, 5.64 mmol) in anhydrous DMF (5 mL) was added methyl bromoacetate (0.63 mL, 5.92 mmol). The reaction was heated at 120 °C for 4 h under argon. The mixture was cooled to r.t. and water (20 mL) was added. The reaction mixture was extracted with DCM (3 × 50 mL). The organic phase was dried over anhydrous MgSO4, filtered and concentrated under reduced pressure. The resulting residue was purified using flash chromatography (98/2 DCM/MeOH) to yield 0.45 g (33%) of the desired compound as a yellow solid. Rf= 0.40 (DCM/MeOH 93/7); ¹H NMR (400 MHz, DMSO) δ 6.90 (s, 2H), 5.63 (s, 4H), 4.25 (s, 2H), 3.67 (s, 3H); ¹³C NMR (101 MHz, DMSO) δ 169.13, 168.50, 140.52, 121.47, 107.37, 52.72, 38.69.

### 2-(5,6-diamino-1,3-dioxoisoindolin-2-yl)acetic acid (34)

### Method A

To a solution of *tert*-butyl 2-(5,6-diamino-1,3-dioxoisoindolin-2-yl)acetate (0.4 g, 1.37 mmol) in DCM (6 mL) was added TFA (2 mL). The reaction was stirred at r.t. for 4 h and then concentrated under reduced pressure to give 0.32 g (99%) of the desired compound as a yellow solid. Rf = 0.29 (DCM/MeOH 99/1 +0.1% AcOH); ¹H NMR (400 MHz, DMSO) δ 6.90 (s, 2H), 4.13 (s, 2H); ¹³C NMR (101 MHz, DMSO) δ 169.95, 168.60, 140.16, 121.80, 107.71, 38.86.

### Method 8

To a solution of methyl 2-(5,6-diamino-1,3-dioxoisoindolin-2-yl)acetate (0.25 g, 1.01 mmol) in dioxane (6 mL) was added conc. HCl (1 mL). The reaction was stirred at 70 °C for 24 h and then concentrated under reduced pressure to give 0.23 g (99%) of the desired compound as a yellow solid. Rf = 0.29 (DCM/MeOH 99/1 +0.1% AcOH); ¹H NMR (400 MHz, DMSO) δ 6.90 (s, 2H), 4.13 (s, 2H); 13C NMR (101 MHz, DMSO) δ 169.95, 168.60, 140.16, 121.80, 107.71, 38.86.

### tert-Butyl (E)-2-(1,3-dioxo-1,3,6,7,10,11-hexahydro-2H-cycloocta[b]pyrrolo [3,4-g]quinoxalin-2-yl)acetate (35)

A solution of *tert*-butyl 2-(5,6-diamino-1,3-dioxoisoindolin-2-yl)acetate (0.4 g, 1.40 mmol) and acetic acid (0.8 mL, 14.03 mmol) in DMF (5 mL) and water (1 mL) was added to (*E*)-cyclooct-5-ene-1,2-dione (0.21 g, 1.54 mmol). The reaction was stirred at r.t. under dark for 6 h. The solution was diluted with 10 mL of water and purified directly by preparative HPLC to give 0.09 g (17%) of the desired compound as a yellow solid. Rf = 0.36 (Heptane/EtOAc 75/25); ¹H NMR (400 MHz, CDCl3) δ 8.61 - 8.37 (m, 2H), 6.10 (ddd, J = 15.8, 10.7, 3.9 Hz, 1H), 5.15 (ddd, J = 16.3, 9.5, 5.8 Hz, 1H), 4.42 (s, 2H), 3.40 - 3.28 (m, 2H), 3.24 (dd, J = 10.9, 5.1 Hz, 1H), 3.08 (td, J = 12.8, 2.8 Hz, 1H), 2.85 - 2.66 (m, 2H), 2.45 (q, J = 11.9 Hz, 1H), 2.39 - 2.24 (m, 1H), 1.48 (s, 9H).

### Methyl (E)-2-(1,3-dioxo-1,3,6,7,10,11-hexahydro-2H-cycloocta[b]pyrrolo [3,4-g]quinoxalin-2-yl)acetate (36)

A solution of methyl 2-(5,6-diamino-1,3-dioxoisoindolin-2-yl)acetate (0.38 g, 1.40 mmol) and acetic acid (0.8 mL, 14.03 mmol) in DMF (5 mL) and water (1 mL) was added to (*E*)-cyclooct-5-ene-1,2-dione (0.21 g, 1.54 mmol). The reaction was stirred at r.t. under dark for 6 h. The solution was diluted with 10 mL of water and purified directly by preparative HPLC to give 0.15 g (26%) of the desired compound as a yellow solid. Rf = 0.31 (Heptane/EtOAc 75/25); ¹H NMR (600 MHz, CDCl3) δ 8.53 - 8.46 (m, 2H), 6.10 (ddd, J = 15.7, 10.8, 3.9 Hz, 1H), 5.14 (ddd, J = 16.2, 9.5, 5.8 Hz, 1H), 4.53 (s, 2H), 3.79 (s, 3H), 3.34 (ddt, J = 14.9, 11.1, 5.4 Hz, 2H), 3.24 (dd, J = 11.2, 5.1 Hz, 1H), 3.08 (td, J = 12.8, 2.8 Hz, 1H), 2.84 - 2.65 (m, 2H), 2.50 - 2.39 (m, 1H), 2.33 (ddd, J = 11.1, 9.3, 5.5 Hz, 1H).

### (E)-2-(1,3-Dioxo-1,3,6,7,10,11-hexahydro-2H-cycloocta[b] pyrrolo [3,4-g]quinoxalin-2-yl)acetic acid (37)

A solution of 2-(5,6-diamino-1,3-dioxoisoindolin-2-yl)acetic acid (0.33 g, 1.40 mmol) and acetic acid (0.8 mL, 14.03 mmol) in DMF (5 mL) and water (1 mL) was added to (*E*)-cyclooct-5-ene-1,2-dione (0.21 g, 1.54 mmol). The reaction was stirred at r.t. under dark for 6 h. The solution was diluted with 10 mL of water and purified directly by preparative HPLC to give 0.13 g (27%) of the desired compound as a yellow solid. Rf = 0.45 (DCM/MeOH 99/1 +0.1% AcOH); ¹H NMR (600 MHz, DMSO) δ 13.33 (s, 1H), 8.47 (d, J = 9.4 Hz, 2H), 6.18 (ddd, J = 17.2, 10.7, 3.8 Hz, 1H), 5.17 - 5.08 (m, 1H), 4.42 (s, 2H), 3.52 - 3.43 (m, 1H), 3.23 (dd, J = 9.2, 3.1 Hz, 2H), 3.12 (dd, J = 11.2, 5.1 Hz, 1H), 2.71 (tt, J = 17.0, 6.0 Hz, 2H), 2.32 (qd, J = 11.0, 7.6 Hz, 1H), 2.17 (tt, J = 11.3, 5.4 Hz, 1H); ¹³C NMR (151 MHz, DMSO) δ 169.10, 166.50, 161.93, 161.59, 143.21, 143.07, 137.16, 132.73, 130.81, 130.63, 125.34, 125.12, 46.31, 41.35, 34.30, 28.33.

### 2,5-Dioxopyrrolidin-1-yl (E)-2-(1,3-dioxo-1,3,6,7,10,11-hexahydro-2H-cycloocta[b]pyrrolo[3,4-g]quinoxalin-2-yl)acetate (38)

To a solution of (*E*)-2-(1,3-dioxo-1,3,6,7,10,11-hexahydro-2*H*-cycloocta[*b*] pyrrolo [3,4-*g*]quinoxalin-2-yl)acetic acid (0.06 g, 0.18 mmol) in anhdrous DMF (3mL) under argon was added DCC (0.04 g, 0.19 mmol) and then N-hydroxysuccinimide (0.04 g, 0.35 mmol). The reaction was stirred overnight at r.t. under argon. EtOAc(10 mL) was added, and the organic layer washed with water (2 × 10 mL) and brine (2 × 10 mL). The organic phase was dried over anhydrous MgSO4, filtered and concentrated under reduced pressure. The residue was solubilized in dry cold MECN (4 mL), filtered and concentrated to give a yellow solid. The solid was triturated in diethyl ether and filtered to give 0.056 g (72%) of the desired compound as a yellow solid. Rf = 0.41 (n-Heptane/EtOAc 50/50); ¹H NMR (400 MHz, CDCl3) δ 8.56 - 8.47 (m, 2H), 6.10 (td, J = 12.1, 5.9 Hz, 1H), 5.25 - 5.07 (m, 1H), 4.87 (s, 2H), 3.40 - 3.30 (m, 2H), 3.30 - 3.21 (m, 1H), 3.15 - 3.04 (m, 1H), 2.92 - 2.68 (m, 6H), 2.45 (q, J = 11.9 Hz, 1H), 2.33 (dt, J = 12.1, 6.1 Hz, 1H); ¹³C NMR (101 MHz, CDCl3) δ 168.16, 165.58, 163.15, 161.67, 161.31, 143.40, 143.24, 136.54, 132.67, 130.16, 130.00, 126.26, 126.07, 46.74, 41.81, 37.05, 34.19, 28.26, 25.55.

### Example 3.3

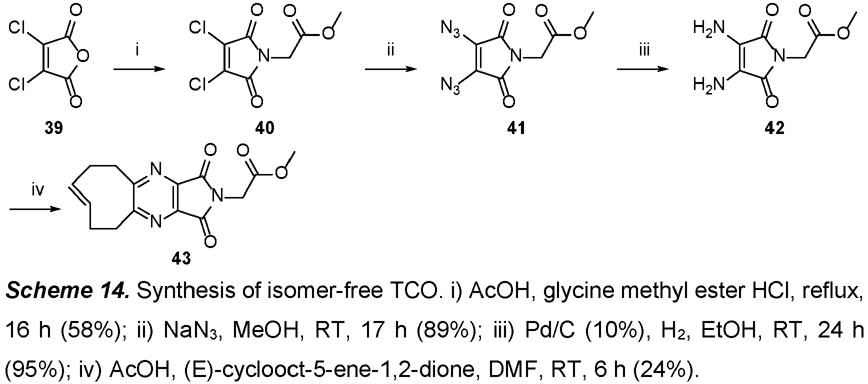

### Methyl 2-(3,4-dichloro-2,5-dioxo-2,5-dihydro-1H-pyrrol-1-yl)acetate (40)

To a solution of dichloromaleic anhydride (5.0 g, 29.95 mmol) in acetic acid (100 mL) was added glycine methyl ester hydrochloride (4.51 g, 35.94 mmol). The mixture was heated at reflux during 16 h. The solvent was removed under reduced pressure. Water (100 mL) was added, and the resulting suspension was stirred for 1 h. The solid was filtered to give 4.15 g (58%) of the desired product as a beige solid. ¹H NMR (400 MHz, DMSO) δ 4.41 (s, 2H), 3.70 (s, 3H); ¹³C NMR (101 MHz, DMSO) δ 167.96, 162.82, 133.39, 53.09, 40.13.

### Methyl 2-(3,4-diazido-2,5-dioxo-2,5-dihydro-1H-pyrrol-1-yl)acetate (41)

A solution of 2-(3,4-dichloro-2,5-dioxo-2,5-dihydro-1*H*-pyrrol-1-yl)acetate (4.15 g, 17.43 mmol) and sodium azide (2.72 g, 41.84 mmol) in methanol (70 mL) was stirred overnight. The solvent was removed under vacuum, the residue dissolved in dichloromethane (50 mL), washed with water (2 × 30 mL) and brine (2 × 30 mL), dried over anhydrous sodium sulphate, and the solvent removed by vacuum to give the compound (3.89 g, 89%) as a yellow solid. ¹H NMR (400 MHz, DMSO) δ 4.34 (s, 2H), 3.70 (s, 3H); ¹³C NMR (101 MHz, DMSO) δ 168.14, 164.29, 120.16, 53.03, 39.25.

### Methyl 2-(3,4-diazido-2,5-dioxo-2,5-dihydro-1H-pyrrol-1-yl)acetate (42)

To solution of the 2-(3,4-diazido-2,5-dioxo-2,5-dihydro-1*H*-pyrrol-1-yl)acetate (1.6 g, 6.37 mmol) in EtOH (40 mL) was added 10% Pd/C (0.34 g, 0.31 mmol). The mixture was stirred at rt and put under H2 atmosphere for 24 h. The catalyst was then filtered on a celite pad and the solvent removed to give 1.2 g (95%) of the desired compound as a red oil. NMR ok. ¹H NMR (400 MHz, DMSO) δ 4.94 (s, 4H), 4.09 (s, 2H), 3.65 (s, 4H); ¹³C NMR (101 MHz, DMSO) δ 169.38, 169.16, 118.89, 52.63, 38.46.

### Methyl (E)-2-(1,3-dioxo-1,3,5,6,9,10-hexahydro-2H-cycloocta[b]pyrrolo[3,4-e]pyrazin-2-yl)acetate (43)

A solution of methyl 2-(3,4-diazido-2,5-dioxo-2,5-dihydro-1*H*-pyrrol-1-yl)acetate (0.28 g, 1.40 mmol) and acetic acid (0.8 mL, 14.03 mmol) in DMF (5 mL) and water (1 mL) was added to (*E*)-cyclooct-5-ene-1,2-dione (0.21 g, 1.54 mmol). The reaction was stirred at r.t. under dark for 6 h. The solution was diluted with 10 mL of water and purified directly by preparative HPLC to give 0.1 g (24%) of the desired compound as a yellow solid. Rf = 0.28 (Heptane/EtOAc 75/25); ¹H NMR (600 MHz, CDCl3) δ 6.11 (ddd, J = 15.7, 10.8, 3.9 Hz, 1H), 5.13 (ddd, J = 16.2, 9.5, 5.8 Hz, 1H), 4.53 (s, 2H), 3.77 (s, 3H), 3.35 (ddt, J = 14.9, 11.1, 5.4 Hz, 2H), 3.26 (dd, J = 11.2, 5.1 Hz, 1H), 3.08 (td, J = 12.8, 2.8 Hz, 1H), 2.85 - 2.63 (m, 2H), 2.50 - 2.39 (m, 1H), 2.32 (ddd, J = 11.1, 9.3, 5.5 Hz, 1H): ¹³C NMR (151 MHz, CDCl3) δ 167.20, 164.20, 163.25, 143.86, 128.71, 52.87, 38.83, 35.36, 27.06.

### Example 3.4

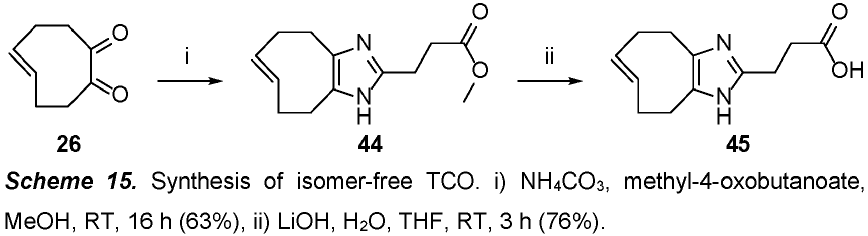

### Methyl (E)-3-(4,5,8,9-tetrahydro-1H-cycloocta[d]imidazol-2-yl)propanoate (44)

In a 15 mL screw neck vial ammonium bicarbonate (384 mg, 4.86 mmol) and methyl-4-oxobutanoate (85%, 331 mg, 2.43 mmol) was stirred in 2 mL MeOH at rt for 15 min. (*E*)-cyclooct-5-ene-1,2-dione (305 mg, 2.21 mmol) in 3 mL MeOH was added to the suspension and stirred for 16 h. The reaction mixture was diluted with 0.1% TFA_{(aq.)} and submitted to preparative HPLC to yield methyl (E)-3-(4,5,8,9-tetrahydro-1H-cycloocta[d]imidazol-2-yl)propanoate as a beige, viscous liquid (325 mg, 1.39 mmol, 63%). ¹H NMR (599.65 MHz, MeOD) δ = 5.64 - 5.56 (m, 2H), 3.70 (s, 3H), 3.20 - 3.15 (m, 2H), 2.95 (dt, J = 13.8, 6.5 Hz, 2H), 2.88 (t, J = 7.0 Hz, 2H), 2.76 - 2.70 (m, 2H), 2.35 (d, J = 6.1 Hz, 2H), 2.28 (h, J = 6.7 Hz, 2H). ¹³C NMR (150.78 MHz, MeOD) δ = 173.2, 136.0, 129.9, 56.6, 52.5, 31.5, 30.81, 29.0, 22.1.

### (E)-3-(4,5,8,9-Tetrahydro-1H-cycloocta[d]imidazol-2-yl)propanoic acid (45)

In a 5 mL screw neck vial 1M LiOH solution (861 µL, 861 µmol) was added to methyl (*E*)-3-(4,5,8,9-tetrahydro-1*H*-cycloocta[*d*]imidazol-2-yl)propanoate (100 mg, 287 µmol) in 2.5 mL THF/water (3:2 v/v). The suspension was stirred at rt for 3 h. The reaction was quenched with 1M TFA_{(aq.)} and submitted to preparative HPLC to yield (E)-3-(4,5,8,9-Tetrahydro-1H-cycloocta[d]imidazol-2-yl)propanoic acid as a light yellow, amorphous solid. (73 mg, 218 µmol, 76%). ¹H NMR (599.65 MHz, DMSO) δ = 12.52 (s, 1H), 5.57 - 5.48 (m, 2H), 3.03 (t, J = 7.2 Hz, 2H), 2.88 (dt, J = 14.2, 6.4 Hz, 2H), 2.77 (t, J = 7.2 Hz, 2H), 2.65 (ddd, J = 14.4, 6.7, 4.9 Hz, 2H), 2.24 (ddd, J = 11.5, 9.4, 4.5 Hz, 2H), 2.16 (ddd, J = 12.1, 7.4, 4.3 Hz, 2H). ¹³C NMR (150.78 MHz, DMSO) δ = 172.6, 142.67, 134.9, 127.6, 30.5, 29.2, 27.8, 20.8.

### Example 3.4

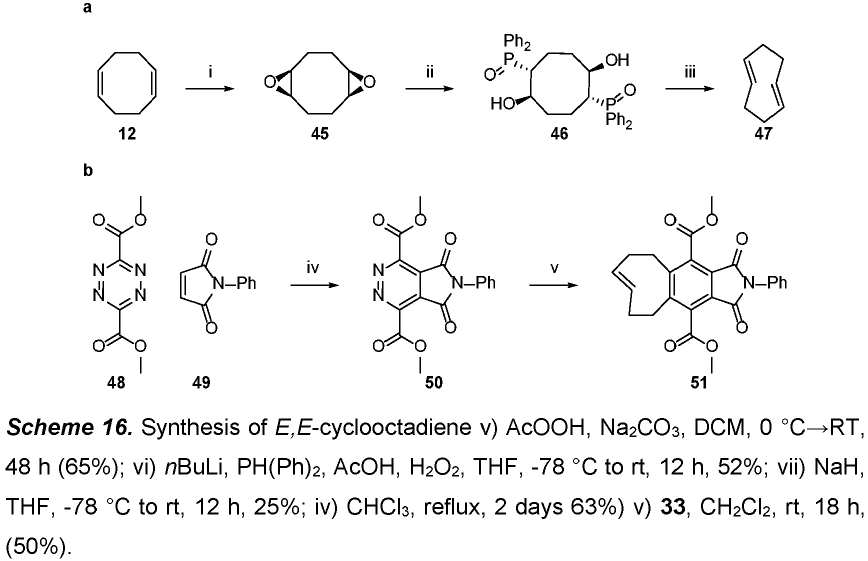

### cis,cis-5,10-Dioxatricyclo[7.1.0.04,6]decane (45)

The compound was synthesized as described in Stöckmann, H.; Neves, A. A.; Day, H. A.; Stairs, S.; Brindle, K. M.; Leeper, F. J. (E,E)-1,5-Cyclooctadiene: a small and fast click-chemistry multitalent. Chemical Communications 2011, 47, 7203-7205. Sodium carbonate (20.5 g, 50.84 mmol, 1.1 eq) was added to a stirred solution of (Z,Z)-1,5-cyclooctadiene (5 g, 46.2 mmol, 1.0 eq) in DCM (25 mL). The suspension was cooled to 0 _{°}C and peracetic acid (36%, 17.9 mL, 97.1 mmol, 2.1 eq) was added. The mixture was warmed to r.t. and stirred until the reaction had gone to completion, after 48 hours. The reaction was quenched with sat. Na2CO3 solution, followed by extraction with EtOAc (4 × 25 mL). The combined organic layers were dried over MgSO4, concentrated and distilled to give cis,cis-5,10-dioxatricyclo[7.1.0.04,6]decane (4.19 g, 29.89 mmol, 65%). ¹H NMR (400 MHz, CDCl₃) δ 2.97 - 2.88 (m, 4H), 2.00 - 1.89 (m, 4H), 1.89 - 1.77 (m, 4H); ¹³C NMR (101 MHz, CDCl₃) δ 56.02, 22.01.

### ((trans,trans)-2,6-Dihydroxycyclooctane-1,5-diyl)bis(diphenylphosphine oxide) (46)

The compound was synthesized as described in Stöckmann, H.; Neves, A. A.; Day, H. A.; Stairs, S.; Brindle, K. M.; Leeper, F. J. (E,E)-1,5-Cyclooctadiene: a small and fast click-chemistry multitalent. Chemical Communications 2011, 47, 7203-7205. To a solution of diphenylphosphine (10.43 mL, 11.16 g, 59.92 mmol, 2.1 eq.) and cis,cis-5,10-dioxatricyclo-[7.1.0.04,6]decane (4.0 g, 28.5 mmol, 1.0 eq.) in THF (120 mL), BuLi (2 M solution in hexanes,29.96 mL, 59.92 mmol, 2.1 eq.) was added dropwise at -78 _{°}C. The mixture was stirred for 1 h, allowed to warm to room temperature and stirred for 12 h. The brown solution was cooled to 0 °C, diluted with THF (86 mL) and quenched by the addition of AcOH (4.9 mL, 5.14 g, 85.60 mmol, 3 eq) and H₂O₂ (30%, 8.7 mL, 85.60 mmol, 3 eq). The mixture became clear, followed by the formation of a white precipitate. The mixture was removed from the ice bath and stirred vigorously at 25 _{°}C for 2 h The suspension was then transferred to a separatory funnel, EtOAc (150 mL) and H₂O (50 mL) were added, the aqueous layer removed, and the organic layer washed with brine (2 × 20 mL). The precipitate was filtered off, which yield ((*trans,trans*)-5,8-dihydroxycyclooctane-1,4-diyl)bis(diphenylphosphine oxide). The combined organic layers were dried over MgSO4, filtered and concentrated to afford a white solid. The remaining AcOH was azeotropically removed with toluene to yield an isomeric mixture of phosphine oxides (8.14 g, 14.9 mmol, 52%). Spectroscopic data were in agreement with previously published data.

### (E,E)-1,5-Cyclooctadiene (47)

The compound was synthesizedas described in Stöckmann, H.; Neves, A. A.; Day, H. A.; Stairs, S.; Brindle, K. M.; Leeper, F. J. (E,E)-1,5-Cyclooctadiene: a small and fast click-chemistry multitalent. Chemical Communications 2011, 47, 7203-7205. The mixture of phosphine oxides (6.00 g, 11.0 mmol, 1.0 eq) was dissolved in DMF (30 mL) with stirring under an Ar atmosphere. The clear solution became cloudy and NaH (60% in mineral oil, 1.44 g, 34.2 mmol, 3.1 eq) was added in portions over 5 min at r.t. under a positive Ar stream. The reaction mixture was stirred for 12 h at room temperature, cooled to 0 _{°}C, diluted with pentane (50 mL) and quenched with half-saturated NH4Cl (22 mL). The aqueous layer was extracted with pentane (50 mL) and the combined organic layers were washed with water (5 × 22 mL) and brine (1 × 22 mL) to give (E,E)-1,5-cyclooctadiene (25% yield). Spectroscopic data were in agreement with previously published data.

### Dimethyl 6-benzyl-5,7-dioxo-6,7-dihydro-5H-pyrrolo[3,4-d]pyridazine-1,4-dicarboxylate (50)

Dimethyl 1,2,4,5-tetrazine-3,6-dicarboxylate (50 mg, 0.25 mmol) was dissolved in CHCl₃ (1 mL), to which was added 1-phenyl-1*H*-pyrrole-2,5-dione (53 mg, 0.30 mmol) and the reaction was stirred for two days at reflux. Upon reaction, the dihydropyridazine spontaneously oxidized to the corresponding pyridazine. The reaction was cooled down to room temperature, upon complete consumption of dimethyl 1,2,4,5-tetrazine-3,6-dicarboxylate. The product was purified by CombiFlash, which yielded a pale solid (54 mg, 0.16 mmol, 63%). ¹H NMR (600 MHz, CDCl₃) δ 7.54 (dd, J = 8.3, 6.8 Hz, 2H), 7.50 - 7.47 (m, 1H), 7.42 - 7.38 (m, 2H), 4.17 (s, 6H). ¹³C NMR (151 MHz, CDCl₃) δ 162.5, 162.2, 159.2, 149.5, 138.2, 130.3, 129.7, 126.5, 54.3.

### Dimethyl (E)-1,3-dioxo-2-phenyl-2,3,5,6,9,10-hexahydro-1H-cycloocta[f]isoindole-4,11-dicarboxylate (51)

To a solution of 35 (20 mg, 0.06 mmol) in CH₂Cl₂ was added compound **33** in pentane (0.06 mmol). The solution was stirred ra room temperature for 18 h. The volatiles were then removed under reduced pressure. Purification by flash chromatography afforded 12 mg (50%) of the desired product as a white solid. ¹H NMR (400 MHz, CDCl₃) δ 7.45 (dd, *J* = 8.4, 6.9 Hz, 2H), 7.37 (t, *J* = 7.4 Hz, 1H), 7.35 - 7.30 (m, 2H), 5.77 (t, *J* = 5.5 Hz, 2H), 3.89 (s, 6H), 2.61 - 2.36 (m, 2H), 2.36 - 2.21 (m, 2H), 1.65 (d, *J* = 8.1 Hz, 4H).

### Example 4

### Screening of oxidants for the oxidation of the dihydropyridazines to pyridazines,

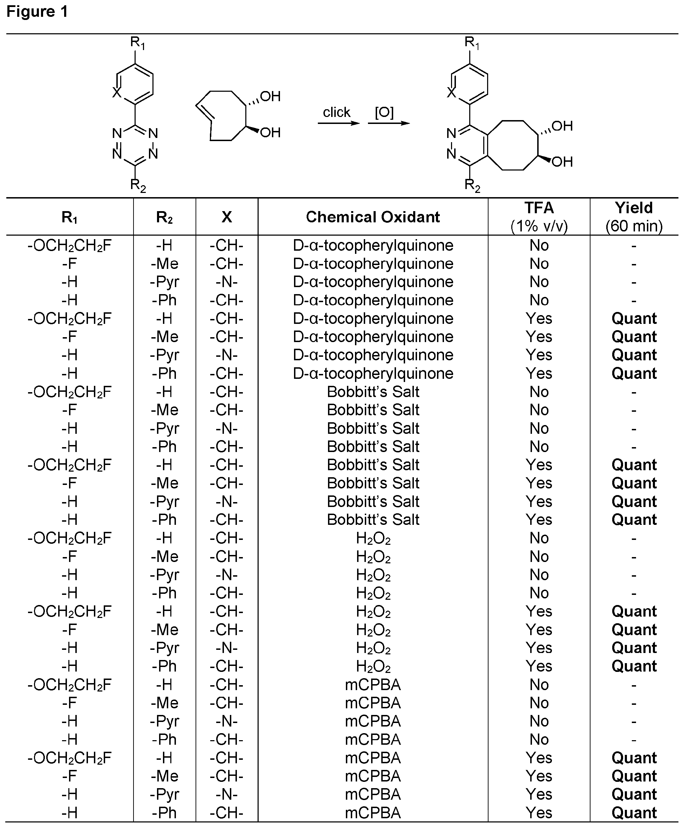

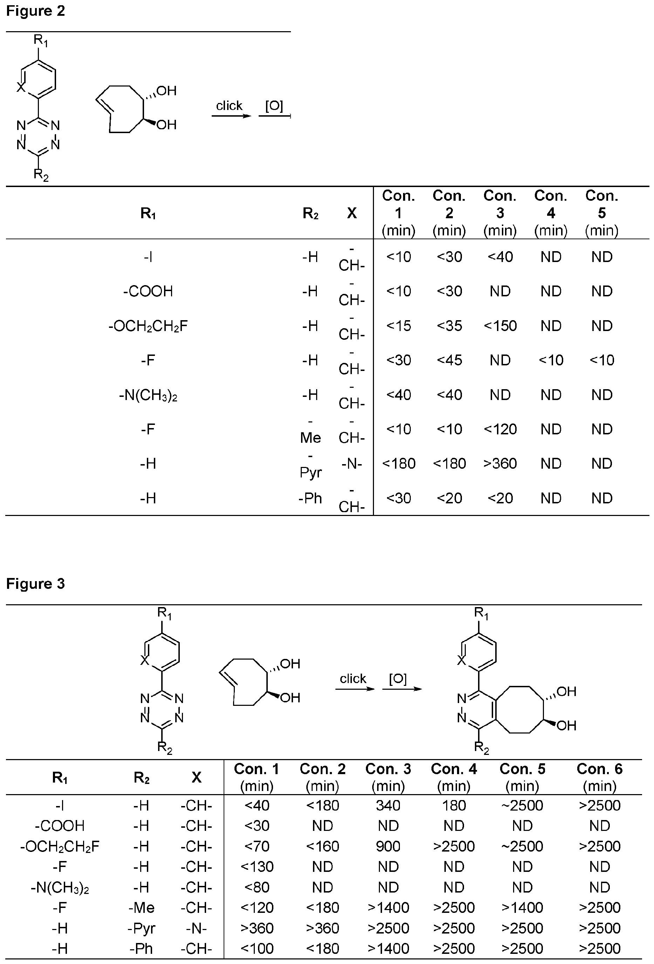

Figures 1-4 shows the reaction between a tetrazine and a TCO, dissolved in 1:1 H₂O/EtOH (% v/v). In Figures 2, 3, and 4, results obtained under various conditions during the oxidation step are shown. The cycloaddition is completed within 5 minutes to give several isomeric dihydropyridazines. The acid and oxidants are then added to give the final pyridizine product. Each oxidant (5 equivalents) was added to the mixture and the reaction was analyzed by HPLC-MS after 60 minutes. The time for the reactions to oxidize is also shown in the Figures. These screening tests surprisingly showed that not all oxidants, in combination with an acid, could be applied to provide the pyridazine. In most cases the addition of acid (TFA) catalyzes the process. For all the quinone-based oxidants, the corresponding pyridzine is formed, with the more electron deficient quinone, preforming better, i.e. more rapid oxidation. The examples listed of the inorganic-based oxidants are not able to transform the click-product (dihydropyridazines) to the corresponding pyridzine, within 60 minutes. In the case of Bobbitt's salt, the addition of acid (TFA) catalyzed this process. In the case of the examples listed of the organic-based oxidants, mixed results were obtained. For the peroxides and the peracids, the addition of acid (TFA) catalyzed this process.

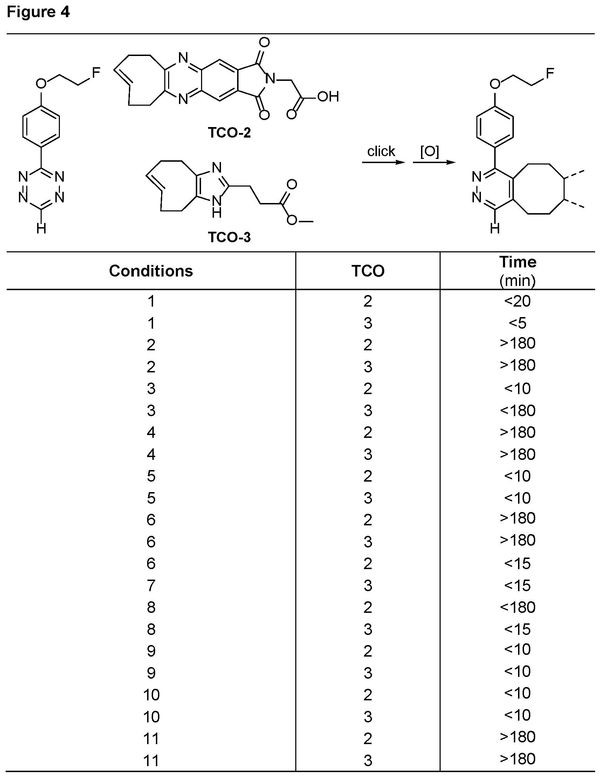

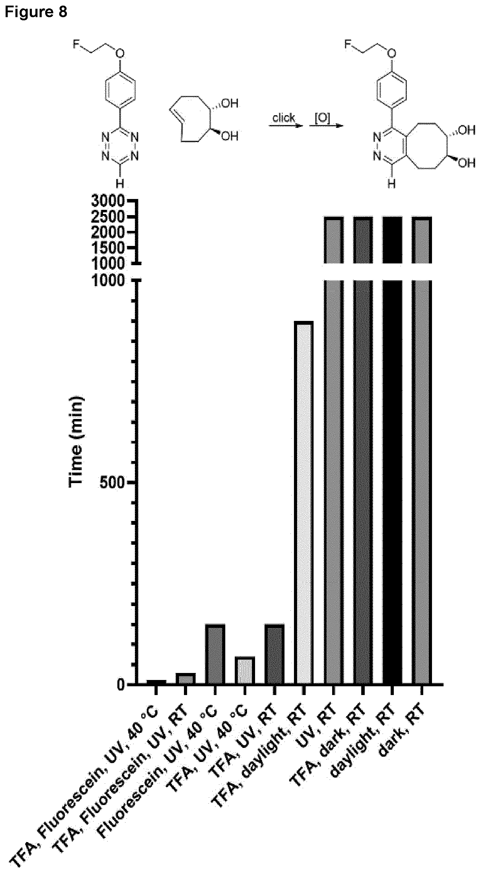

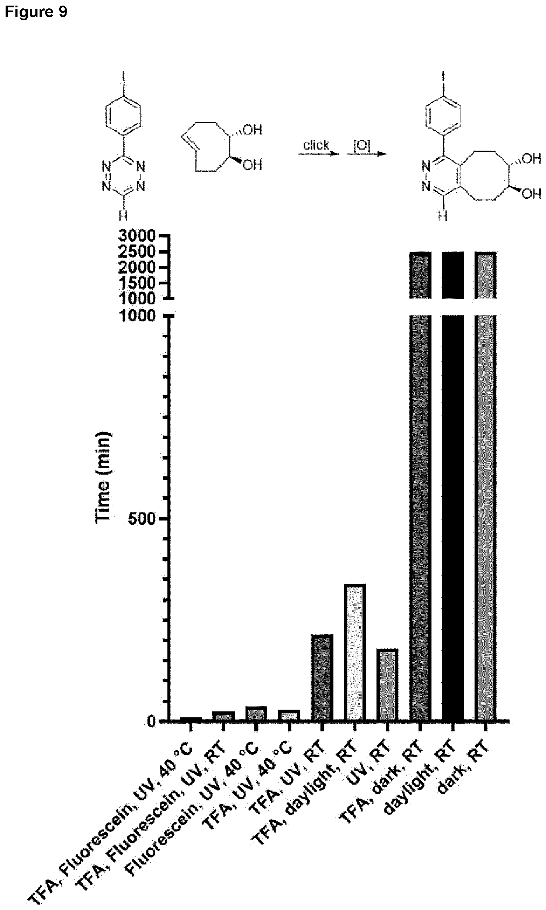

Figures 4-7 show the results of the photocatalysed oxidations, performed in a Rayonet^{®} reactor equipped with 16×254 nm 35 W lamps (RPR-2537 A, UV irradiance of 12 mW/cm²), as well as the addition of acids and/or a photosensitizer. The combination of these parameters shows a clear non-additive effect. Figures 8 and 9 show the data of various oxidants for oxidation of two different dihydropyridazine compounds.

For the tests shown in Figure 2, the following conditions (con.) were applied:
Tz (1 eq, 0.0018 mmol), TCO(OH)₂ (2 eq, 0.0035 mmol), 1:1 H₂O/EtOH (v/v%), in borosilicate glass. Time is in minutes till >90% conversion.
Con. 1: TFA (1% v/v), Fluorescein (0.4 eq), UV₍₂₅₄ₙₘ₎, 40 °C
Con. 2: TFA (1% v/v), Fluorescein (0.4 eq), UV₍₂₅₄ₙₘ₎, RT
Con. 3: Fluorescein (0.4 eq), UV₍₂₅₄ₙₘ₎, 40 °C
Con. 4: TFA (1% v/v), 5-(4-carboxyphenyl)-10,15,20-(tri-*N*-methyl-4-pyridyl)porphyrin trichloride (0.4 eq), UV₍₂₅₄ₙₘ₎, RT
Con. 5: TFA (1% v/v), 5-(4-carboxypropylcarbamoylphenyl)-10,15,20-(tri-4-sulfonatophenyl)porphyrin triammonium (0.4 eq), UV₍₂₅₄ₙₘ₎, RT.

For the tests shown in Figure 3, the following conditions (con.) were applied:
Tz (1 eq, 0.0018 mmol), TCO(OH)₂ (2 eq, 0.0035 mmol), 1:1 H₂O/EtOH (v/v%), in borosilicate glass. Time is in minutes till >90% conversion.
Con. 1: TFA (1% v/v), UV₍₂₅₄ₙₘ₎, 40 °C
Con. 2: TFA (1% v/v), UV₍₂₅₄ₙₘ₎, RT
Con. 3: TFA (1% v/v), daylight, RT (control)
Con. 4: UV₍₃₆₅ₙₘ₎, RT (control)
Con. 5: TFA (1% v/v), dark, RT (control)
Con. 6: daylight, RT (control)

For the tests shown in Figure 4, the following conditions (con.) were applied:
Tz (1 eq, 0.0018 mmol), TCO (1 eq, 0.0018 mmol), 1:1 H₂O/EtOH (v/v%). Time is in minutes till >90% conversion.
Con. 1: TFA (1% v/v), mCPBA, RT
Con. 2: mCPBA, RT (control)
Con. 3: TFA (1% v/v), Bobbitt's salt, RT
Con. 4: Bobbitt's salt, RT (control)
Con. 5: TFA (1% v/v), D-α-tocopherylquinone, RT
Con. 6: D-α-tocopherylquinone, RT (control)
Con. 7: TFA (1% v/v), Fluorescein (0.4 eq), UV₍₂₅₄ₙₘ₎, RT (in borosilicate glass)
Con. 8: TFA (1% v/v), UV₍₂₅₄ₙₘ₎, RT (in borosilicate glass)
Con. 9: TFA (1% v/v), 5-(4-carboxyphenyl)-10,15,20-(tri-*N*-methyl-4-pyridyl)porphyrin trichloride (0.4 eq), UV₍₂₅₄ₙₘ₎, RT (in borosilicate glass)
Con. 10: TFA (1% v/v), 5-(4-carboxypropylcarbamoylphenyl)-10,15,20-(tri-4-sulfonatophenyl)porphyrin triammonium (0.4 eq), UV₍₂₅₄ₙₘ₎, RT (in borosilicate glass)
Con. 11: TFA (1% v/v), daylight (in borosilicate glass) (control).

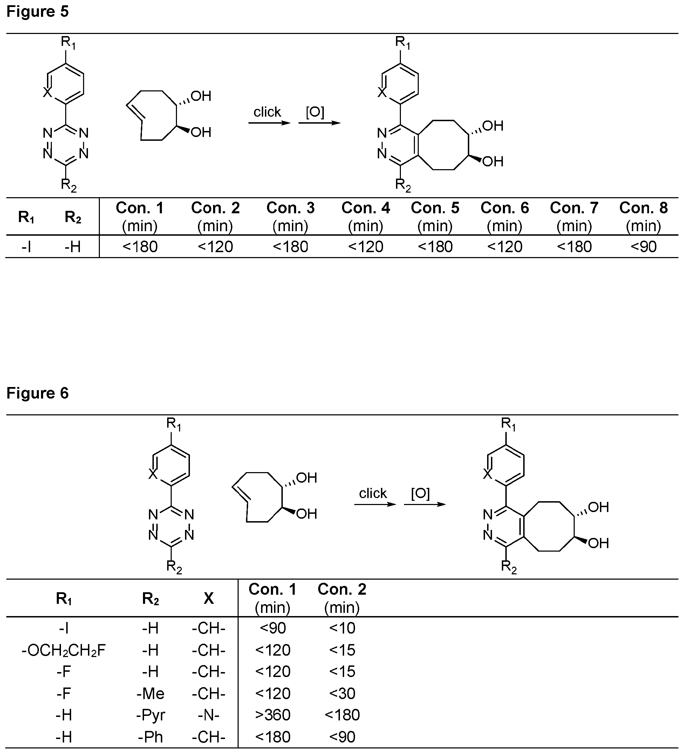

For the tests shown in Figure 5, the following conditions (con.) were applied:
Tz (1 eq, 0.0018 mmol), TCO(OH)₂ (2 eq, 0.0035 mmol), 1:1 H₂O/EtOH (v/v%), in borosilicate glass. Time is in minutes till >90% conversion.
Con. 1: TFA (0.1 M), UV₍₂₅₄ₙₘ₎, RT
Con. 2: HCl (0.1 M), UV₍₂₅₄ₙₘ₎, RT
Con. 3: H₃PO₄ (0.5 M), UV₍₂₅₄ₙₘ₎, RT
Con. 4: TFA (0.5 M), UV₍₂₅₄ₙₘ₎, RT
Con. 5: AcOH (1 M), UV₍₂₅₄ₙₘ₎, RT
Con. 6: TFA (1 M), UV₍₂₅₄ₙₘ₎, RT
Con. 7: H₂SO₄ (1 M), UV₍₂₅₄ₙₘ₎, RT
Con. 8: HCl (1 M), UV₍₂₅₄ₙₘ₎, RT.

For the tests shown in Figure 6, the following conditions (con.) were applied:
Tz (1 eq, 0.0018 mmol), TCO(OH)₂ (2 eq, 0.0035 mmol), 1:1 H₂O/EtOH (v/v%).
Time is in minutes till >90% conversion.
Con. 1: HCl (1 M), UV₍₂₅₄ₙₘ₎, RT, borosilicate vial (open)
Con. 2: HCl (1 M), UV₍₂₅₄ₙₘ₎, RT, quartz flask (open)

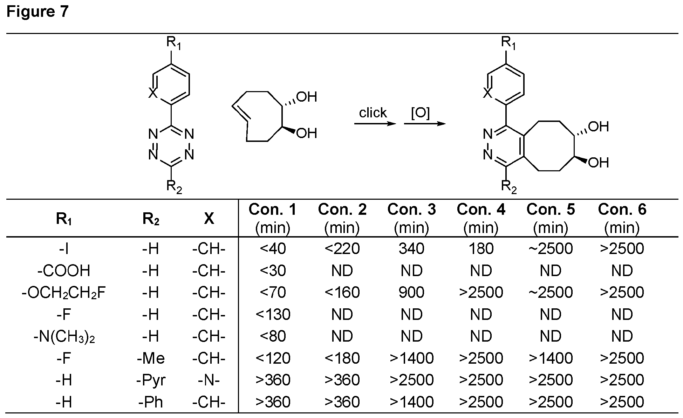

For the tests shown in Figure 7, the following conditions (con.) were applied:
Tz (1 eq, 0.0018 mmol), TCO(OH)₂ (2 eq, 0.0035 mmol), 1:1 H₂O/EtOH (v/v%).
Time is in minutes till >90% conversion.
Con. 1: TFA (1% v/v), UV₍₂₅₄ₙₘ₎, 40 °C
Con. 2: TFA (1% v/v), UV₍₂₅₄ₙₘ₎, RT
Con. 3: TFA (1% v/v), daylight, RT (control)
Con. 4: UV₍₂₅₄ₙₘ₎, RT (control)
Con. 5: TFA (1% v/v), dark, RT (control)
Con. 6: daylight, RT (control).

Figure 8 and 9 show the non-additive effect of the combination of UV-light and acid that was observed when oxidizing the resulting dihydropyridazines (from the ligation between a Tz and a cyclic unsaturated hydrocarbon, with at least one double bond in the trans-configuration, as shown above each of the Figures), to provide the corresponding pyridazine.

### Example 5

### Screening of oxidants for the oxidation of the radiolabeled dihydropyridazines to radiolabeled pyridazines

### Oxidations of radiolabeled compounds

The crude solution of ¹⁸F-click product (50-100 µL), obtained by mixing ¹⁸F-Tz and TCO solutions as described in the Click section (Example 4), was used for oxidation experiments. Oxidation protocol consisted of two steps: acidification and the introduction of oxidant.

Acidification: solution of click product was mixed with acid solution at 19:1 or 9:1 v/v ratio. Acid solution was either neat acid or an aqueous solution of the desired acid.

Oxidation with solid oxidants: acidified solution of click product was further mixed at 9:1 v/v ratio with 10 mg/mL solution of the desired solid oxidant in the same organic co-solvent as was used for the click reaction (i.e. ethanol for ethanol-water mixtures and acetonitrile for acetonitrile-water mixtures). When the oxidant could not be dissolved to 10 mg/mL in the selected co-solvent, a saturated solution was used instead. The oxidation mixture was left standing in a closed vial at room temperature for the desired time period, then a sample was withdrawn and analyzed by HPLC. Oxidation with air oxygen: acidified solution of click product (60-100 µL) was left standing in an open vial at room temperature for the desired time period, then a sample was withdrawn and analyzed by HPLC.

Oxidation with medical oxygen: medical oxygen from a rubber balloon was bubbled through acidified solution of click product (60-100 µL) at room temperature. Where the total volume of the solution was reduced more than 2-fold by evaporation, it was reconstituted using the same water-organic mixture as used for the click reaction. Then, a sample was withdrawn and analyzed by HPLC.

UV-assisted oxidation: acidified solution of click product was mixed at 9:1 v/v ratio with concentrated solution of the desired photosensitizer in water or in the corresponding organic co-solvent (for fluorescent dyes). The resulting mixture was irradiated under a CAMAG UV Lamp equipped with 2×254 nm 8 W lamps UV irradiance of 2 mW/cm² for the desired time period, then a sample was withdrawn and analyzed by HPLC.

### Radio oxidations with chemical oxidants:

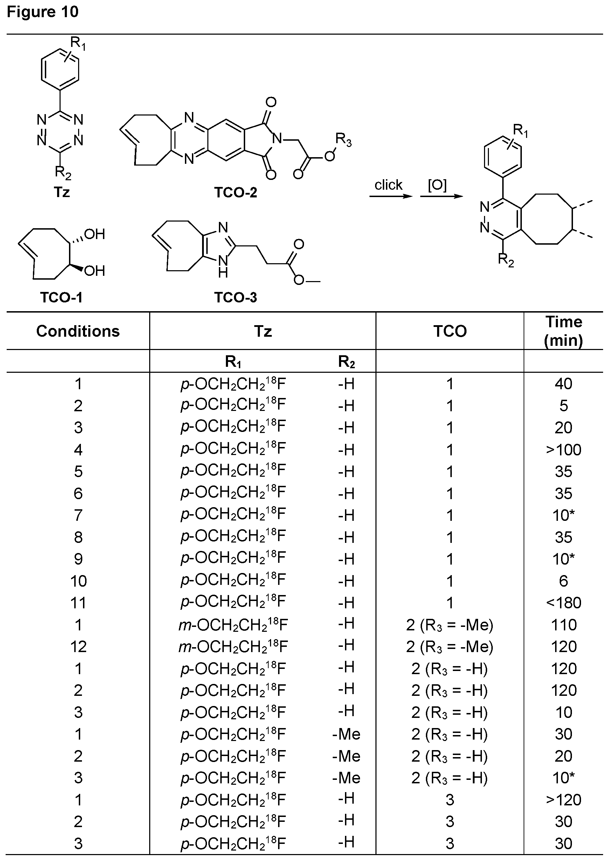

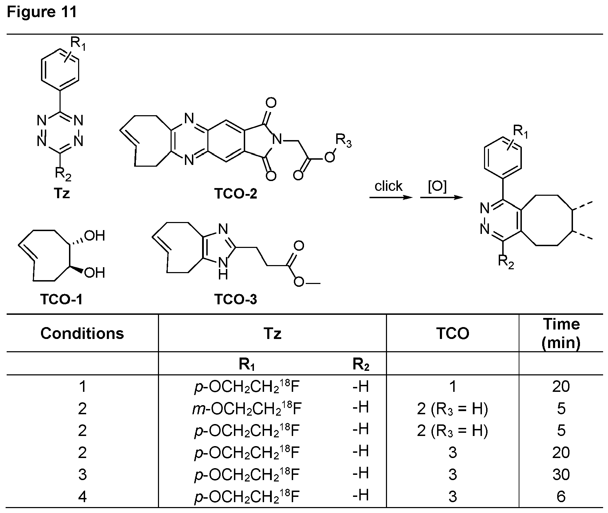

Here and further, time needed to reach >90% oxidation is shown in Figures 10 and 11.

For the tests shown in Figure 10, the following conditions (con.) were applied:
Con. 1: TCO (30 µM), 0.1M HCl in 9:1 MeCN/H₂O (v/v%), open vial, RT (control)
Con. 2: TCO (30 µM), 0.1M HCl in 9:1 MeCN/H₂O (v/v%), 1 mg/mL Fluoranil, closed vial, RT
Con. 3: TCO (30 µM), 0.1M HCl in 9:1 MeCN/H₂O (v/v%), O₂ gas bubbling, open vial, RT (control)
Con. 4: TCO( (30 µM), 1% TFA in 1:1 EtOH/H₂O (v/v%), open vial, RT (control)
Con. 5: TCO (30 µM), 1% TFA in 1:1 EtOH/H₂O (v/v%), 1 mM Duroquinone, closed vial, RT
Con. 6: TCO (30 µM), 1% TFA in 1:1 EtOH/H₂O (v/v%), 1 mM Bobbitt's salt, closed vial, RT
Con. 7: TCO (30 µM), 1% TFA in 1:1 EtOH/H₂O (v/v%), 1 mM mCPBA, closed vial, RT
Con. 8: TCO (30 µM), 1% TFA in 1:1 EtOH/H₂O (v/v%), 3% H₂O₂, closed vial, RT
Con. 9: TCO (30 µM), 1% TFA in 1:1 EtOH/H₂O (v/v%), 1 mM PIDA, closed vial, RT
Con. 10: TCO (30 µM), 1% TFA in 1:1 EtOH/H₂O (v/v%), 1 mM TEMPO, closed vial, RT
Con. 11: TCO (30 µM), 1% TFA in 1:1 EtOH/H₂O (v/v%), 1 mM D-α-tocopherylquinone, closed vial, RT
Con. 12: TCO (10 µM), 0.1M HCl in 9:1 MeCN/H₂O (v/v%), 1 mg/mL chloranil, closed vial, RT.

For the tests shown in Figure 11, the following conditions (con.) were applied:
Con. 1: TCO (30 µM), 0.1M HCl in 9:1 MeCN/H₂O (v/v%), UV₍₂₅₄ₙₘ₎, open vial, RT
Con. 2: TCO (5 µM), 0.1M HCl in 9:1 MeCN/H₂O (v/v%), UV₍₂₅₄ₙₘ₎, open vial, RT
Con. 3: TCO (5 µM), 0.1M HCl in 9:1 MeCN/H₂O (v/v%), O₂ saturated solution, UV₍₂₅₄ₙₘ₎, open vial, RT
Con. 4: TCO (5 µM), 0.1M HCl in 9:1 MeCN/H₂O (v/v%), 50 ug/mL Fluorescein, UV₍₂₅₄ₙₘ₎, open vial, RT

### Example 6

### Measurement of second-order rate constants

The second-order rate constant of all the click reactions made during the previous examples were measured by stopped-flow spectrometry in phosphate-buffered saline (PBS) at 25 °C in accordance with the method described in Battisti et al. J. Med. Chem. 2021, 64, 20, 15297-15312 (see page 15310 for experimental details and influencing factors). In short, stopped-flow measurements were performed using an SX20-LED stopped-flow spectrophotometer (Applied Photophysics) equipped with a 535 nm LED (optical pathlength 10 mm and full width half-maximum 34 nm) to monitor the characteristic tetrazine visible light absorbance (520-540 nm). The reagent syringes were loaded with a solution of axial-TCO-PEG₄, and the instrument was primed. The subsequent data were collected in triplicate for each tetrazine. Reactions were conducted at 25 °C in PBS and recorded automatically at the time of acquisition. The data sets were analyzed by fitting an exponential decay using Prism 6 (GraphPad) to calculate the observed pseudo-first-order rate constants that were converted to second-order rate constants by dividing with the concentration of the excess TCO compound.

Only reactions that showed a minimum second-order rate constant of 250 M⁻¹ s⁻¹ in phosphate-buffered saline at 25 °C are considered suitable for providing the sufficient speed kinetics. These measurements confirmed that click reactions between a 1,2,4,5-tetrazine and a dienophile with a cyclic unsaturated hydrocarbon with at least one double bond in the *trans*-configuration and reactions between a 1,2,4,5-tetrazine and a dienophile with unsaturated heterocycle, with at least one double bond in the *trans*-configuration all showed minimum second-order rate constants of 250 M⁻¹ s-'.

## Claims

1. Method for oxidation of dihydropyridazines to pyridazines:
a) labeling a first chemical entity having inverse electron demand Diels-Alder cycloaddition reactivity and being conjugated to a pharmaceutic agent, a diagnostic agent or a therapeutic agent, with a labeling agent; wherein the first chemical entity is a 1,2,4,5-tetrazine or a dienophile selected from the group consisting of a cyclic unsaturated hydrocarbon with at least one double bond in the *trans*-configuration; an unsaturated heterocycle, with at least one double bond in the *trans*-configuration; and
b) ligating the labeled first chemical entity obtained in step a) with a second chemical entity having complementary inverse electron demand Diels-Alder cycloaddition reactivity and being conjugated to a targeting vector; wherein the second chemical entity is a 1,2,4,5-tetrazine or a dienophile selected from the group consisting of an cyclic unsaturated hydrocarbon, with at least one double bond in the *trans*-configuration; an unsaturated heterocycle, with at least one double bond in the *trans*-configuration, and
c) oxidizing the resulting mixture of dihydropyridazines labeled targeting vector obtained from step b) in a solvent at a temperature ranging from 15 °C to 50 °C for up to 180 minutes, by addition of an acid catalyst, wherein the acid catalyst has a pKa of 5 or below in H₂O at 25 °C, at a concentration of 0.1 M - 5 M to the mixture obtained from step b), and an oxidant selected from quinone-based oxidants, Bobbits salt-based oxidants, peroxide-based oxidants, peroxy acid-based oxidants, UV-light with a minimum UV irradiance of 2 mW/cm².

2. Method according to claims 1, wherein the labeling agent in step a) is a radionuclide or a stable isotope of a corresponding element.

3. Method according to claim 2, wherein the labeling agent in step a) is selected from ¹H, ²H, ³H, ¹¹C, ¹²C, ¹³C, ¹⁴C, ¹³N, ¹⁴N, ¹⁵N, ¹⁸F, ¹⁹F, ¹²³I, ¹²⁴I,¹²⁵I, ¹²⁷I, ¹³¹I, ¹⁵O, ¹⁶O, ¹⁷O, ¹⁸O, ⁴³Sc, ⁴⁴Sc, ⁴⁵Sc, ⁴⁵Ti, ⁴⁶Ti, ⁴⁷Ti, ⁴⁸Ti, ⁴⁹Ti, ⁵⁰Ti, ⁵⁵Co, ⁵⁸mCo, ⁵⁹Co, ⁶⁰Cu, ⁶¹Cu, ⁶³Cu, ⁶⁴Cu, ⁶⁵Cu, ⁶⁷Cu, ⁶⁷Ga, ⁶⁸Ga, ⁶⁹Ga, ⁷¹Ga, ⁷⁶Br, ⁷⁷Br, ⁷⁹Br, ⁸⁰mBr, ⁸¹Br, ⁷²As, ⁷⁵As, ⁸⁶Y, ⁸⁹Y, ⁹⁰Y, ⁸⁹Zr, ⁹⁰Zr, ⁹¹Zr, ⁹²Zr, ⁹⁴Zr, ¹⁴⁹Tb, ¹⁵²Tb, ¹⁵⁹Tb, ¹⁶¹Tb, ¹¹¹In, ¹¹³In, ¹¹⁴mIn, ¹¹⁵mIn, ¹⁷⁵Lu, ¹⁷⁷Lu, ¹⁸⁵Re, ¹⁸⁶Re, ¹⁸⁸Re, ²⁰¹Tl, ²⁰³Tl, ²⁰⁵Tl, ²⁰⁶Pb, ²⁰⁷Pb, ²⁰⁸Pb, ²¹²Pb, ²⁰⁹Bi, ²¹²Bi, ^{2 13}Bi, ³¹P, ³²P, ³³P, ³²S, ³⁵S, ⁴⁵Sc, ⁴⁷Sc, ⁸⁴Sr, ⁸⁶Sr, ⁸⁷Sr, ⁸⁸Sr, ⁸⁹Sr, ¹⁶⁵Ho, ¹⁶⁶Ho, ¹⁵⁶Dy, ¹⁵⁸Dy, ¹⁶⁰Dy, ¹⁶¹Dy, ¹⁶²Dy, ¹⁶³Dy, ¹⁶⁴Dy, ¹⁶⁵Dy, ²²⁷Th, ²³²Th, ⁵¹Cr, ⁵²Cr, ⁵³Cr, ⁵⁴Cr, ⁷³Se, ⁷⁴Se, ⁷⁵Se, ⁷⁶Se, ⁷⁷Se, ⁷⁸Se, ⁸⁰Se, ⁸²Se, ⁹⁴Tc, ^{99m}Tc, ¹⁰³Rh,¹⁰³mRh, ¹¹⁹Sb, ¹²¹Sb, ¹²³Sb, ¹³⁵La, ¹³⁸La, ¹³⁹La, ¹⁶²Er, ¹⁶⁴Er, ¹⁶⁵Er, ¹⁶⁶Er, ¹⁶⁷Er, ¹⁶⁸Er, ¹⁷⁰Er, ¹⁹³mPt, ¹⁹⁵mPt, ¹⁹²Pt, ¹⁹⁴Pt, ¹⁹⁵Pt, ¹⁹⁶Pt, ¹⁹⁸Pt, ²¹¹At, ²²³Ra, ²²⁵Ac.

4. Method according to any of the previous claims, wherein the targeting vector in step b) is an antibody, a nanobody, a polymer, a nanomedicine, a cell, a protein, a peptide, or a small molecule.

5. Method according to any of the previous claims, wherein the solvent in step c) comprises 5 - 95% water and the oxidant in step c) is UV-light with a minimum UV irradiance of 2 mW/cm².

6. Method according to any of the previous claims, wherein step c) is performed in a quartz container.

7. Method according to any of the previous claims, wherein step c) is performed in an open container.

8. Method according to any of the previous claims, wherein the oxidant in step c) is solid phase supported.

9. Method according to any of the previous claims, further comprising the addition of a photosensitizer in step c) when the oxidant is UV-light with a minimum UV irradiance of 2 mW/cm².

10. Method according to claim 9, wherein the photosensitizer is a fluorescein based photosensitizer or a porphyrin based photosensitizer

11. Method according to any of the previous claims, wherein the tetrazine is of formula Tz1: 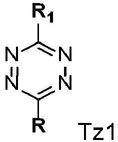
wherein R and R₁ are independently selected from -H, -Me or 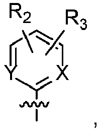 wherein the curly sign indicates the link to the tetrazine; and where R₂ is -H or (i) an isotope labeling agent directly connected to the aromatic ring; or (ii) an isotope labeling agent connected to the aromatic ring via a linker, said linker being selected from the group consisting of -(CH₂)ₙ, LO(CH₂)ₙ, -LNH(CH₂)ₙ, -LCONH(CH₂)ₙ, - LNHCO(CH₂)ₙ, where L is -(CH₂)ₘ or -(CH₂CH₂O)ₘ , where n and m are independently selected from 1-25; or (iii) an isotope labeling agent that is chelated through a chelator selected from: 1,4,7,10-tetraazacyclododecane-*N,N',N',N"-*tetraacetic acid (DOTA), *N,N'*-bis(2-hydroxy-5-(carboxyethyl)benzyl)ethylenediamine *N,N'*-diacetic acid (HBED-CC), 14,7-triazacyclononane-1,4,7-triacetic acid (NOTA), 2-(4.7-bis(carboxymethyl)-1,4,7-triazonan-1-yl)pentanedioic acid (NODAGA), 2-(4,7,10-tris(carboxymethyl)-1,4,7,10-tetraazacyclododecan-1- yl)pentanedioic acid (DOTAGA), 14,7-triazacyclononane phosphinic acid (TRAP), 14,7-triazacyclononane-1-methyl(2-carboxyethyl)phosphinic acid-4,7-bis(methyl(2-hydroxymethyl)phosphinic acid (NOPO), 3,6,9,15-tetraazabicyclo9.3.1.pentadeca-1 (15), 11,13-triene-3,6,9-triacetic acid (PCTA), *N*'-(5-acetyl (hydroxy)aminopentyl-N-(5-(4-(5-aminopentyl)(hydroxy)amino-4-oxobutanoyl)amino)pentyl-*N*-hydroxysuccinamide (DFO), diethylenetriaminepentaacetic acid (DTPA), *trans*-cyclohexyl-diethylenetriaminepentaacetic acid (CHX-DTPA), 1-oxa-4,7,10-triazacyclododecane-4,7,10-triacetic acid (OXO-Do3A), *p*-isothiocyanatobenzyl-DTPA (SCN-BZ-DTPA), 1-(*p*-isothiocyanatobenzyl)-3-methyl-DTPA (1B3M), 2-(p-isothiocyanatobenzyl)-4-methyl-DTPA (1M3B), and 1-(2)-methyl-4-isocyanatobenzyl-DTPA (MX-DTPA) connected to the aromatic ring through a linker, said linker being selected from the group consisting of -(CH₂)ₙ, -LO(CH₂)ₙ, - LNH(CH₂)ₙ, -LCONH(CH₂)ₙ, -LNHCO(CH₂)ₙ, where L is -(CH₂)ₘ or -(CH₂CH₂O)ₘ, and n and m are independently selected from 1-25;
wherein, when R₂ is either (i) or (ii) the isotope labeling agent is selected from the group consisting of:
¹H, ²H, ³H, ¹¹C, ¹²C, ¹³C ¹⁴C, ¹³N, ¹⁴N, ¹⁵N ¹⁸F, ¹⁹F, ¹²³I, ¹²⁴I,¹²⁵I, ¹²⁷I, ¹³¹I, ²¹¹At, ¹⁵O, ¹⁶O, ¹⁷O,¹⁸O, ⁴³Sc, ⁴⁴Sc, ⁴⁵Sc, ⁴⁵Ti, ⁴⁶Ti, ⁴⁷Ti, ⁴⁸Ti, ⁴⁹Ti, ⁵⁰Ti, ⁵⁵Co, ⁵⁸mCo, ⁵⁹Co, ⁶⁰Cu, ⁶¹Cu, ⁶³Cu, ⁶⁴Cu, ⁶⁵Cu, ⁶⁷Cu, ⁶⁷Ga, ⁶⁸Ga, ⁶⁹Ga, ⁷¹Ga, ⁷⁶Br, ⁷⁷Br, ⁷⁹Br, ⁸⁰mBr, ⁸¹Br, ⁷²As, ⁷⁵As, ⁸⁶Y, ⁸⁹Y, ⁹⁰Y, ⁸⁹Zr, ⁹⁰Zr, ⁹¹Zr, ⁹²Zr, ⁹⁴Zr, ¹⁴⁹Tb, ¹⁵²Tb, ¹⁵⁹Tb, ¹⁶¹Tb, ¹¹¹In, ¹¹³In, ¹¹⁴mIn, ¹¹⁵mIn, ¹⁷⁵Lu, ¹⁷⁷Lu, ¹⁸⁵Re, ¹⁸⁶Re, ¹⁸⁸Re, ²⁰¹Tl, ²⁰³Tl, ²⁰⁵Tl, ²⁰⁶Pb, ²⁰⁷Pb, ²⁰⁸Pb, ²¹²Pb, ²⁰⁹Bi, ²¹²Bi, ²¹³Bi, ³¹P, ³²P, ³³P, ³²S, ³⁵S, ⁴⁵Sc, ⁴⁷Sc, ⁸⁴Sr, ⁸⁶Sr, ⁸⁷Sr, ⁸⁸Sr, ⁸⁹Sr, ¹⁶⁵Ho, ¹⁶⁶Ho, ¹⁵⁶Dy, ¹⁵⁸Dy, ¹⁶⁰Dy, ¹⁶¹Dy ¹⁶²Dy, ¹⁶³Dy, ¹⁶⁴Dy, ¹⁶⁵Dy, ²²⁷Th, ²³²Th, ⁵¹Cr, ⁵²Cr, ⁵³Cr, ⁵⁴Cr, ⁷³Se, ⁷⁴Se, ⁷⁵Se, ⁷⁶Se, ⁷⁷Se, ⁷⁸Se, ⁸⁰Se, ⁸²Se, ⁹⁴Tc, ^{99m}Tc, ¹⁰³Rh, ¹⁰³mRh, ¹¹⁹Sb, ¹²¹Sb, ¹²³Sb, ¹³⁵La, ¹³⁸La, ¹³⁹La, ¹⁶²Er, ¹⁶⁴Er, ¹⁶⁵Er, ¹⁶⁶Er, ¹⁶⁷Er, ¹⁶⁸Er, ¹⁷⁰Er, ¹⁹³mPt, ¹⁹⁵mPt ¹⁹²Pt, ¹⁹⁴Pt, ¹⁹⁵Pt, ¹⁹⁶Pt, ¹⁹⁸Pt, ²¹¹At, ²²³Ra, ²²⁵Ac,
and wherein X and Y are independently selected from: -CH and -N- ;
and wherein R₃ is independently selected from H or a moiety selected from the group consisting of a hydroxy group, a sulfonamide, a guanidyl group, a carboxyl group, a sulfonyl group, amine, a substituted amine with 1-25 polyethylene glycol unit(s), a -(O-CH₂-CH₂)ₙ₋-OCH₂-COOH, and n is selected from 1-25; or Methyl, Ethyl, Propyl, optionally substituted heteroaryl, and optionally substituted arylalkyl; wherein optionally substituted in relation to said substituted amine means one or more substituents selected from, a halogen, a hydroxy group, a sulfonamide, a carboxyl group, a sulfonyl group, amine, (C1-C10) alkyl, (C2-C10)alkenyl, (C2-C10)alkynyl, (C1-C10)alkylene, (C1-C10)alkoxy, (C2-C10)dialkylamino, (C1-C10)alkylthio, (C2-C10)heteroalkyl, (C2-C10)heteroalkylene, (C3-C10)cycloalkyl, (C3-C10)heterocycloalkyl, (C3-10)cycloalkylene, (C3-C10)heterocycloalkylene, (C1-C10)haloalkyl, (C1-C10)perhaloalkyl, (C2-C10)-alkenyloxy, (C3-C10)-alkynyloxy, aryloxy, arylalkyloxy, heteroaryloxy, heteroarylalkyloxy, (C1-C6)alkyloxy-(C1-C4)alkyl, optionally substituted aryl, optionally substituted heteroaryl, and optionally substituted arylalkyl; wherein optionally substituted means one or more substituents selected from a halogen, a hydroxy group, a sulfonamide, a carboxyl group, a sulfonyl group, amine, a substituted amine with 1-25 polyethylene glycol unit(s), a -(O-CH₂-CH₂)ₙ-OCH₂-COOH, and n is selected from 1-25; or H, Methyl, Ethyl, Propyl, optionally substituted heteroaryl, and optionally substituted arylalkyl; wherein optionally substituted in relation to said substituted amine means one or more substituents selected from a halogen, a hydroxy group, a sulfonamide, a carboxyl group, a sulfonyl group, and amine;
and wherein R and R₁ are identical or differs only in the isotope number of the labelling agent.

12. Method according to claim 11 wherein the tetrazine is selected from:

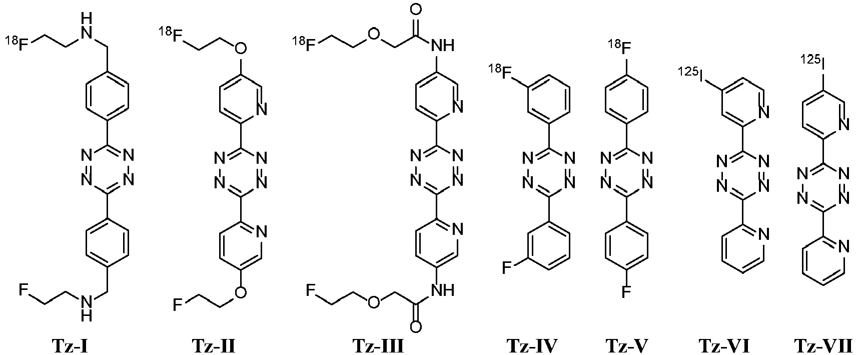

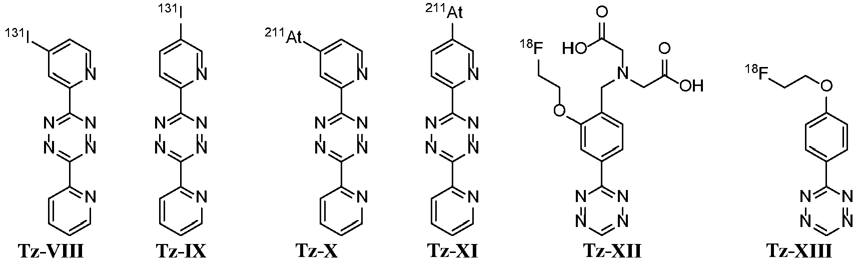

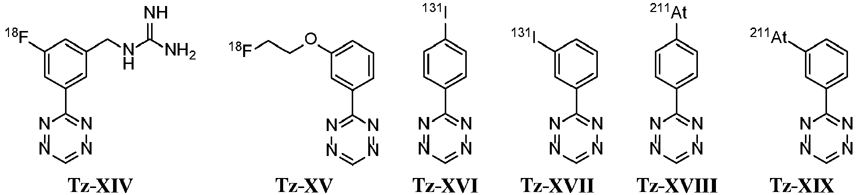

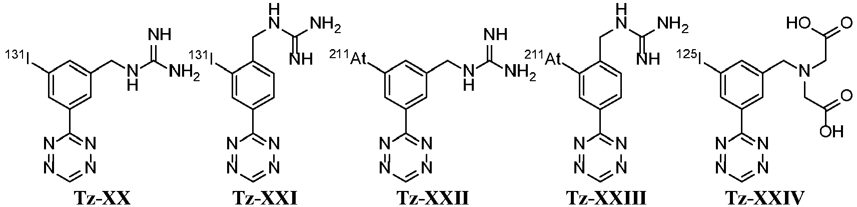

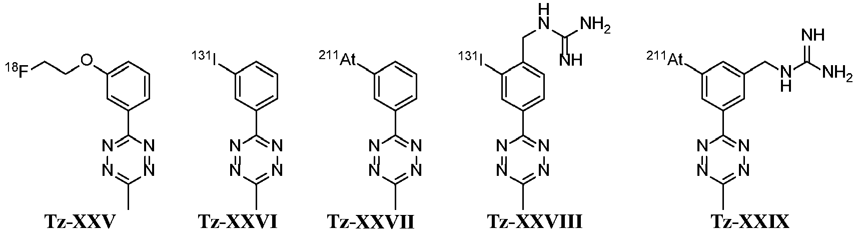

13. Method according to any of claims the previous claims, wherein the second chemical entity is selected from: 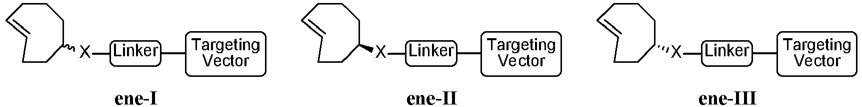 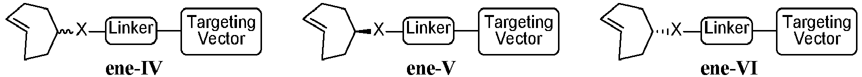 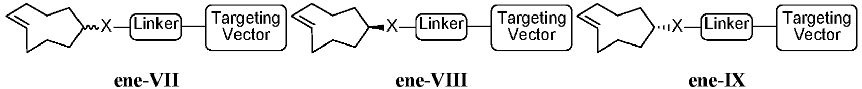 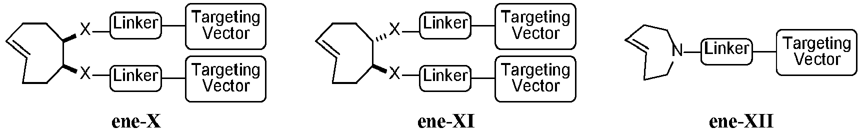 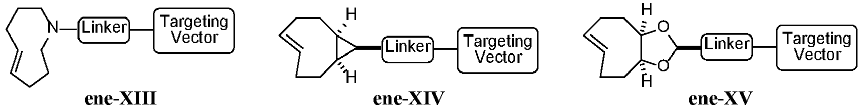 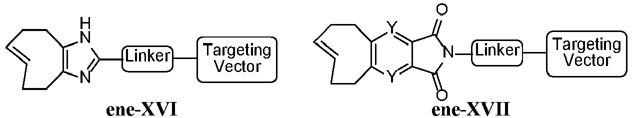 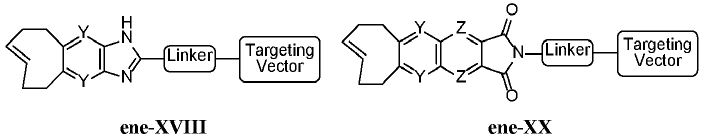 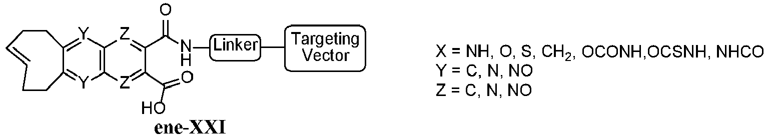
wherein X is NH, O, S, CH₂, OCONH, OCSNH, NHCO; Y is N, NO or CR₈; Z is N, NO or CR₈; R₈ is selected from the group consisting of: -H, -F, -OH, -NH₂, -COOH, - COOCH₃, CF₃, -Cl, -CONH₂, CONHCH₃, -CON(CH₃)₂, , -CH₂OH, -CH₂NH₂,-CH₂CH₂OH, -CH₂CH₂NH₂, -CHCH₂N(CH₃)₂ and
wherein the linker is selected from the group comprising: -(CH₂)ₙ- (CH₂)ₙNH, (CH₂)ₙCO, (CH₂)ₙO, (CH₂CH₂O)ₙ, (CH₂CH₂O)ₙCH₂CH₂NH, (CH₂CH₂O)ₙCH₂CH₂CO, - CO(CH)₂- CO(CH₂)ₙNH, CO(CH₂)ₙCO, CO(CH₂)ₙO, CO(CH₂CH₂O)ₙ CO(CH₂CH₂O)ₙCH₂CH₂NH, CO(CH₂CH₂O)ₙCH₂CH₂CO, COO(CH)₂- COO(CH₂)ₙNH, COO(CH₂)ₙCO, COO(CH₂)ₙO, COO(CH₂CH2O)ₙ COO(CH₂CH₂O)ₙCH₂CH₂NH, COO(CH₂CH₂O)ₙCH₂CH₂CO, CONH(CH)₂-CONH(CH₂)ₙNH, CONH(CH₂)ₙCO, CONH(CH₂)ₙO, CONH(CH₂CH₂O)ₙ, CONH(CH₂CH₂O)ₙCH₂CH₂NH, CONH(CH₂CH₂O)ₙCH₂CH₂CO, -CONHPhCO, -COOPhCO, -COPhCO, CONHCHMCO, (CH₂)ₙNHCHMCO, (CH₂)ₙOCONHCHMCO, (CH₂)ₙNHCHMCO, (CH₂)ₙNHCOCHMNH, (CH₂)OCOCHMNH, (CH₂CH₂O)ₙCH₂CH₂NHCHMCO, (CH₂CH₂O)ₙCH₂CH₂CONHCHMCO, (CH₂CH₂O)ₙCH₂CH₂NHCHMCO, (CH₂CH₂O)ₙCH₂CH₂NHCOCHMNH, (CH₂CH₂O)ₙCOCHMNH, where n is 0-25 and where M is a side chain selected from the group consisting of side chains of the natural amino acids: H, CH₃, CH₂SH, CH₂COOH, CH₂CH₂COOH, CH₂C₆H₅, CH₂C₃H₃N₂, CH(CH₃)CH₂CH₃, (CH₂)₄NH₂, CH₂CH(CH₃)₂, CH₂CH₂SCH₃, CH₂CONH₂, (CH₂)₄NHCOC₄H₅NCH₃, CH₂CH₂CH₂, CH₂CH₂CONH₂, (CH₂)₃NH-C(NH)NH₂, CH₂OH, CH(OH)CH₃, CH₂SeH, CH(CH₃)₂, CH₂C₈H₆N, CH₂C₆H₄OH;
and where the targeting vector is an antibody, a nanobody, a polymer, a nanomedicine, a cell, a protein, a peptide, or a small molecule.
